# EUROPEAN PATENT SPECIFICATION

(11) **EP 4 172 345 B1**
(45) Date of publication and mention of the grant of the patent: **09.12.2026**
(21) Application number: 21748644.8
(22) Date of filing: 30.06.2021
(51) Int. Cl.: C12N 15/86, A61P 13/12, A61P 37/00

(54) **VECTOR**
VEKTOR
VECTEUR

(30) Priority: 30.06.2020 GB 202010009
(43) Date of publication of application: 03.05.2023
(73) Proprietor: Syncona IP Holdco (3) Limited, London WC1B 3SR (GB); The University of Bristol, Bristol BS8 1QU (GB)
(72) Inventor: GRIFFITH, Alan William, London WC1B 3SR (GB); STANCZUK, Lukas, London WC1B 3SR (GB); KUZMUK, Valeryia, London WC1B 3SR (GB); HOLLOWOOD, Chris, London WC1B 3SR (GB); SCHMIDT, Dominic, London WC1B 3SR (GB); SALEEM-UDDIN, Moin Ahson, Bristol BS8 1QU (GB); WELSH, Gavin Iain, Bristol BS8 1QU (GB)
(74) Representative: Stratagem IPM Limited
(86) International application number: PCT/GB2021/051668
(87) International publication number: WO 2022/003357

(56) References cited:
- WO-A2-2005/012351
- SALVADORI MAURIZIO: "Complement involvement in kidney diseases: From physiopathology to therapeutical targeting", WORLD JOURNAL OF NEPHROLOGY, vol. 4, no. 2, 6 May 2015 (2015-05-06), pages 169 - 184, XP093119570, ISSN: 2220-6124, DOI: 10.5527/wjn.v4.i2.169
- DATABASE EMBASE [online] ELSEVIER SCIENCE PUBLISHERS, AMSTERDAM, NL; 2019, DING W Y ET AL: "Adeno-associated virus gene therapy prevents progression of kidney disease in genetic human and mouse models of nephrotic syndrome", XP002804349, Database accession no. EMB-633771110
- DING W Y ET AL: "Adeno-associated virus gene therapy prevents progression of kidney disease in genetic human and mouse models of nephrotic syndrome", JOURNAL OF THE AMERICAN SOCIETY OF NEPHROLOGY 2019 AMERICAN SOCIETY OF NEPHROLOGY NLD, vol. 30, 2019, pages 94 CONF 20191105 to 20191110 Washington, DC - Kidney We, ISSN: 1533-3450
- M T BIRKE ET AL: "AAV-mediated expression of human PRELP inhibits complement activation, choroidal neovascularization and deposition of membrane attack complex in mice", GENE THERAPY, vol. 21, no. 5, 27 March 2014 (2014-03-27), GB, pages 507 - 513, XP055336648, ISSN: 0969-7128, DOI: 10.1038/gt.2014.24
- WILLOWS JAMIE ET AL: "The role of complement in kidney disease", CLINICAL MEDICINE, vol. 20, no. 2, 1 March 2020 (2020-03-01), London, UK, pages 156 - 160, XP055847264, ISSN: 1470-2118, Retrieved from the Internet <URL:https://www.ncbi.nlm.nih.gov/pmc/articles/PMC7081808/pdf/clinmed-20-2-156.pdf> DOI: 10.7861/clinmed.2019-0452

## Description

### FIELD OF THE INVENTION

The present invention relates to a vector for use in treating complement-mediated kidney diseases.

### BACKGROUND TO THE INVENTION

The kidney is particularly susceptible to damage by chronic, uncontrolled, and excessive activation of the complement cascade. The reasons for this susceptibility are incompletely understood but may be related to the presence of the fenestrae continuously exposing the acellular subendothelial tissues to complement activators, a lower baseline expression of complement regulators, and/or differences in the composition of the glycocalyx.

C3G is a rare, complement-mediated kidney disease and includes two overlapping pathologies, dense deposit disease (DDD) and C3 glomerulonephritis. C3G at presentation is clinically indistinguishable from other glomerulonephritides, presenting with non-visible haematuria, proteinuria, hypertension, nephrotic syndrome and renal impairment. Diagnosis is therefore histopathological on renal biopsy, with sole or dominant C3 deposition in the glomeruli. Median age of onset is 23 years, 50% of patients develop end-stage kidney disease by 10 years after diagnosis, and risk of recurrence in a renal transplant is high (Willows, J., et al., 2020. Clinical Medicine, 20(2), p.156).

Dysregulation of complement has also been implicated in IgA Nephropathy (IgAN). IgAN is the most common primary glomerulonephritis in the world. A diagnosis of IgAN is associated with an average reduction in life expectancy of 6-10 years and approximately 40% of IgAN patients older than 30 years age at diagnosis develop end stage renal disease (ESRD) over 20 years.

However, development of complement inhibiting drugs is challenging due to the high concentration of complement proteins in the circulation and uncertainty whether abnormal complement in a disease-state is a response to or driver of pathology. Moreover, a major potential side effect of systemic complement inhibiting drugs is infection with encapsulated organisms, and a 1,000-fold increase in life-threatening meningococcal infections has been observed (Willows, J., et al., 2020. Clinical Medicine, 20(2), p.156)
In "Adeno-associated virus gene therapy prevents progression of kidney disease in genetic human and mouse models of nephrotic syndrome", Ding W Y et al. Journal of The American Society of Nephrology 2019 American Society of Nephrology NLD, vol. 30, 2019, page 94 is described a method of using AAV vectors and a podocyte-specific promoter to target a monogenic disease of the kidney.

In "AAV-mediated expression of human PRELP inhibits complement activation, choroidal neovascularization and deposition of membrane attack complex in mice", Birke M. T. et al. Gene Therapy, vol. 21, no. 5, 27 March 2014 pages 507-513 is described a method of delivering of an inhibitor of complement mediated damage via the subretinal route using AAV vectors for the treatment of age related macular degeneration (AMD).

In WO 2005/012351 (The Brigham and Women's Hospital, Inc.) is described a method of using AAV vectors to deliver an inhibitor of the complement system to mucosal epithelial surfaces.

In "The role of complement in kidney disease", Willows J et al. Clinical Medicine, vol. 20, no. 2 1 March 2020 (2020-03-01), pages 156-160 are described renal diseases in which complement over-activation is known to cause tissue injury, and the contribution of complement more widely to the pathophysiology of renal disease.

In "Complement involvement in kidney diseases: From physiopathology to therapeutical targeting", Salvadori Maurizio. World Journal of Nephrology, vol. 4, no. 2, 6 May 2015 (2015-05-06), pages 169-184 is described the physiopathology of complement involvement in renal diseases and transplantation and the several complement components that could represent a therapeutic target.

In WO 2009/061448 (The General Hospital Corporation et al.) is described a viral vector comprising a podocyte-specific promoter for the treatment of a complement-mediated kidney disease. WO 2009/061448 does not disclose the use of AAV vectors, nor the specific promoters and inhibitors of the complement system disclosed herein.

### SUMMARY OF THE INVENTION

The inventors have shown that podocytes may play an important role in complement-mediated kidney disease. For example, the inventors have shown that secreted podocyte complement inhibitors (e.g. CFH) may contribute to local complement regulation. The inventors have also shown that a complement inhibitor rescues the diseased phenotype in a mouse model of podocyte-driven complement-mediated disease (e.g. stx mediated HUS).

Accordingly, the present inventors have developed a viral vector for use in treating complement-mediated kidney diseases, such as C3 glomerulopathy and IgA Nephropathy, which is targeted to podocytes.

The present inventors have shown that use of a podocyte-specific promoter and/or a viral vector which is capable of specifically transducing podocytes may be used to target expression of complement proteins to podocytes. For example, the present inventors have shown that AAV-LK03 vectors can achieve high transduction of close to 100% in human podocytes and can be used to transduce podocytes specifically.

In one aspect, the present invention provides a viral vector comprising a nucleotide sequence encoding a complement protein, wherein the nucleotide sequence is operably linked to a podocyte-specific promoter and/or the viral vector is capable of specifically transducing podocytes.

The viral vector is an adeno-associated virus (AAV) vector. In embodiments not part of the claimed invention the viral vector may be an adenoviral vector, a herpes simplex viral vector, a retroviral vector, or a lentiviral vector.

The AAV vector may be an AAV vector particle. In some embodiments the AAV vector particle comprises AAV3B capsid proteins, LK03 capsid proteins, or AAV9 capsid proteins. Preferably, the AAV vector particle comprises AAV3B capsid proteins.

The podocyte-specific promoter is a promoter selected from a NPHS1 promoter and a NPHS2 promoter. In embodiments not part of the claimed invention the podocyte-specific promoter may be a promoter selected from a WT1 promoter, a FOXC2 promoter, a ABCA9 promoter, a ACPP promoter, a ACTN4 promoter, a ADM promoter, a ANGPTL2 promoter, a ANXA1 promoter, a ASB15 promoter, a ATP8B1 promoter, a B3GALT2 promoter, a BB014433 promoter, a BMP7 promoter, a C1QTNF1 promoter, a CAR13 promoter, a CD2AP promoter, a CD55 promoter, a CD59A promoter, a CD59B promoter, a CDC14A promoter, a CDH3 promoter, a CDKN1B promoter, a CDKN1C promoter, a CEP85L promoter, a CLIC3 promoter, a CLIC5 promoter, a COL4A1 promoter, a COL4A2 promoter, a COL4A3 promoter, a COL4A4 promoter, a COL4A5 promoter, a COLEC12 promoter, a CRIM1 promoter, a CST12 promoter, a DEGS1 promoter, a DOCK4 promoter, a DOCK5 promoter, a EGF promoter, a ENPEP promoter, a EPHX1 promoter, a FAM81A promoter, a FAT1 promoter, a FGFBP1 promoter, a FOXD1 promoter, a FRYL promoter, a GABRB1 promoter, a GALC promoter, a GM10554 promoter, a H2-D1 promoter, a H2-Q7 promoter, a H2BC4 promoter, a H3C15 promoter, a HS3ST3A1 promoter, a HTRA1 promoter, a IFNGR1 promoter, a IL18 promoter, a ILDR2 promoter, a ITGB5 promoter, a ITGB8 promoter, a KIRREL promoter, a LAMA1 promoter, a LAMA5 promoter, a LAMB1 promoter, a LAMB2 promoter, a LMX1B promoter, a MAFB promoter, a MAGI2 promoter, a MELA promoter, a MERTK promoter, a MGAT4A promoter, a MYO1D promoter, a MYO1E promoter, a MYOM2 promoter, a MYZAP promoter, a NEBL promoter, a NES promoter, a NOD1 promoter, a NPR3 promoter, a NR2F2 promoter, a NUPR1 promoter, a OPTN promoter, a P3H2 promoter, a PAK1 promoter, a PARD3B promoter, a PDPN promoter, a PLAT promoter, a PLCE1 promoter, a PLSCR2 promoter, a PODXL promoter, a PROS1 promoter, a PTPRO promoter, a RAB3B promoter, a RDH1 promoter, a RDH9 promoter, a SDC4 promoter, a SEMA3E promoter, a SERPINB6B promoter, a SH3BGRL2 promoter, a SLC41A2 promoter, a SLCO2A1 promoter, a ST3GAL6 promoter, a SYNPO promoter, a TDRD5 promoter, a THSD7A promoter, a TIMP3 promoter, a TJP1 promoter, a TLR7 promoter, a TM4SF1 promoter, a TMEM108 promoter, a TMEM54 promoter, a TMTC1 promoter, a TOP1MT promoter, a TRAV10 promoter, a TRAV10N promoter, a TRAV5-4 promoter, a TSHB promoter, a UACA promoter, a UBA1Y promoter, a UPRT promoter, a VEGFA promoter, a VTCN1 promoter, a ZBTB20 promoter, and a 5730407I07RIK promoter, or a fragment of derivative thereof. Preferably, the podocyte-specific promoter is selected from a NPHS1 promoter, a NPHS2 promoter, a WT1 promoter, a FOXC2 promoter, a ACTN4 promoter, a BMP7 promoter, a CD2AP promoter, a CDH3 promoter, a CDKN1B promoter, a CDKN1C promoter, a COL4A1 promoter, a COL4A2 promoter, a COL4A3 promoter, a COL4A4 promoter, a COL4A5 promoter, a CRIM1 promoter, a FAT1 promoter, a FOXD1 promoter, a KIRREL promoter, a LAMA1 promoter, a LAMA5 promoter, a LAMB1 promoter, a LAMB2 promoter, a LMX1B promoter, a MAFB promoter, a NES promoter, a NR2F2 promoter, a PODXL promoter, a PTPRO promoter, a SYNPO promoter, a TJP1 promoter, and a VEGFA promoter, or a fragment of derivative thereof. Preferably, the podocyte-specific promoter is a minimal NPHS1 promoter or a minimal NPHS2 promoter.

The complement protein is an inhibitor of the complement system. The inhibitor of the complement system may be selected from the list consisting of CFI, CFH, FHL-1, C1INH, C4BP, CD55, CD35, CD46, CD59, vitronectin, and clusterin, or fragments or derivatives thereof. Preferably, the inhibitor of the complement system is CFI, CFH, or FHL-1, or a fragment or derivative thereof.

The nucleotide sequence encoding a complement protein may be operably linked to a Woodchuck hepatitis post-transcriptional regulatory element (WPRE), a polyadenylation signal, and/or a Kozak sequence.

In another aspect, the present invention provides an isolated cell comprising the viral vector of the present invention.

In another aspect, the present invention provides a pharmaceutical composition comprising the viral vector or the isolated cell of the present invention, in combination with a pharmaceutically acceptable carrier, diluent or excipient.

In another aspect, the present invention provides the viral vector, the isolated cell, or the pharmaceutical composition of the present invention for use as a medicament.

In a related aspect, the present invention provides for use of the viral vector, the isolated cell, or the pharmaceutical composition of the present invention for the manufacture of a medicament.

In a related aspect, the present invention provides a method comprising administering the viral vector, the isolated cell, or the pharmaceutical composition of the present invention to a subject in need thereof.

In another aspect, the present invention provides the viral vector, the isolated cell, or the pharmaceutical composition of the present invention for use in preventing or treating a complement-mediated kidney disease.

In a related aspect, the present invention provides for use of the viral vector, the isolated cell, or the pharmaceutical composition of the present invention for the manufacture of a medicament for preventing or treating a complement-mediated kidney disease.

In a related aspect, the present invention provides a method of preventing or treating a complement-mediated kidney disease comprising administering the viral vector, the isolated cell, or the pharmaceutical composition of the present invention to a subject in need thereof.

The complement-mediated kidney disease may be IgA nephropathy, C3 glomerulopathy, atypical hemolytic uremic syndrome (aHUS), stx-associated HUS, lupus nephritis, cryoglobulinemia, anti-GBM disease, ANCA-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III. Preferably, the complement-mediated kidney disease is IgA Nephropathy or C3 glomerulopathy, preferably wherein the C3 glomerulopathy is dense deposit disease or C3 glomerulonephritis.

The viral vector, the isolated cell, or the pharmaceutical composition of the present invention may be administered to a human subject. The viral vector, the isolated cell, or the pharmaceutical composition of the present invention may be administered systemically and/or by intravenous injection. The viral vector, the isolated cell, or the pharmaceutical composition of the present invention may be administered by injection into the renal artery or by ureteral or subcapsular injection.

### DESCRIPTION OF DRAWINGS

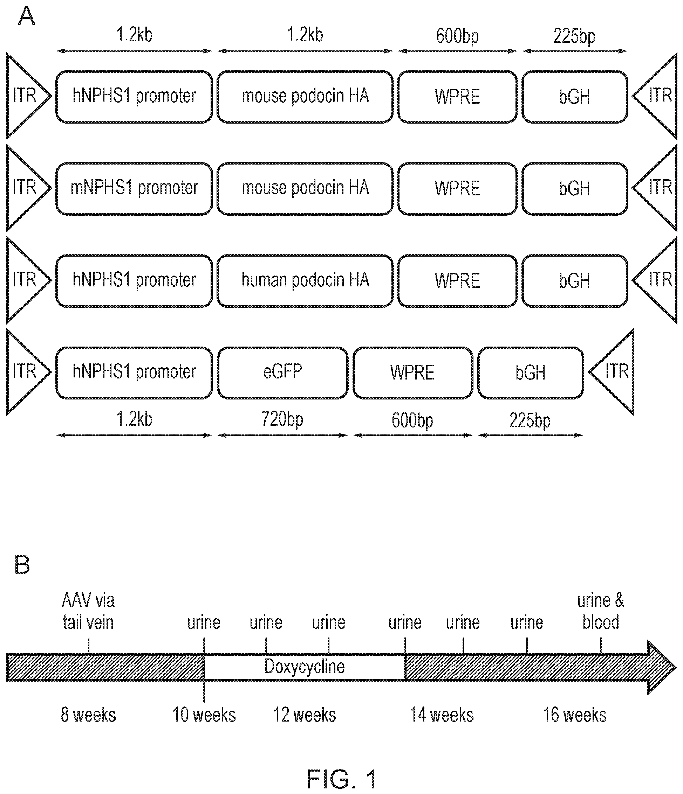

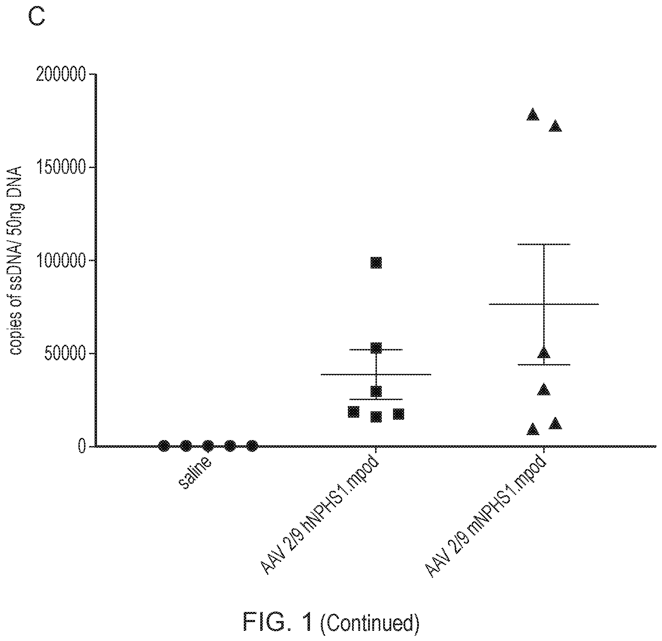

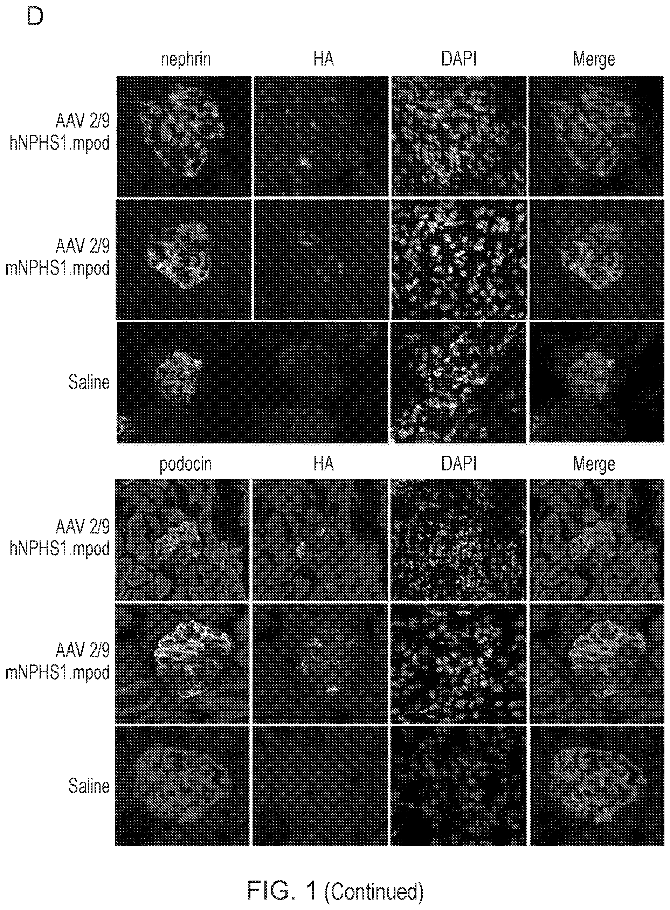

**Figure 1** - **AAV 2/9 administered by tail vein injection transduces the kidney and expresses HA-tagged podocin in the podocyte.**
   **A)** AAV vectors used to express mouse or human podocin or GFP. All vectors contained the Kozak sequence between the promoter and the transgene, as well as WPRE (Woodchuck hepatitis post-transcriptional regulatory element) and the bovine growth hormone (bGH) polyadenylation signal. **B)** Vector or saline was injected via tail vein in iPod NPHS2^{fl/fl} mice at 8 weeks of age, and induction with doxycycline commenced 10-14 days later. **C)** qPCR showing presence of AAV ITRs in mouse kidney cortex in mice injected with the viral vector. **D)** Representative immunofluorescence showing expression of HA tagged podocin with podocyte-specific proteins nephrin and podocin in iPod NPHS2^{fl/fl} mice injected with AAV 2/9. Control (saline) images are of mice without the full iPod NPHS2^{fl/fl} genotype injected and hence did not develop proteinuria or diseased glomeruli, as mice with diseased glomeruli showed loss of podocyte markers.

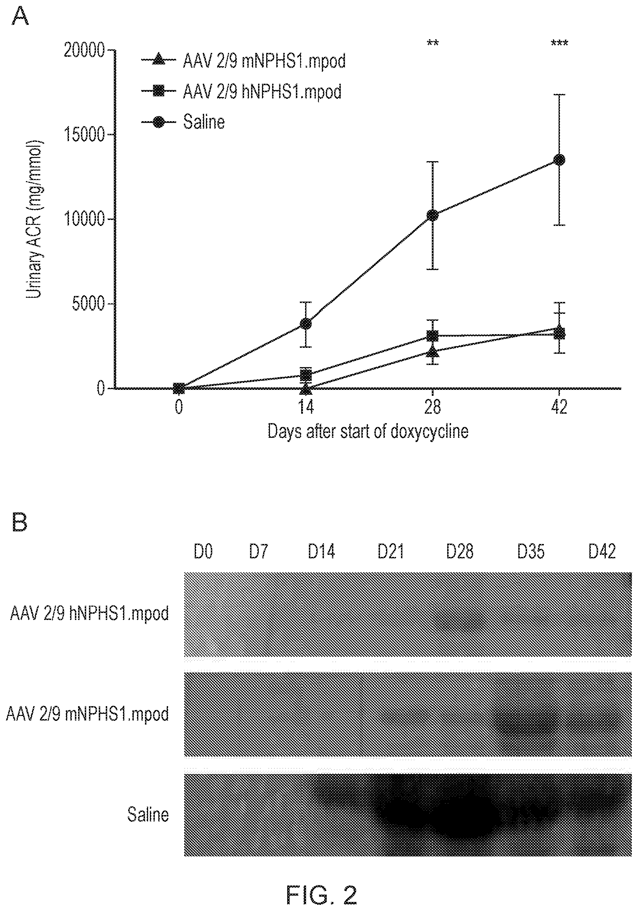

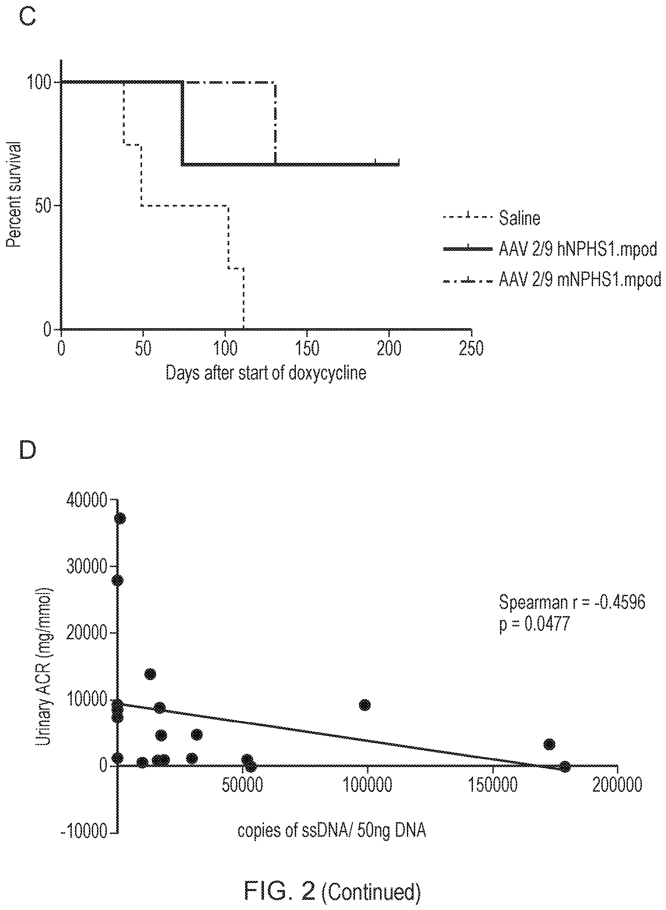

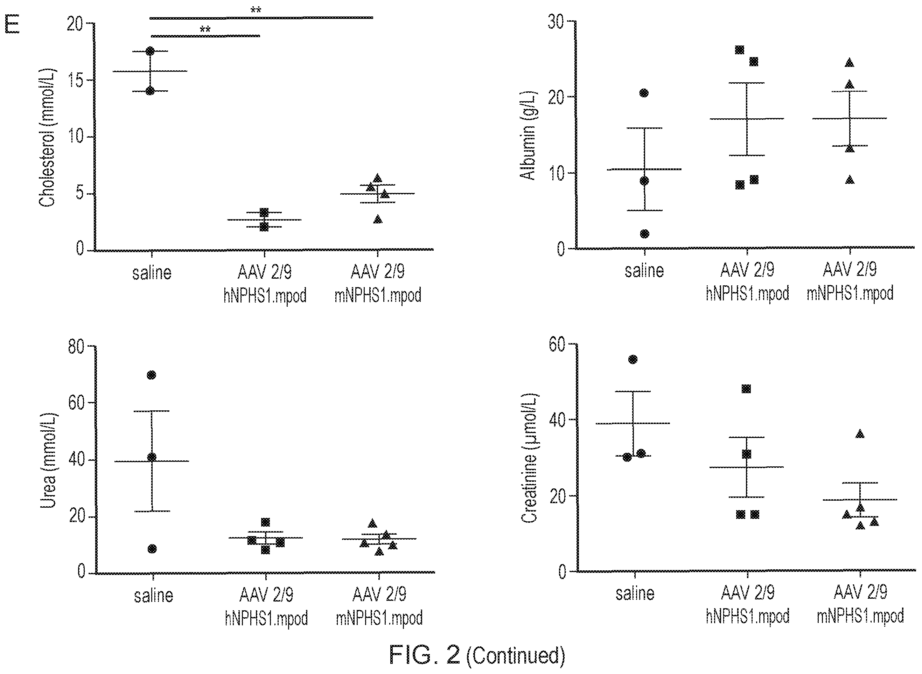

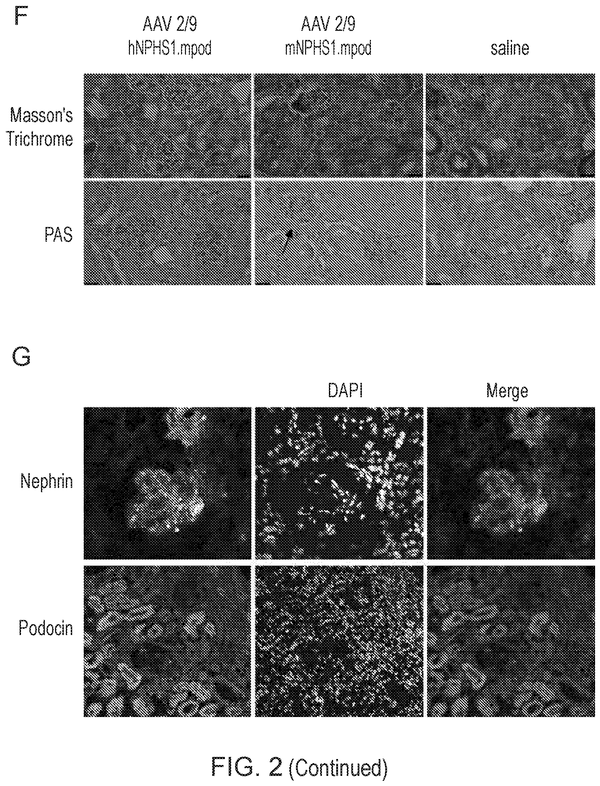

**Figure 2** - **tail vein injection of AAV 2/9 expressing wild-type podocin under a podocyte-specific promoter ameliorates proteinuria in the conditional podocin knockout mouse model (iPod NPHS2^{fl/fl})**
   **A)** Urinary albumin:creatinine ratio of mice injected with AAV 2/9 mNPHS1.mpod versus AAV 2/9 hNPHS1.mpod versus saline (n=9 in each group, **p<0.01 ***p<0.001). **B)** Coomassie staining showing representative images of degree of albuminuria in one mouse from each experimental group. The saline group showed proteinuria from day 14 onwards and showed a large amount of albumin while the vector treated groups showed later onset of albuminuria and milder albuminuria. **C)** Survival curve showing improved survival in mice injected with either AAV 2/9 hNPHS1.mpod or AAV 2/9 mNPHS1.mpod (Log-rank (Mantel-Cox) test p=0.049, n=3 in each virus group and n=4 in the saline group). **D)** The number of copies of viral DNA per 50ng total DNA has an inverse correlation with urinary albumin:creatinine ratio at day 42 (Spearman r= -0.4596, p=0.0477) **E)** Blood results including cholesterol, albumin, urea, and creatinine at 6 weeks post-doxycycline. (n=minimum of 3 mice in each group except for cholesterol with minimum of n=2 in each group) **F)** Histology showing representative images from each group on light microscopy. Saline injected group showed glomerular hypertrophy, increased collagen deposition and segmental sclerosis, along with tubular dilatation, consistent with FSGS. Those injected with AAV 2/9 expressing mouse podocin exhibited a range of histological findings which roughly correlated with their urine albumin:creatinine ratio at death. Some mice had healthy normal glomeruli, while others showed mild evidence of disease like pseudo-crescent formation (arrow) seen in the mouse injected with AAV 2/9 mNPHS1.mpodHA. **G)** iPod NPHS2^{fl/fl} mice injected with saline showed loss of podocin, while nephrin expression showed a change from predominantly membranous staining to a diffuse pattern.

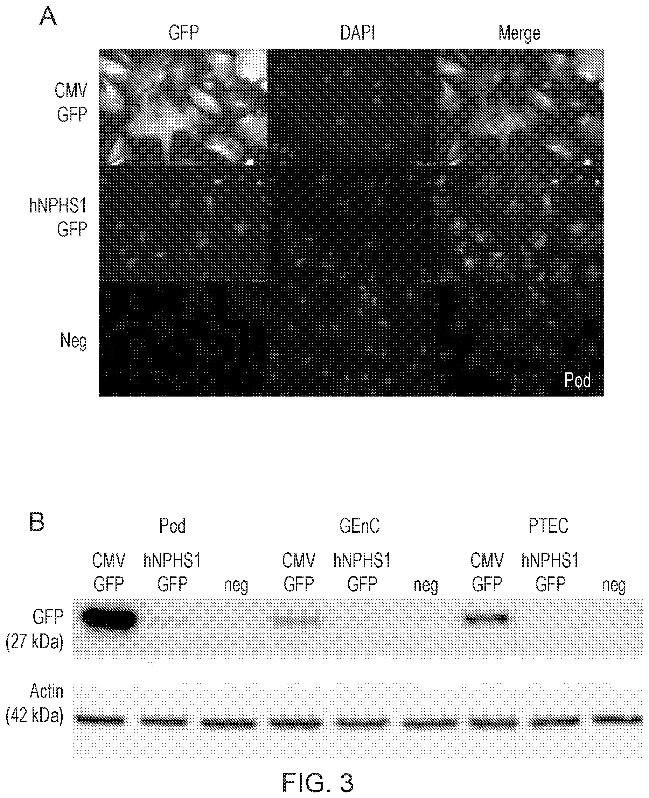

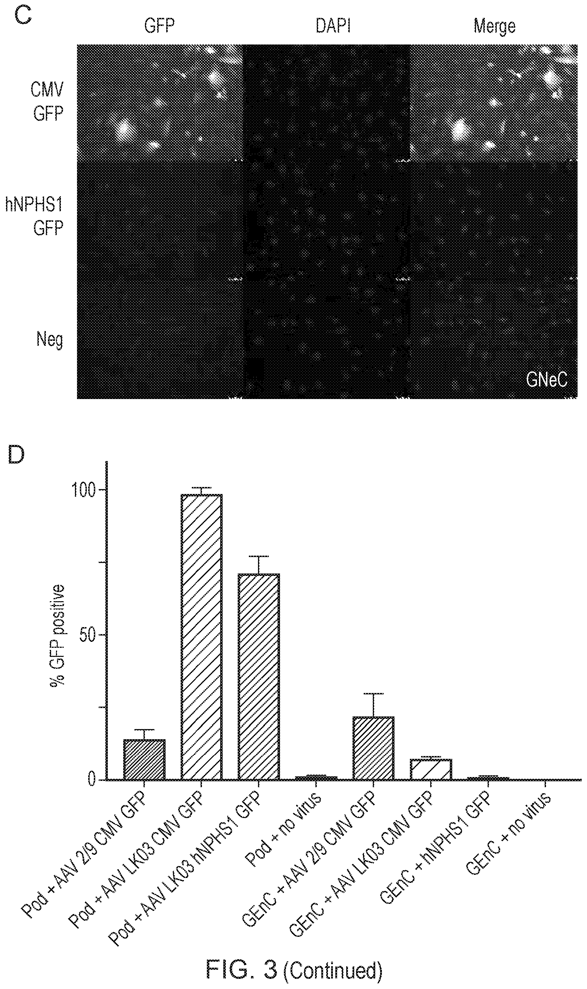

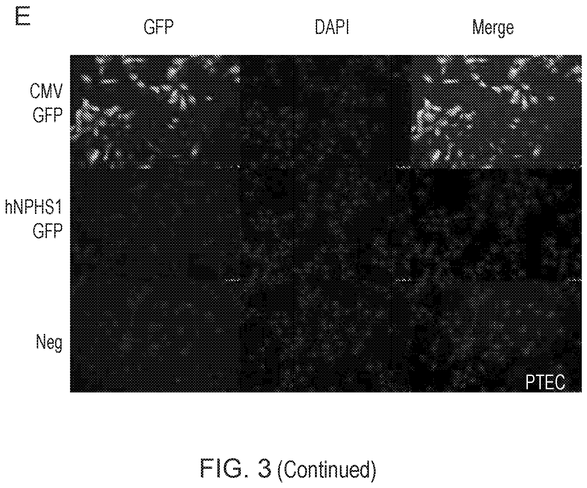

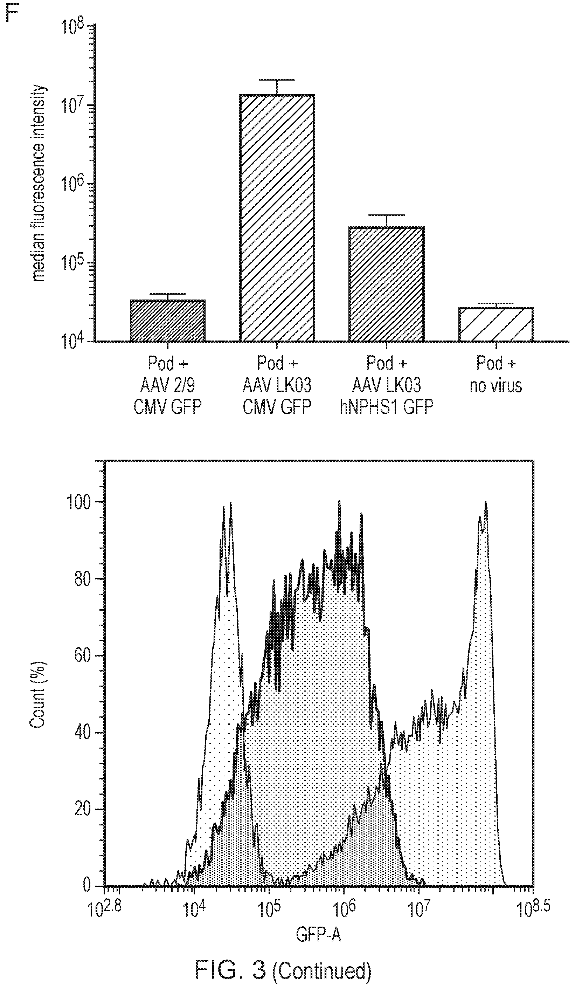

**Figure 3** - **AAV LK03 shows efficient transduction of human podocytes in vitro with the minimal human nephrin promoter.**
   **A, C, E)** immunofluorescence demonstrating transduction of human podocytes (Pod), glomerular endothelial cells (GEnC) and proximal tubule epithelial cells (PTEC) by AAV LK03 CMV GFP, with only expression of GFP in podocytes when using the minimal nephrin promoter AAV LK03 hNPHS1 GFP. **B)** Western blot demonstrating GFP expression in podocytes only when using the minimal human nephrin promoter using AAV LK03. **D)** Flow cytometry demonstrating highly efficient transduction of podocytes using AAV LK03 CMV GFP, and confirming the GFP expression using the minimal nephrin promoter was only seen in podocytes. In comparison, AAV 2/9 CMV GFP showed low transduction efficiencies in podocytes (n=3) **F)** Bar chart showing median fluorescence intensity in podocytes transduced with AAV LK03 and histogram showing the degree of green fluorescence in podocytes transduced with AAV LK03 CMV GFP (right-hand peak), AAV LK03 hNPHS1 GFP (central peak) and untransduced cells (left-hand peak).

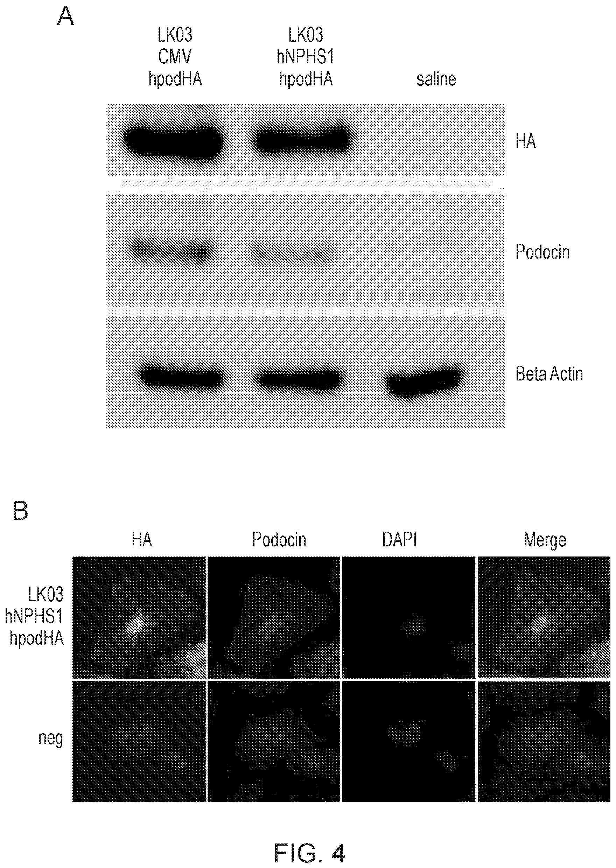

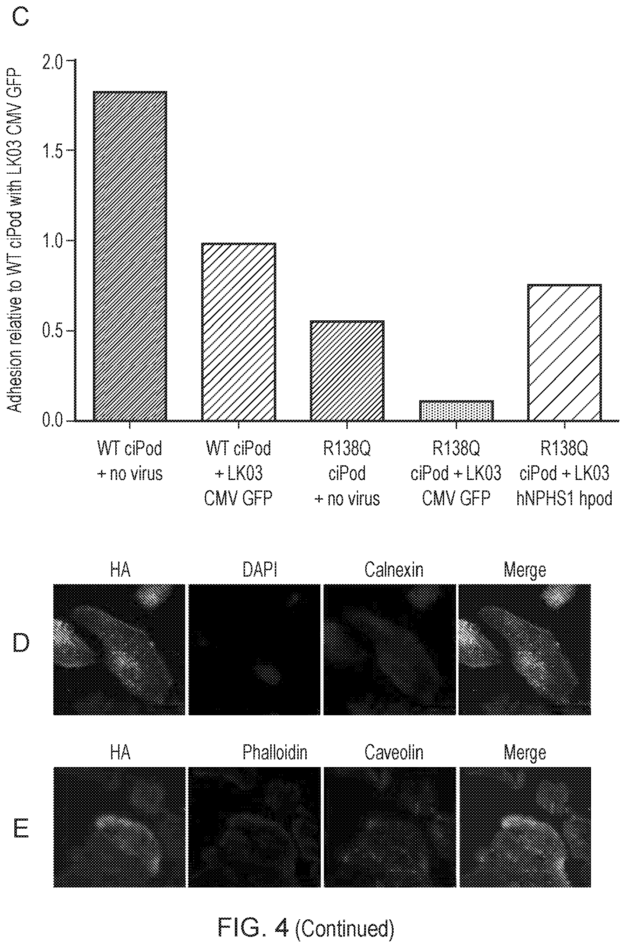

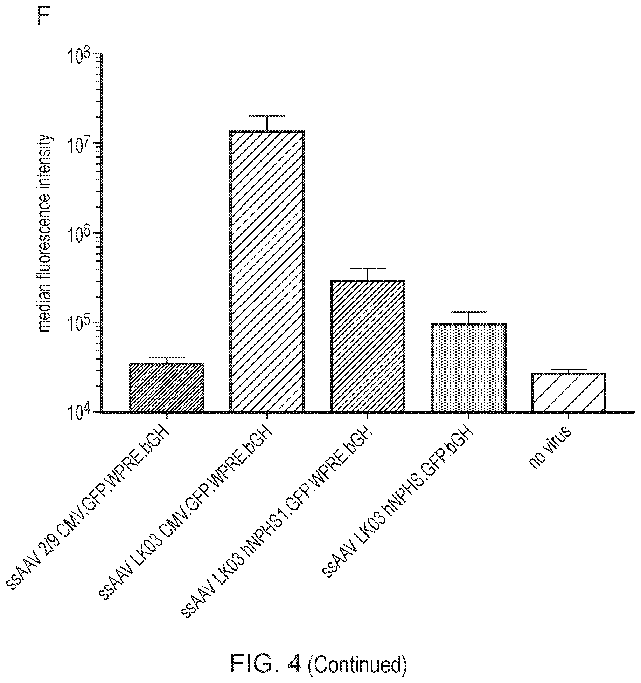

**Figure 4** - **AAV LK03 expressing wild type human podocin shows functional rescue in the mutant podocin R138Q podocyte cell line.**
   **A)** Western blot showing AAV LK03.CMV.hpodocinHA and AAV LK03.hNPHS1.hpodocinHA transduces R138Q podocytes and expresses HA-tagged podocin. **B)** Immunofluorescence demonstrating expression of HA tagged wild type podocin in the mutant podocin R138Q podocytes. **C)** Adhesion assay showing a decrease in adhesion in mutant podocin R138Q podocytes, with rescue of adhesion in R138Q podocytes treated with AAV LK03.hNPHS1.hpodHA.WPRE.bGH. **D)** confocal microscopy showing HA tagged podocin does not colocalize with calnexin, an endoplasmic reticulum marker **E)** TIRF microscopy demonstrating expression of HA-tagged podocin within 100nm of the plasma membrane with some colocalisation with caveolin, a lipid raft marker. **F)** GFP expression in human podocytes *in vitro,* determined by flow cytometry. Constructs which were assayed included: ssAAV 2/9 CMV.GFP.WPRE.bGH, ssAAV LK03 CMV.GFP.WPRE.bGH, ssAAV LK03 hNPHS1.GFP.WPRE.bGH, and ssAAV.LK03 hNPHS1.GFP.bGH.
**Figure 5** - **Human podocytes transduced with AAV LK03**
   Human podocytes transduced with either HAVDR **(A)** or HASmad7 **(B)** using AAV LK03 with the minimal human nephrin promoter.
**Figure 6** - **Complement proteins C3 and CFH are expressed and secreted by unstimulated human podocytes (HPC)**
   **(A)** Detection of PCR products for C3 (320 bp) and CFH (783 bp) in conventional reverse transcriptase PCR compared to -RT control (-RT = control of cDNA without addition of RT, bp = basepairs). **(B)** Protein expression for C3 and CFH was determined by Western blot from whole cell podocyte lysate (HPC) and cell culture supernatant after 24 hours in serum-free media (SFM). Normal human serum (NHS, 1:1000) and recombinant CFH or C3 were used as positive controls (rC3/rCFH, 1:1000), SFM was used as negative control. The CFH gene has two protein products: CFH and factor H like-protein 1 (FHL-1). Both are detected in this western blot in the podocyte cell lysate, in NHS and to a small extend in the cell culture supernatant (representative Western blots, n = 4). **(C)** Protein expression of C3 and **(D)** CFH was confirmed in immunofluorescence on the surface of non-permeabilized cultured podocytes compared to isotype negative control (Neg-Ctrl) **(E).** (n = 3, C3 and CFH = green, nucleus = blue, scale bar 50 µm, 40x). **(F)-(J)** Detection of PCR products for: **(F)** C1q, C1r, C1s; **(G)** C2 and C4; **(H)** C5; **(I)** Factor B, Factor D, Properdin; and **(J)** CD55, CD59, CD46 in conventional reverse transcriptase PCR compared to -RT control (-RT = control of cDNA without addition of RT, bp = basepairs).

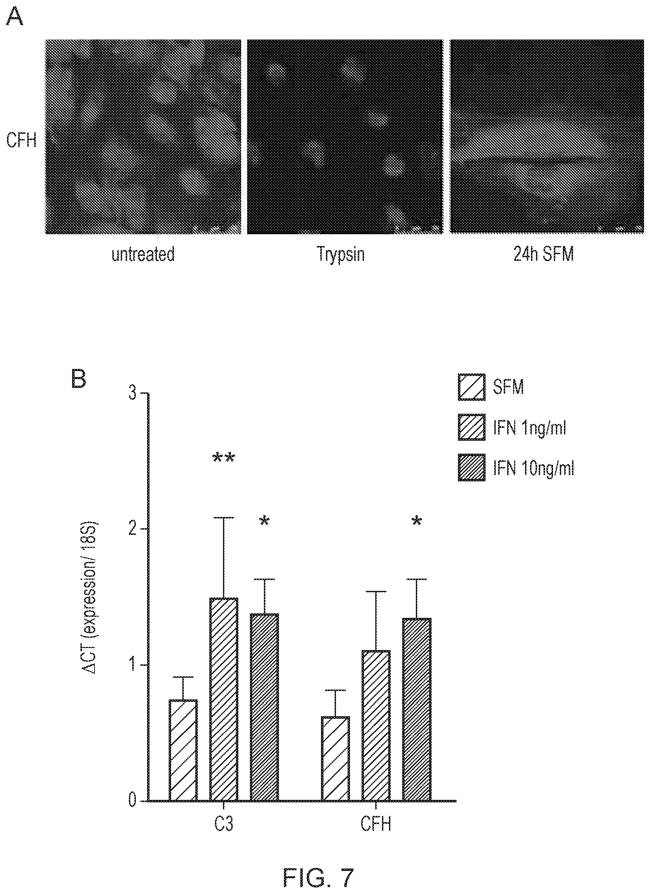

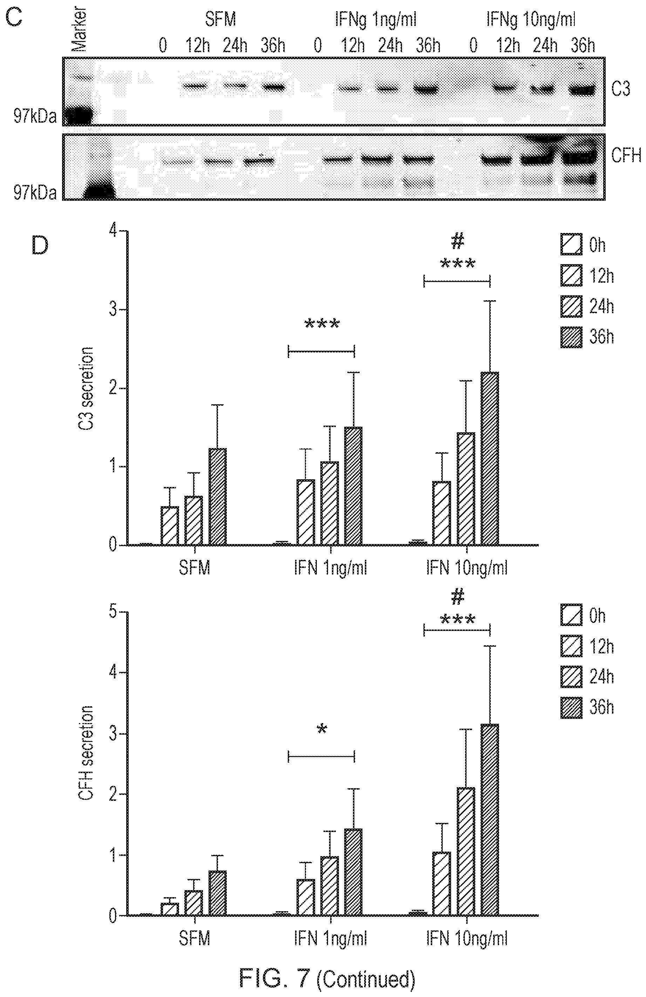

**Figure 7** **- Production and secretion of C3 and CFH is an active and inducible process.**
   **(A)** CFH was removed from the surface of cultured podocytes using low dose trypsin (10 µg/ml, 3 minutes), the cells were then incubated in SFM for 24 hours and the expression of CFH on the cell surface is determined in immunofluorescence staining. The pictures are showing the detection of CFH (in red) before treatment with trypsin (left), immediately after the treatment (middle) and after 24 hours recovery in SFM (right) (n = 3, DAPI = blue, 60x, scale bar 25 µm). **(B-E)** Expression and secretion of C3 and CFH was enhanced by interferon γ (IFNg). **(B)** mRNA expression of C3 and CFH in human podocytes incubated 6 hours with SFM (white bars), IFNg 1 ng/ml (grey bars) and IFNg 10 ng/ml (black bars) (* p<0.05, ** p<0.001 compared to expression in SFM) **(C)** Western blot against C3 (above) and CFH (below) of supernatant from podocytes incubated in SFM, IFNg 1 ng/ml or IFNg 10 ng/ml for 0-36 hours. Quantitative analysis of Western blots for the secretion of C3 **(D)** and CFH **(E)** from podocytes incubated in SFM, IFNg 1 and 10 ng/ml for 0 (white bars), 12 (light grey bars), 24 (dark grey) and 36 (black bars) hours (* p<0.05 and *** p<0.001 compared to secretion with SFM, ^{#} p<0.05 and ^{##} p<0.01 compared to incubation with IFNg 1 ng/ml) Data are shown in mean and SD. Mann Whitney U test, n=5 independent experiments in **(B), (D)** and **(E).**

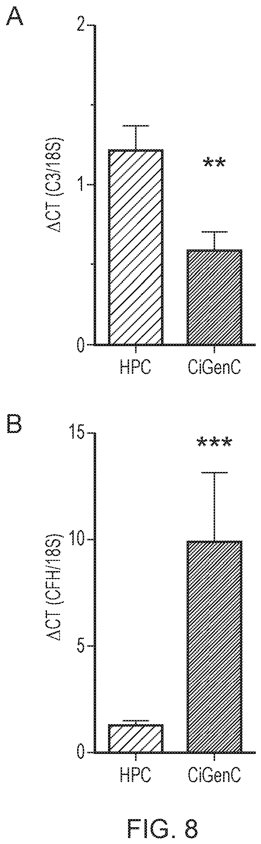

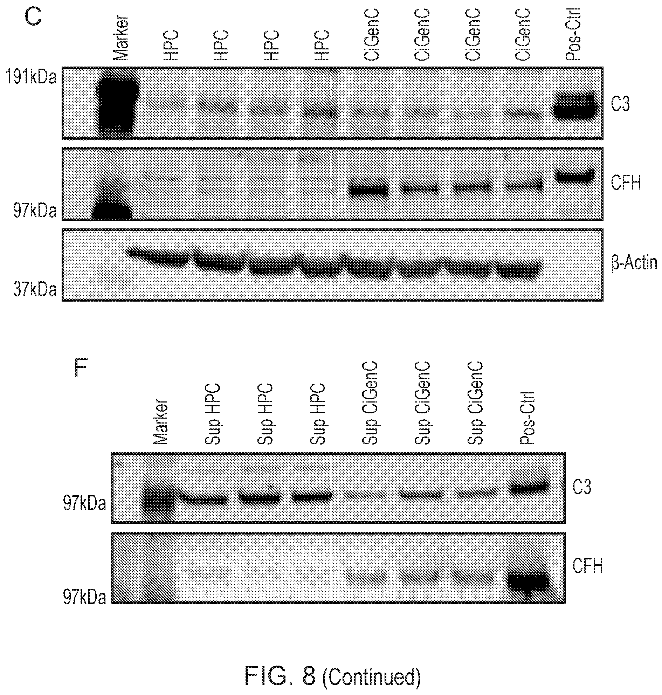

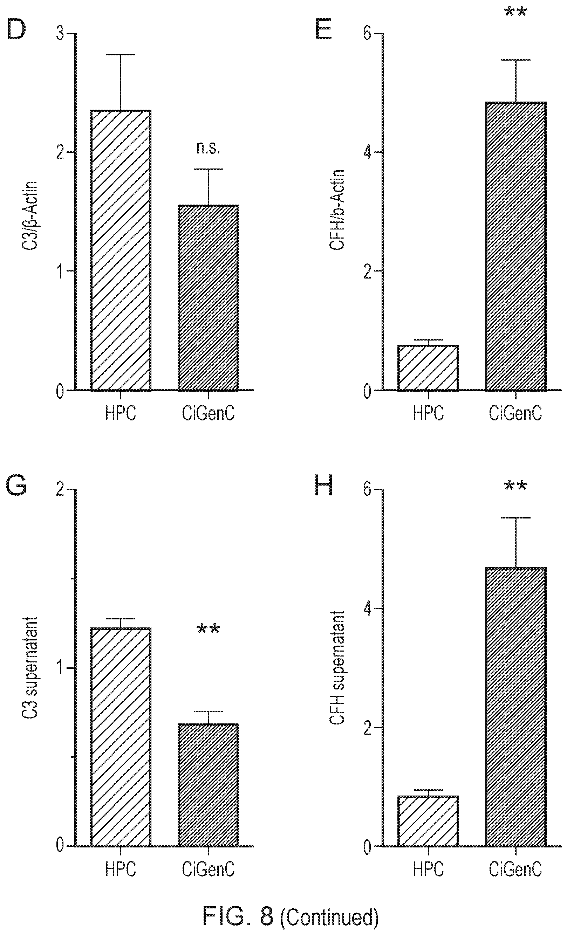

**Figure 8** - **Expression of complement factor C3 and CFH is different in cultured podocytes and glomerular endothelial cells.**
   Conditionally immortalized human podocytes (HPC) and human glomerular endothelial cells (CiGenC) were compared for their expression of C3 and CFH on mRNA- and protein-level and secretion of both proteins. **(A)** mRNA-expression of C3/18S and **(B)** CFH/18S in HPC (white bars) and CiGenC (black bars) (n = 12). Protein expression of C3 and CFH was analyzed in Western blot **(C)** of whole cell lysates. Densitometry of C3 **(D)** and CFH **(E)** in HPC (white bars) and CiGenC (black bars) normalized to expression of beta (b)-actin (n = 6). Secretion of C3 and CFH into cell culture supernatant was tested in similar numbers of seeded cells and analyzed in Western Blot **(F).** Western blot densitometry of C3 **(G)** and CFH **(H)** from HPC-supernatants (white bars) and CiGenC-supernatants (black bars) (n = 6) Recombinant C3 and CFH were used as positive controls (Pos-Ctrl) in **(C)** and **(F).** Data (relative expression) are shown in mean and SD in **(A+B), (D+E)** and **(G+H),** ** p<0.01, *** p<0.001, Mann Whitney U test)

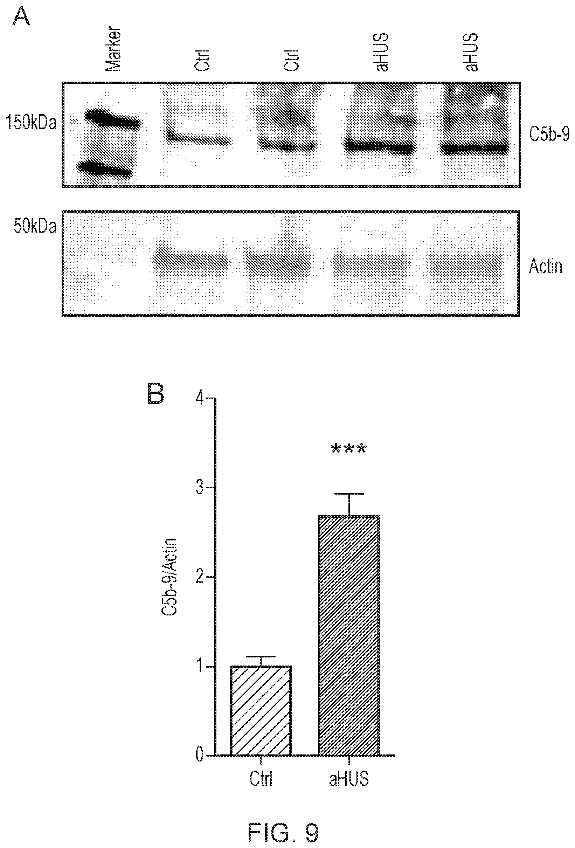

**Figure 9** - **Podocyte-secreted CFH is functionally active**
   **(A)** Western blot against C5b-9 on normal human podocytes (Ctrl) and aHUS patient's podocytes. **(B)** After complement activation aHUS podocytes (black bar) showed increased C5b-9 deposits compared to control cells (white bar) (relative deposition of C5b-9 related to actin, *** p<0.001, Mann Whitney U test, n=4 independent experiments.

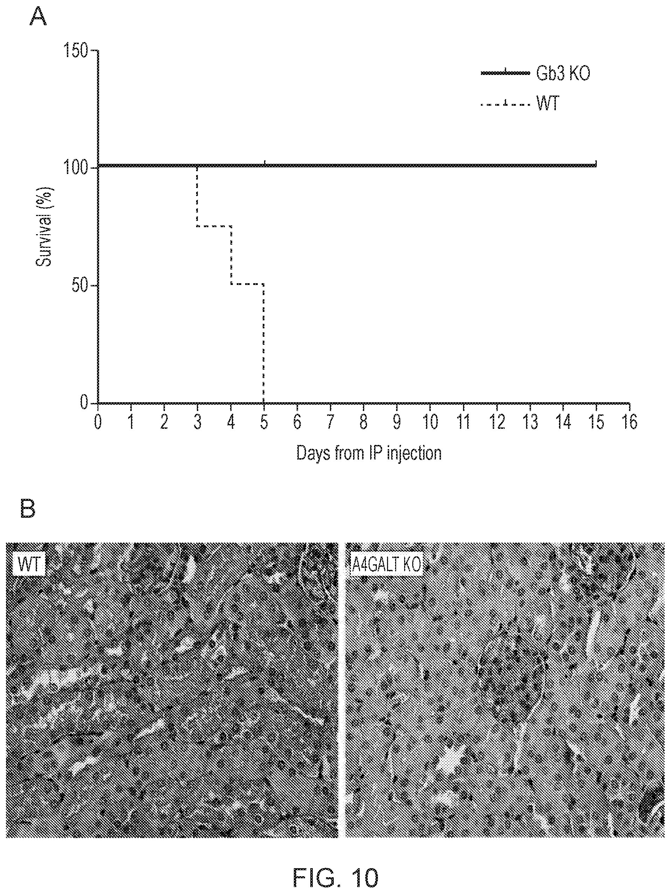

**Figure 10** - **Gb3 synthase KO mice do not develop HUS phenotype when injected with Shiga Toxin (10 ng/g)**
   **(A)** Survival rate of mice injected with 10 ng/g of Shiga toxin. Dotted line = WT mice; solid line = Gb3 synthase KO mice. **(B)** Histological samples from mice injected with 10 ng/g of Shiga toxin. WT mice showed clear evidence of acute tubular necrosis with oedematous tubules and vacuolations. Gb3 synthase KO mice (A4GALT KO mice) showed no changes in the tubular or glomerular morphology.

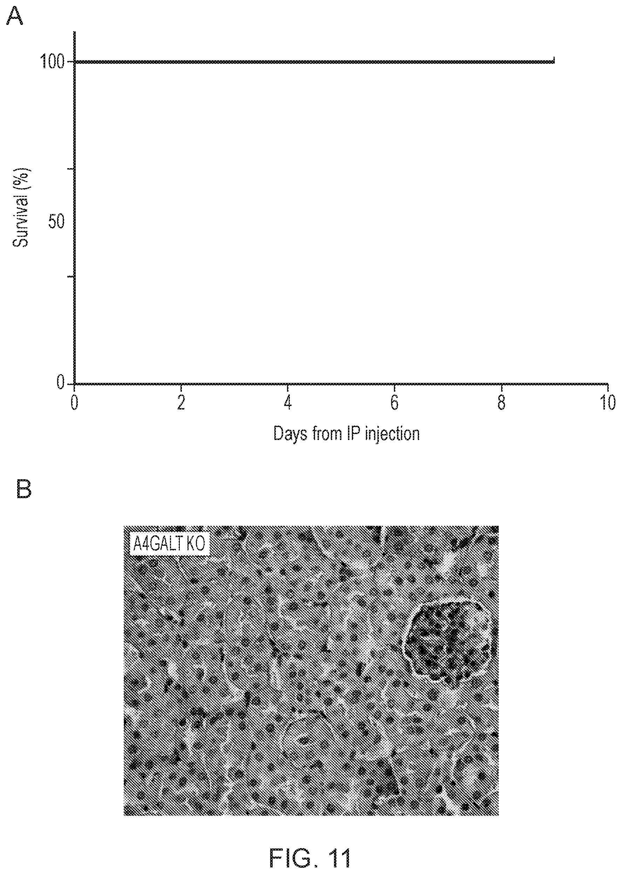

**Figure 11** - **Gb3 synthase KO mice do not develop HUS phenotype when injected with Shiga Toxin (100 ng/g)**
   **(A)** Survival rate of mice injected with 100 ng/g of Shiga toxin. Solid line = Gb3 synthase KO mice. **(B)** Histological samples from mice injected with 100 ng/g of Shiga toxin. Gb3 synthase KO mice (A4GALT KO mice) showed no changes in the tubular or glomerular morphology.

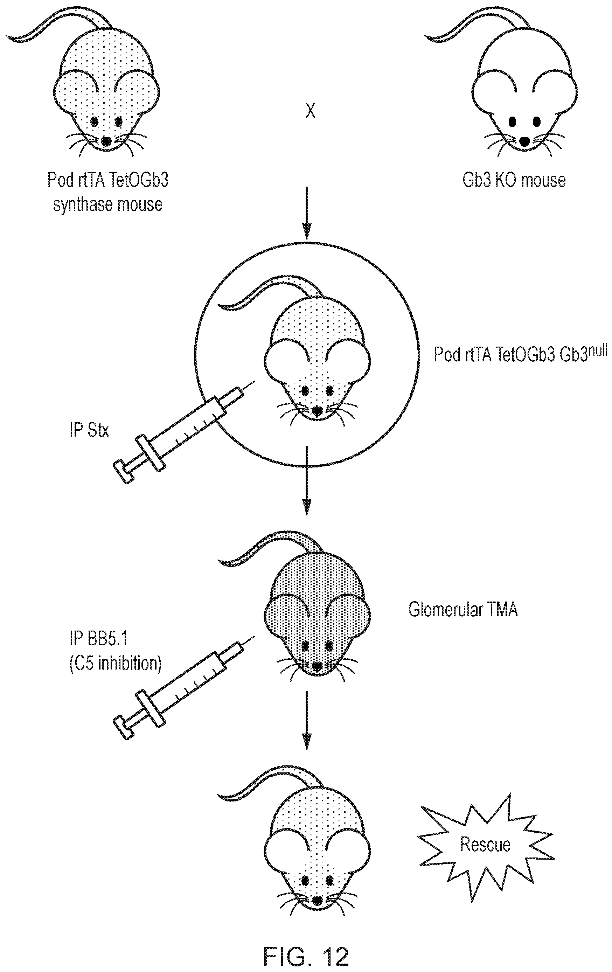

**Figure 12** - **Mouse model of STEC-HUS and rescue with complement inhibitor**
   Gb3 KO mice and were crossed with podocyte Gb3 expressing mice (Pod rtTA TetOGb3 synthase mice) to produce podocyte Gb3 expressing mice on Gb3 null background (Pod rtTA TetOGb3 Gb3^{null} mice). These mice were then injected with Shiga toxin (Stx). Following development of HUS symptoms (including glomerular thrombic microangiopathy) mice were injected with BB5.1 (a C5 inhibitor) which rescued the HUS phenotype.

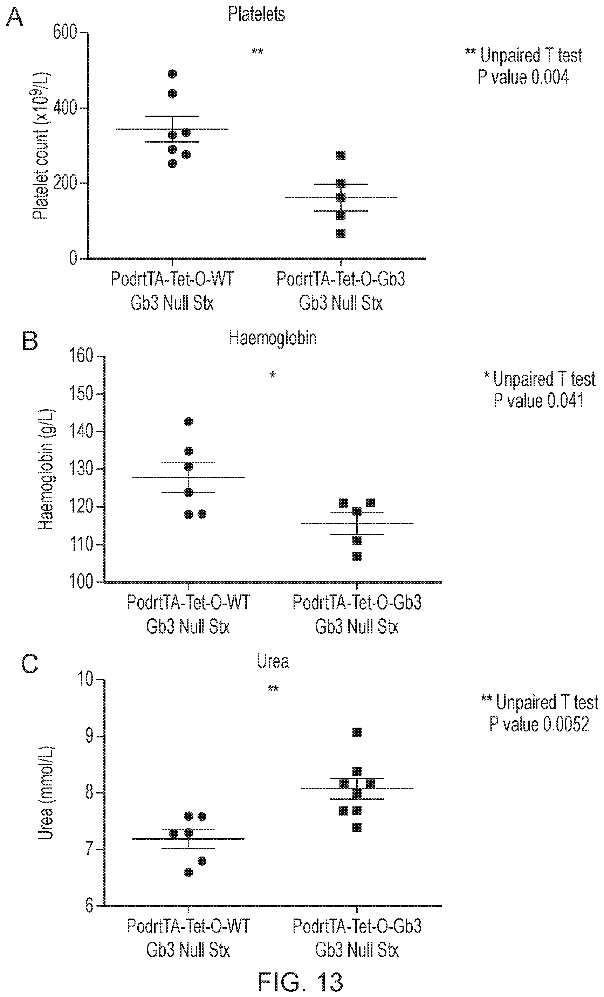

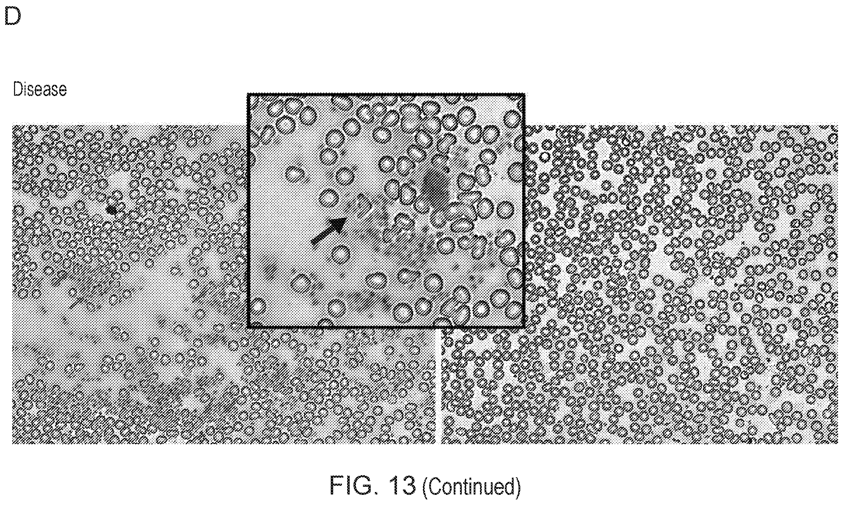

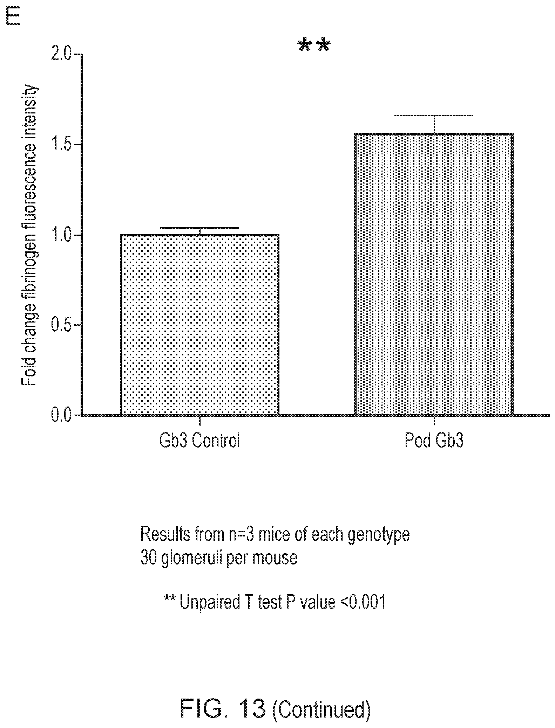

**Figure 13** - **Podocyte Gb3 expressing mice on Gb3 null background develop HUS phenotype when given IP Shiga toxin**
   Podocyte Gb3 expressing mice on Gb3 null background were injected with Shiga toxin (10 ng/g) and HUS phenotype was determined: **(A)** platelet counts (day 10); **(B)** haemoglobin levels (day 10); **(C)** plasma urea concentration (pooled days 12-16); **(D)** blood films analysis (day 10, x40); **(E)** fibrinogen levels (N=3, 30 glomeruli per mouse).

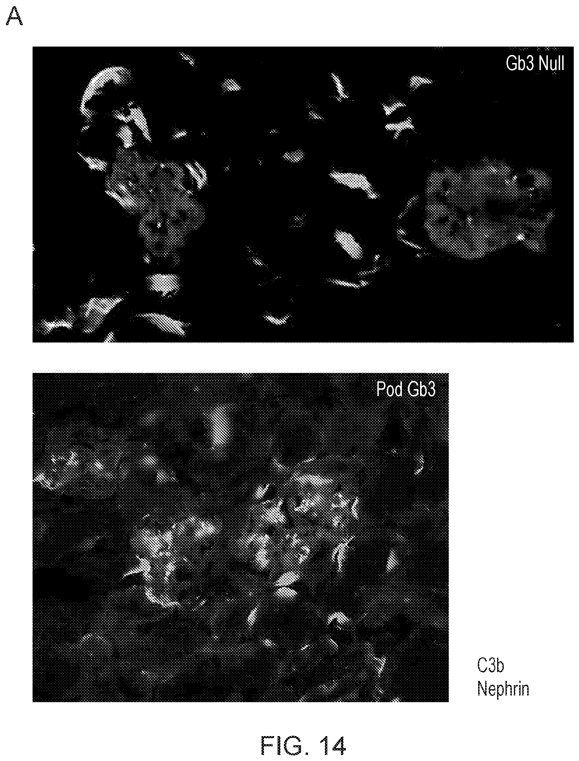

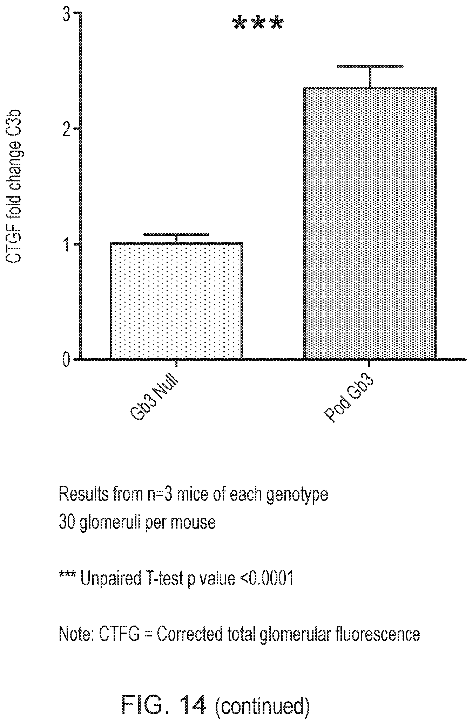

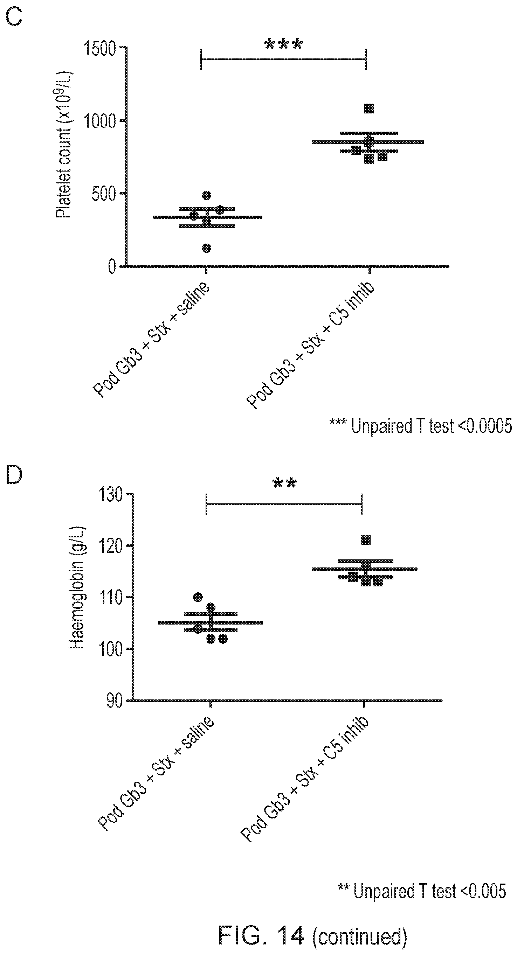

**Figure 14** - **Complement inhibitor rescue of the HUS phenotype**
   Podocyte Gb3 expressing mice on Gb3 null background were injected with Shiga toxin (10 ng/g). **(A)** Fluorescence overlay of C3b (green) and nephrin (red) in podocytes. **(B)** C3b fluorescence (N=3, 30 glomeruli per mouse). Subsequently, Pod rtTA TetOGb3 Gb3^{null} mice were injected with Shiga toxin on day 0, and on day 7, the mice were injected with saline, or BB5.1 (C5 inhibitor) and HUS phenotype was determined: **(C)** platelet counts; **(D)** haemoglobin levels.

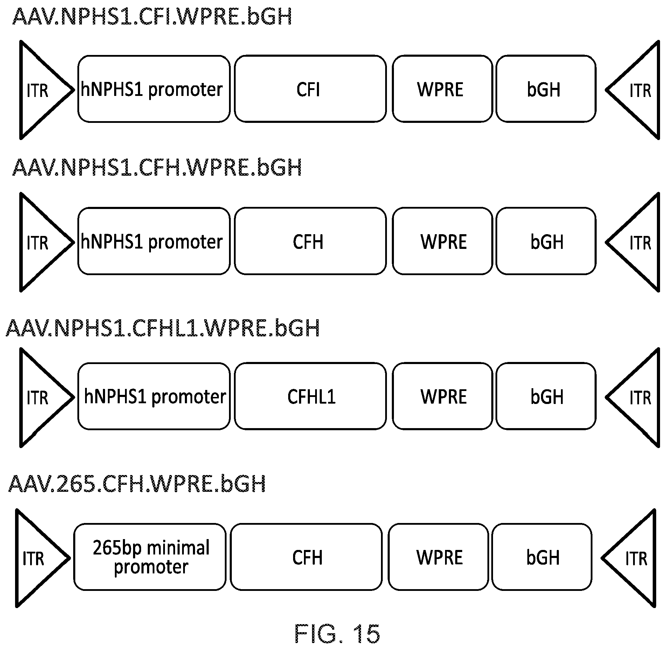

**Figure 15** - **Exemplary podocyte-targeted AAVs encoding a complement inhibitor**
   Exemplary AAV constructs which are capable of transducing podocytes and inducing expression and secretion of complement inhibitors from the podocytes. The AAV constructs may be packaged with AAV3B, LK03, or AAV9 serotypes to effectively transduce podocytes.

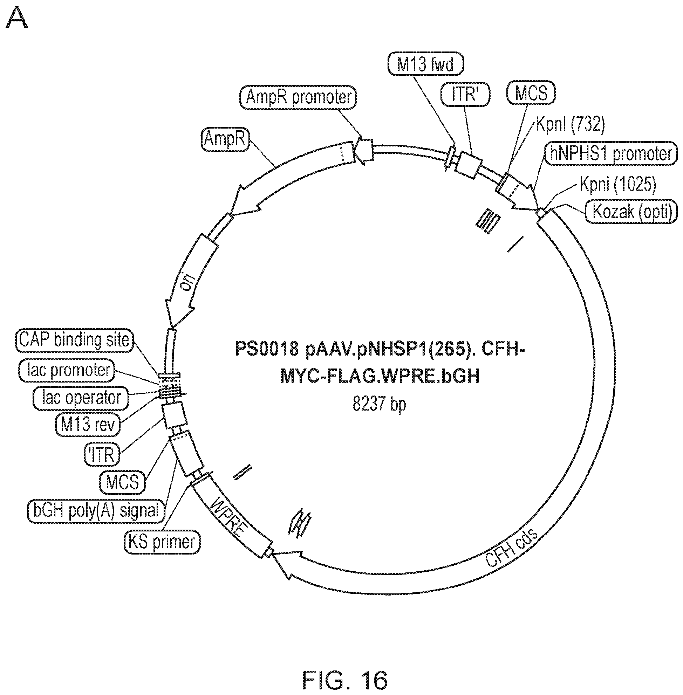

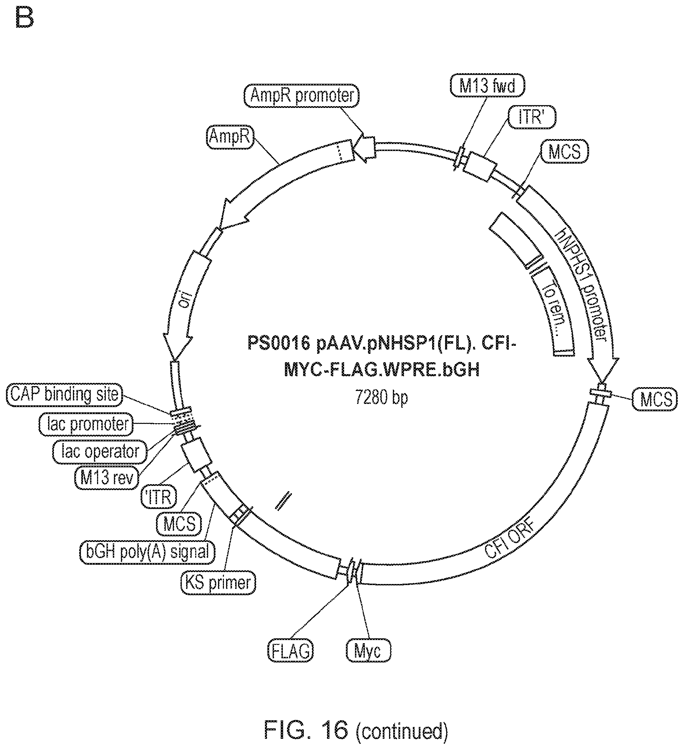

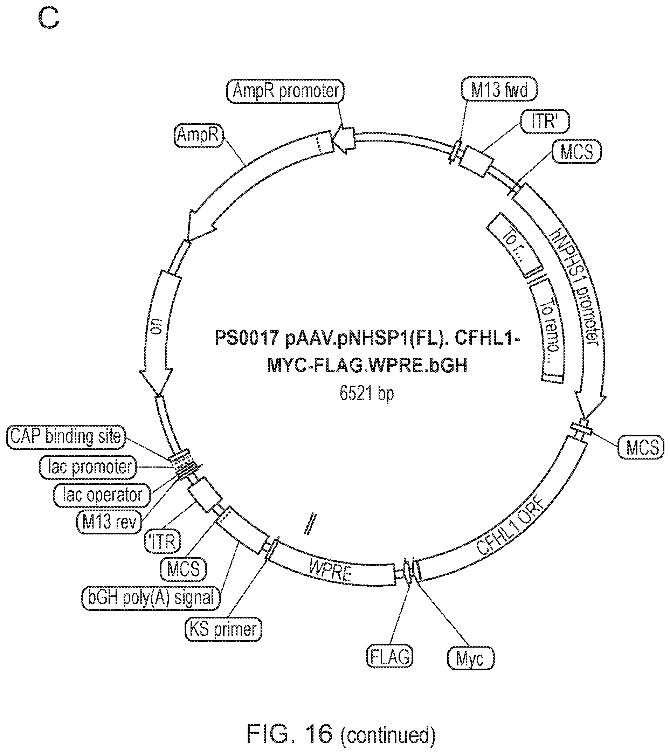

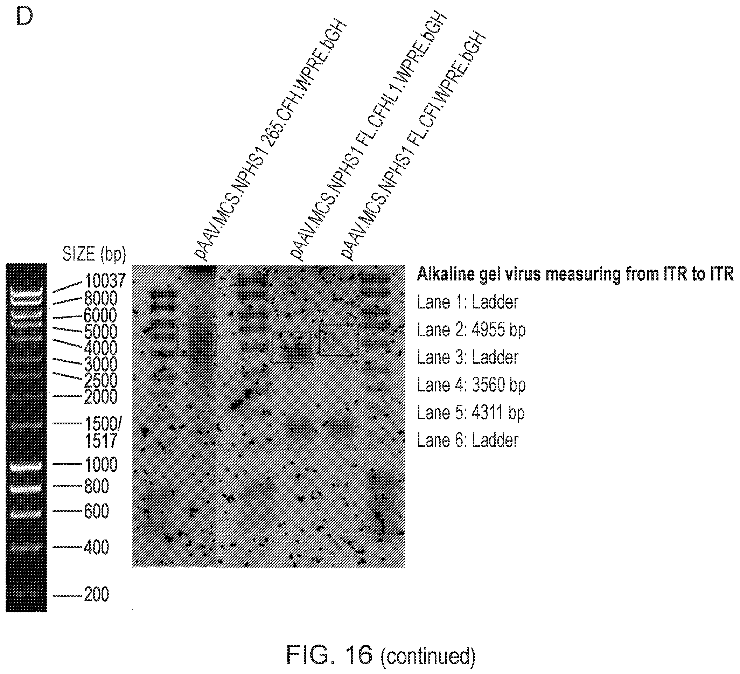

**Figure 16** - **Vector production**
   **(A)** Exemplary plasmid encoding CFH under control of a 265 bp minimal nephrin promoter (hNPHS1). **(B)** Exemplary plasmid encoding CFI under control of a full-length (FL, 1249bp) minimal nephrin promoter (hNPHS1). **(C)** Exemplary plasmid encoding FHL-1 under control of a full-length (FL, 1249bp) minimal nephrin promoter (hNPHS1). **(D)** Alkaline gel electrophoresis demonstrating that intact virus was identified following ultracentrifugation.

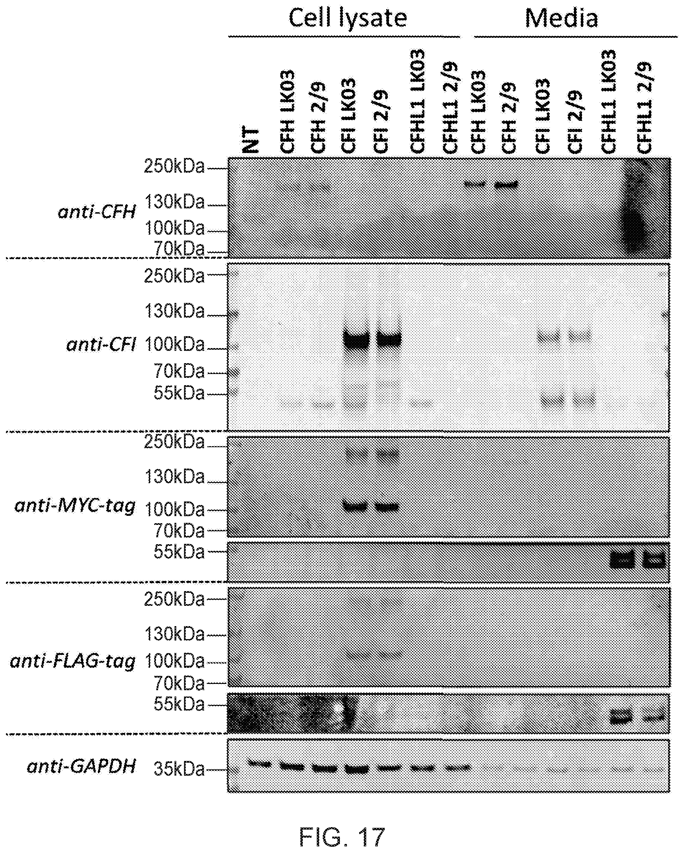

**Figure 17** - **Expression of CFH, CFI and CFHL1 in HEK cells**
   Protein expression analysis by western blot in 293T Human Embryonic Kidney cells. Cells were transfected with pAAV-265-CFH, pAAV-FL-CFI or pAAV-FL-CFHL1. NT (non-transfected 293T HEK cells). Expression of each of the transgenes is demonstrated in the cell lysate and/or media using protein-specific antibodies or anti-FLAG- or anti-MYC-tag antibodies.

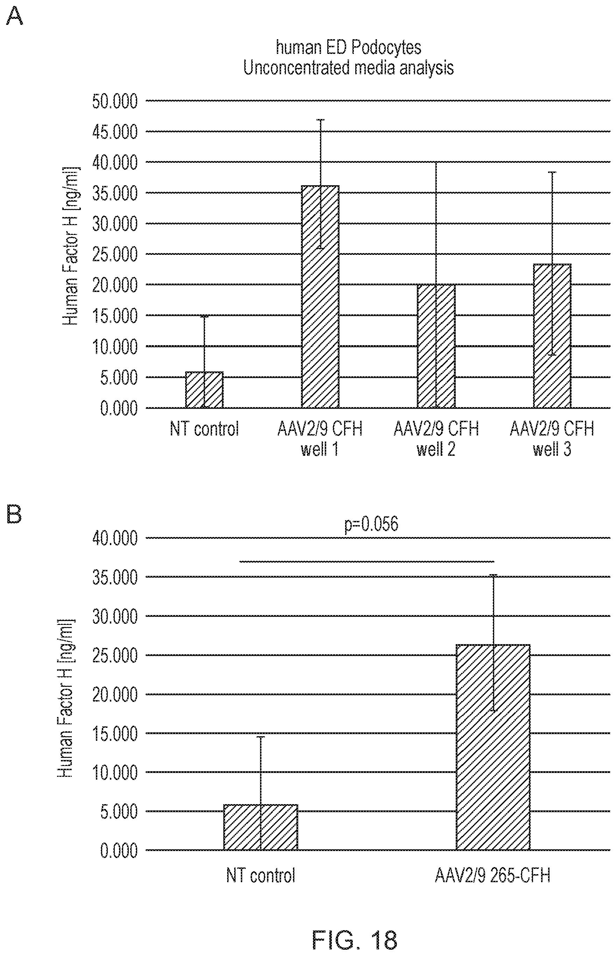

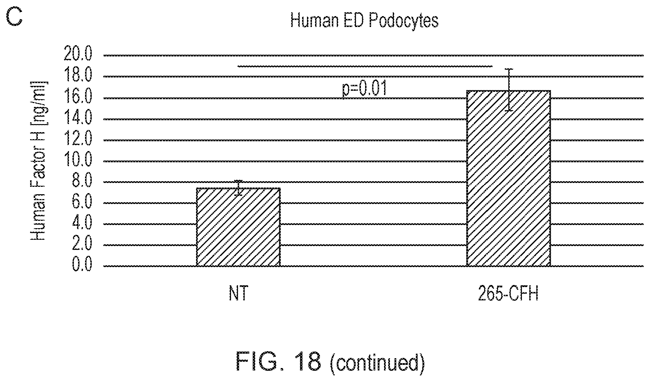

**Figure 18** - **Transduction of Factor H mutated podocytes ("Human early disease (ED) podocytes") with AAV2/9 265-CFH or plasmid encoding CFH**
   **(A)** Analysis of human Factor H concentration using an ELISA assay. Podocytes transduced with AAV2/9 virus containing the CFH transgene demonstrated higher concentrations of human Factor H in the culture media than the non-transduced control. **(B)** The average human Factor H concentration from (A). **(C)** Analysis of human Factor H concentration using an ELISA assay. Podocytes transfected with plasmid expressing the CFH transgene under the control of the 265bp minimal nephrin promoter demonstrated higher concentrations of human Factor H than the non-transfected control.

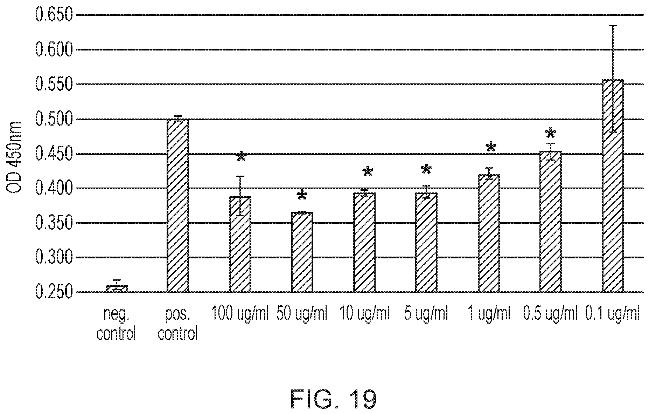

**Figure 19** - **Complement inhibition assay on glomerular endothelial cells with human CFH**
   Detection of C5b9 on human glomerular endothelial cells (GEnCs) using a cell-ELISA method. To allow specific activation of the complement alternative pathway, cells were incubated with 10% human Factor H-depleted serum, Zymosan and Mg/EGTA. Various concentrations (between 0.1ug/ml and 100ug/ml) of Factor H purified from normal human serum were added to the cell culture to inhibit the complement pathway on the surface of GEnCs. The negative control is a no human serum control and the positive control has all components of the reaction except Factor H.

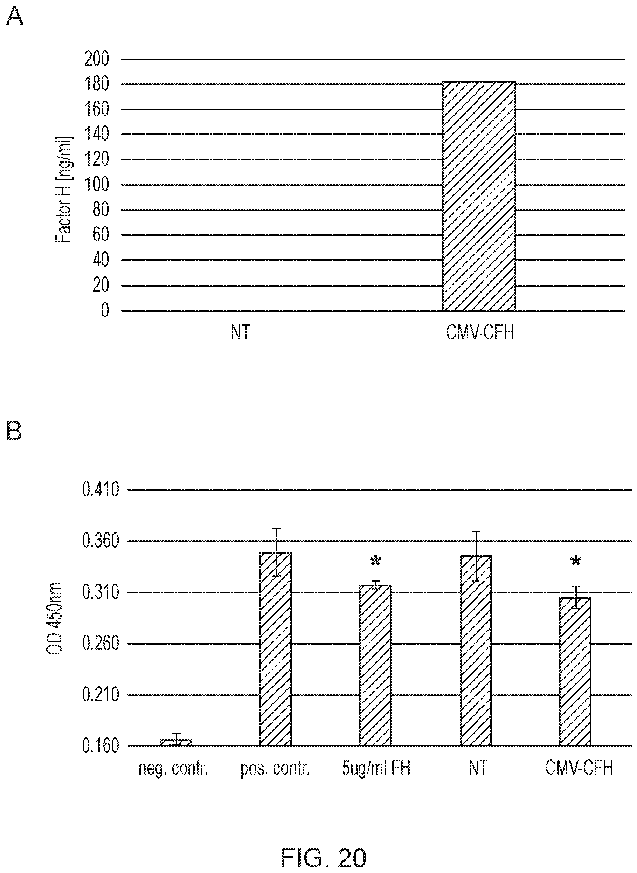

**Figure 20** **- Inhibition of a complement activation assay in 293T HEK cells**
   **(A)** Media from 293T HEK non-transfected (NT) control cells and cells transfected with plasmid expressing CFH under the control of a CMV promoter were analysed for the presence of soluble CFH using ELISA. Media from transfected cells showed an increase in the concentration of Factor H compared to media from the non-transfected control. **(B)** Media from 293T HEK non-transfected (NT) control cells and from CFH-expressing cells were tested by GEnC cell-ELISA method for its ability to inhibit the alternative complement pathway. Media taken from the CFH-expressing cells was able to inhibit the complement activation assay to the same extent as 5ug/ml of purified Factor H. Media from the non-transfected control cells demonstrated no inhibition of the complement activation assay. Error bars mean, ± s.d. Statistical analysis using student t test, *p<0.05.

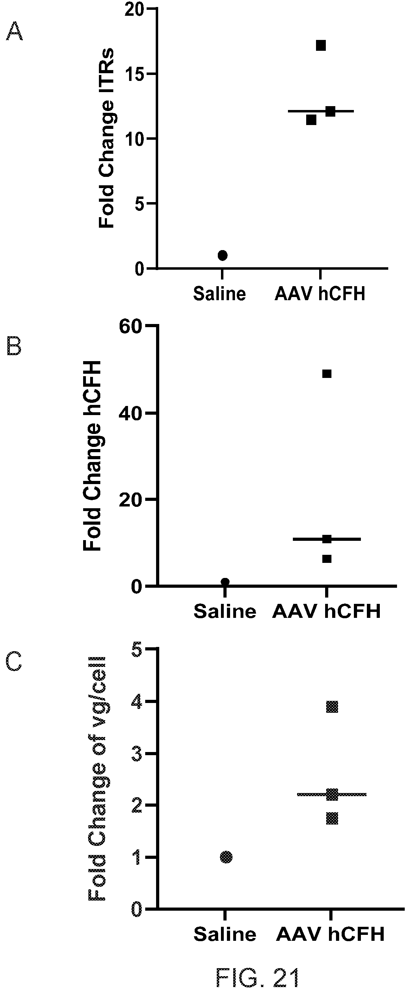

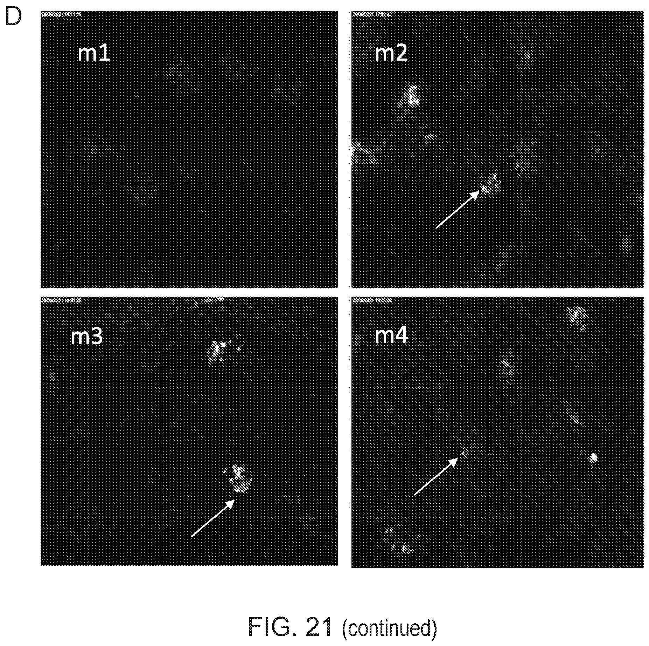

**Figure 21** - **Expression of CFH in the kidney in WT mice**
   Gene expression profile demonstrating an increase in **(A)** viral ITRs, **(B)** human CFH cDNA, and **(C)** viral particles in wild-type mice injected with pAAV.NPHS1(265).hCFH.WPRE.bGH in comparison to mice injected with the saline control. **(D)** Immunofluorescence imaging of kidney sections derived from wild-type C57BL6 mice injected with the saline control (m1) or the AAV gene therapy product (pAAV.NPHS1(265).hCFH.WPRE.bGH, n=3) (m2/m3/m4). Nephrin=red; CFH=green. Arrows indicate the co-localization of nephrin and CFH in the mouse glomerulus. Zoom =20x.

### DETAILED DESCRIPTION

Various preferred features and embodiments of the present invention will now be described by way of non-limiting examples.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the term "consisting of'.

The publications discussed herein are provided solely for their disclosure prior to the filing date of the present application. Nothing herein is to be construed as an admission that such publications constitute prior art to the claims appended hereto.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

### Glomerulus and podocyte gene therapy

The glomerulus is the filtration unit of the kidneys. Approximately 180 litres of plasma are filtered each day, and the healthy glomerular filtration barrier has an astonishing ability to retain about 99.9% of large proteins including albumin over our lifetimes without clogging. The afferent arteriole enters into the glomerular capillary bed, where filtration occurs, and blood leaves the glomerulus via the efferent arteriole. The glomerular filtration barrier (GFB) comprises 3 main layers: the glomerular endothelial cell, the glomerular basement membrane (GBM) and the podocyte.

The podocyte, the third layer of the GFB, plays a key role in the maintenance of the GFB. The podocyte is a highly-specialised cell, comprising of a cell body, major processes, secondary processes and foot processes that interdigitate with foot processes of adjacent podocytes to form the slit diaphragm. Podocytes form an effective and dynamic sieve, and this is predominantly thought to be due to the integrity of the slit diaphragm.

Gene therapy targeting the glomerulus is challenging. For example, although lentivirus might be of utility in transducing the tubules, it has thus far not shown any in vivo transduction of the glomerulus. Moreover, initial attempts to deliver adenovirus via either the renal artery or retrograde delivery via the ureter seemed to mainly result in tubular or interstitial transduction. Initial studies on the rodent kidney using AAV2 demonstrated mostly transduction of the tubules and no expression of the glomerulus.

### Vectors

In one aspect, the present invention provides a vector which is targeted to podocytes and is suitable for use in treating complement-mediated kidney diseases, such as C3 glomerulopathy and IgA Nephropathy.

The vector of the present invention is a viral vector. The vector of the invention is an adeno-associated viral (AAV) vector. In embodiments not forming part of the claimed invention it is contemplated that other viral vectors may be used.

The vector of the present invention may be in the form of an AAV vector particle.

Methods of preparing and modifying viral vectors and viral vector particles, such as those derived from AAV, are well known in the art. Suitable methods are described in Ayuso, E., et al., 2010. Current gene therapy, 10(6), pp.423-436, Merten, O.W., et al., 2016. Molecular Therapy-Methods & Clinical Development, 3, p.16017; and Nadeau, I. and Kamen, A., 2003. Biotechnology advances, 20(7-8), pp.475-489.

The vector of the present invention is preferably capable of transducing podocytes. Preferably, the vector of the present invention is capable of specifically transducing podocytes.

### Adeno-associated viral (AAV) vectors

The vector of the present invention is an adeno-associated viral (AAV) vector. The vector of the present invention may be in the form of an AAV vector particle.

### AAV genome

The AAV vector or AAV vector particle may comprise an AAV genome or a fragment or derivative thereof.

An AAV genome is a polynucleotide sequence, which may encode functions needed for production of an AAV particle. These functions include those operating in the replication and packaging cycle of AAV in a host cell, including encapsidation of the AAV genome into an AAV particle. Naturally occurring AAVs are replication-deficient and rely on the provision of helper functions in trans for completion of a replication and packaging cycle. Accordingly, the AAV genome of the AAV vector of the invention is typically replication-deficient.

The AAV genome may be in single-stranded form, either positive or negative-sense, or alternatively in double-stranded form. The use of a double-stranded form allows bypass of the DNA replication step in the target cell and so can accelerate transgene expression.

AAVs occurring in nature may be classified according to various biological systems. The AAV genome may be from any naturally derived serotype, isolate or clade of AAV.

AAV may be referred to in terms of their serotype. A serotype corresponds to a variant subspecies of AAV which, owing to its profile of expression of capsid surface antigens, has a distinctive reactivity which can be used to distinguish it from other variant subspecies. Typically, an AAV vector particle having a particular AAV serotype does not efficiently cross-react with neutralising antibodies specific for any other AAV serotype. AAV serotypes include AAV1, AAV2, AAV3, AAV4, AAV5, AAV6, AAV7, AAV8, AAV9, AAV10 and AAV11. The AAV vector of the invention may be an AAV3B, LK03, AAV9, or AAV8 serotype.

AAV may also be referred to in terms of clades or clones. This refers to the phylogenetic relationship of naturally derived AAVs, and typically to a phylogenetic group of AAVs which can be traced back to a common ancestor, and includes all descendants thereof. Additionally, AAVs may be referred to in terms of a specific isolate, i.e. a genetic isolate of a specific AAV found in nature. The term genetic isolate describes a population of AAVs which has undergone limited genetic mixing with other naturally occurring AAVs, thereby defining a recognisably distinct population at a genetic level.

Typically, the AAV genome of a naturally derived serotype, isolate or clade of AAV comprises at least one inverted terminal repeat sequence (ITR). An ITR sequence acts in cis to provide a functional origin of replication and allows for integration and excision of the vector from the genome of a cell. ITRs may be the only sequences required in cis next to the therapeutic gene.

Suitably, one or more ITR sequences flank the nucleotide sequence encoding a complement protein (e.g. an inhibitor of the complement system).

The AAV genome may also comprise packaging genes, such as rep and/or cap genes which encode packaging functions for an AAV particle. A promoter may be operably linked to each of the packaging genes. Specific examples of such promoters include the p5, p19 and p40 promoters. For example, the p5 and p19 promoters are generally used to express the rep gene, while the p40 promoter is generally used to express the cap gene. The rep gene encodes one or more of the proteins Rep78, Rep68, Rep52 and Rep40 or variants thereof. The cap gene encodes one or more capsid proteins such as VP1, VP2 and VP3 or variants thereof. These proteins make up the capsid of an AAV particle, which determines the AAV serotype. VP1, VP2, and VP3 may be produced by alternate mRNA splicing (Trempe, J.P. and Carter, B.J., 1988. Journal of virology, 62(9), pp.3356-3363). Thus, VP1, VP2 and VP3 may have identical sequences, but wherein VP2 is truncated at the N-terminus relative to VP1, and VP3 is truncated at the N-terminus relative to VP2.

The AAV genome may be the full genome of a naturally occurring AAV. For example, a vector comprising a full AAV genome may be used to prepare an AAV vector or vector particle.

Preferably, the AAV genome is derivatised for the purpose of administration to patients. Such derivatisation is standard in the art and the invention encompasses the use of any known derivative of an AAV genome, and derivatives which could be generated by applying techniques known in the art. The AAV genome may be a derivative of any naturally occurring AAV. Suitably, the AAV genome is a derivative of AAV1, AAV2, AAV3, AAV4, AAV5, AAV6, AAV7, AAV8, AAV9, AAV10, or AAV11. Suitably, the AAV genome is a derivative of AAV2.

Derivatives of an AAV genome include any truncated or modified forms of an AAV genome which allow for expression of a transgene from an AAV vector of the invention *in vivo.* Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR. A preferred mutant ITR is one having a deletion of a trs (terminal resolution site). This deletion allows for continued replication of the genome to generate a single-stranded genome which contains both coding and complementary sequences, i.e. a self-complementary AAV genome.

This allows for bypass of DNA replication in the target cell, and so enables accelerated transgene expression.

The AAV genome may comprise one or more ITR sequences from any naturally derived serotype, isolate or clade of AAV or a variant thereof. The AAV genome may comprise at least one, such as two, AAV1, AAV2, AAV3, AAV4, AAV5, AAV6, AAV7, AAV8, AAV9, AAV10, or AAV11 ITRs, or variants thereof. Suitably, the AAV genome may comprise at least one, such as two, AAV2 ITRs.

The one or more ITRs will preferably flank the nucleotide sequence encoding a complement protein (e.g. an inhibitor of the complement system) at either end. The inclusion of one or more ITRs is preferred to aid concatamer formation of the AAV vector in the nucleus of a host cell, for example following the conversion of single-stranded vector DNA into double-stranded DNA by the action of host cell DNA polymerases. The formation of such episomal concatamers protects the AAV vector during the life of the host cell, thereby allowing for prolonged expression of the transgene in vivo.

Suitably, the AAV genome may comprise one or more AAV2 ITR sequences flanking the nucleotide sequence encoding a complement protein (e.g. an inhibitor of the complement system). Suitably, the AAV genome may comprise two AAV2 ITR sequences flanking either side of the nucleotide sequence encoding a complement protein (e.g. an inhibitor of the complement system).

Suitably, ITR elements will be the only sequences retained from the native AAV genome in the derivative. A derivative will preferably not include the rep and/or cap genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

The following portions could therefore be removed in a derivative of the invention: one inverted terminal repeat (ITR) sequence, the replication (rep) and capsid (cap) genes. However, derivatives may additionally include one or more rep and/or cap genes or other viral sequences of an AAV genome. Naturally occurring AAV integrates with a high frequency at a specific site on human chromosome 19, and shows a negligible frequency of random integration, such that retention of an integrative capacity in the AAV vector may be tolerated in a therapeutic setting.

The invention additionally encompasses the provision of sequences of an AAV genome in a different order and configuration to that of a native AAV genome. The invention also encompasses the replacement of one or more AAV sequences or genes with sequences from another virus or with chimeric genes composed of sequences from more than one virus. Such chimeric genes may be composed of sequences from two or more related viral proteins of different viral species.

### AAV serotype and capsid proteins

The AAV vector particle may be encapsidated by capsid proteins. The serotype may facilitate the transduction of podocytes, for example specific transduction of podocytes. Preferably, the AAV vector particle is a podocyte-specific vector particle. The AAV vector particle may be encapsidated by a podocyte-specific capsid. The AAV vector particle may comprise a podocyte-specific capsid protein.

Suitably, the AAV vector particles may be transcapsidated forms wherein an AAV genome or derivative having an ITR of one serotype is packaged in the capsid of a different serotype. The AAV vector particle also includes mosaic forms wherein a mixture of unmodified capsid proteins from two or more different serotypes makes up the viral capsid. The AAV vector particle also includes chemically modified forms bearing ligands adsorbed to the capsid surface. For example, such ligands may include antibodies for targeting a particular cell surface receptor.

Where a derivative comprises capsid proteins i.e. VP1, VP2 and/or VP3, the derivative may be a chimeric, shuffled or capsid-modified derivative of one or more naturally occurring AAVs. In particular, the invention encompasses the provision of capsid protein sequences from different serotypes, clades, clones, or isolates of AAV within the same vector (i.e. a pseudotyped vector). The AAV vector may be in the form of a pseudotyped AAV vector particle.

Chimeric, shuffled or capsid-modified derivatives will be typically selected to provide one or more desired functionalities for the AAV vector. Thus, these derivatives may display increased efficiency of gene delivery, decreased immunogenicity (humoral or cellular), an altered tropism range and/or improved targeting of podocytes compared to an AAV vector comprising a naturally occurring AAV genome. Increased efficiency of gene delivery may be effected by improved receptor or co-receptor binding at the cell surface, improved internalisation, improved trafficking within the cell and into the nucleus, improved uncoating of the viral particle and improved conversion of a single-stranded genome to double-stranded form. Increased efficiency may also relate to an altered tropism range or targeting of podocytes, such that the vector dose is not diluted by administration to tissues where it is not needed.

Chimeric capsid proteins include those generated by recombination between two or more capsid coding sequences of naturally occurring AAV serotypes. This may be performed for example by a marker rescue approach in which non-infectious capsid sequences of one serotype are co-transfected with capsid sequences of a different serotype, and directed selection is used to select for capsid sequences having desired properties. The capsid sequences of the different serotypes can be altered by homologous recombination within the cell to produce novel chimeric capsid proteins.

Chimeric capsid proteins also include those generated by engineering of capsid protein sequences to transfer specific capsid protein domains, surface loops or specific amino acid residues between two or more capsid proteins, for example between two or more capsid proteins of different serotypes.

Shuffled or chimeric capsid proteins may also be generated by DNA shuffling or by error-prone PCR. Hybrid AAV capsid genes can be created by randomly fragmenting the sequences of related AAV genes e.g. those encoding capsid proteins of multiple different serotypes and then subsequently reassembling the fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error prone PCR may be used to randomly mutate AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N- and/or C-terminus of a capsid coding sequence. The unrelated protein or peptide may advantageously be one which acts as a ligand for a particular cell type, thereby conferring improved binding to a target cell or improving the specificity of targeting of the vector to a particular cell population. The unrelated protein may also be one which assists purification of the viral particle as part of the production process, i.e. an epitope or affinity tag. The site of insertion will typically be selected so as not to interfere with other functions of the viral particle e.g. internalisation, trafficking of the viral particle.

The capsid protein may be an artificial or mutant capsid protein. The term "artificial capsid" as used herein means that the capsid particle comprises an amino acid sequence which does not occur in nature or which comprises an amino acid sequence which has been engineered (e.g. modified) from a naturally occurring capsid amino acid sequence. In other words the artificial capsid protein comprises a mutation or a variation in the amino acid sequence compared to the sequence of the parent capsid from which it is derived where the artificial capsid amino acid sequence and the parent capsid amino acid sequences are aligned.

The capsid protein may comprise a mutation or modification relative to the wild type capsid protein which improves the ability to transduce podocytes relative to an unmodified or wild type viral particle. Improved ability to transduce podocytes may be measured for example by measuring the expression of a transgene, e.g. GFP, carried by the AAV vector particle, wherein expression of the transgene in podocytes correlates with the ability of the AAV vector particle to transduce podocytes.

The AAV vector particle may be an AAV3B, LK03, AAV9, or AAV8 vector particle. The present inventors have shown that AAV vector particles with AAV3B, LK03, AAV9 and AAV8 serotypes can transduce podocytes. Preferably, the AAV vector particle is an AAV3B vector particle or an LK03 vector particle. More preferably, the AAV vector particle is an AAV3B vector particle.

The AAV vector particle may comprise an AAV3B, LK03, AAV9, or AAV8 capsid protein. Preferably, the AAV vector particle comprises an AAV3B capsid protein or an LK03 capsid protein. More preferably, the AAV vector particle comprises an AAV3B capsid protein.

The AAV vector particle may comprise AAV3B, LK03, AAV9, or AAV8 capsid proteins VP1, VP2 and VP3. Preferably, the AAV vector particle comprises AAV3B or LK03 capsid proteins VP1, VP2 and VP3. More preferably, the AAV vector particle comprises AAV3B capsid proteins VP1, VP2 and VP3.

The AAV vector particle may comprise one or more AAV2 ITR sequences flanking the nucleotide sequence encoding a complement protein (e.g. an inhibitor of the complement system) and AAV3B capsid proteins, LK03 capsid proteins, AAV9 capsid proteins, or AAV8 capsid proteins. Preferably, the AAV vector particle comprises one or more AAV2 ITR sequences flanking the nucleotide sequence encoding a complement protein (e.g. an inhibitor of the complement system) and AAV3B or LK03 capsid proteins. More preferably, the AAV vector particle comprises one or more AAV2 ITR sequences flanking the nucleotide sequence encoding a complement protein (e.g. an inhibitor of the complement system) and AAV3B capsid proteins.

The AAV vector particle may have an AAV2 genome and AAV3B capsid proteins (AAV2/3B), an AAV2 genome and LK03 capsid proteins, an AAV2 genome and AAV9 capsid proteins (AAV2/9), or an AAV2 genome and AAV8 capsid proteins (AAV2/8). Preferably, the AAV vector particle comprises an AAV2 genome and AAV3B or LK03 capsid proteins. More preferably, the AAV vector particle comprises an AAV2 genome and AAV3B capsid proteins.

### AAV3B serotype

The AAV vector particle may comprise an AAV3B capsid protein. Suitably, the AAV vector particle may be encapsidated by AAV3B capsid proteins.

Two distinct AAV3 isolates (AAV3A and AAV3B) have been cloned. In comparison with vectors based on other AAV serotypes, it is thought that AAV3 vectors inefficiently transduce most cell types. However, AAV3B may efficiently transduce podocytes. AA3B has been described in Rutledge, E.A., et al., 1998. Journal of virology, 72(1), pp.309-319.

The AAV vector particle may comprise an AAV3B VP1 capsid protein, an AAV3B VP2 capsid protein, and/or an AAV3B VP3 capsid protein. Suitably, the AAV vector particle may be encapsidated by AAV3B VP1 capsid proteins, AAV3B VP2 capsid proteins, and/or AAV3B VP3 capsid proteins. Suitably, the AAV vector particle may be encapsidated by AAV3B VP1, VP2, and VP3 capsid proteins.

Suitably, the AAV3B VP1 capsid protein may comprise or consist of the amino acid sequence shown as SEQ ID NO: 1, or a variant which is at least 90% identical to SEQ ID NO: 1.

### Illustrative AAV3B VP1 capsid protein (SEQ ID NO: 1):

MAADGYLPDWLEDNLSEGIREWWALKPGVPQPKANQQHQDNRRGLVLPGYKYLGPGNG
LDKGEPVNEADAAALEHDKAYDQQLKAGDNPYLKYNHADAEFQERLQEDTSFGGNLGRAV
FQAKKRILEPLGLVEEAAKTAPGKKRPVDQSPQEPDSSSGVGKSGKQPARKRLNFGQTGD
SESVPDPQPLGEPPAAPTSLGSNTMASGGGAPMADNNEGADGVGNSSGNWHCDSQWL
GDRVITTSTRTWALPTYNNHLYKQISSQSGASNDNHYFGYSTPWGYFDFNRFHCHFSPRD
WQRLINNNWGFRPKKLSFKLFNIQVKEVTQNDGTTTIANNLTSTVQVFTDSEYQLPYVLGSA
HQGCLPPFPADVFMVPQYGYLTLNNGSQAVGRSSFYCLEYFPSQMLRTGNNFQFSYTFED
VPFHSSYAHSQSLDRLMNPLIDQYLYYLNRTQGTTSGTTNQSRLLFSQAGPQSMSLQARN
WLPGPCYRQQRLSKTANDNNNSNFPWTAASKYHLNGRDSLVNPGPAMASHKDDEEKFFP
MHGNLIFGKEGTTASNAELDNVMITDEEEIRTTNPVATEQYGTVANNLQSSNTAPTTRTVND
QGALPGMVWQDRDVYLQGPIWAKIPHTDGHFHPSPLMGGFGLKHPPPQIMIKNTPVPANP
PTTFSPAKFASFITQYSTGQVSVEIEWELQKENSKRWNPEIQYTSNYNKSVNVDFTVDTNG
VYSEPRPIGTRYLTRN

Suitably, the variant may be at least 95%, at least 96%, at least 97%, at least 98% or at least 99% identical to SEQ ID NO: 1.

Suitably, the AAV3B VP2 and VP3 capsid proteins may be N-terminal truncations of SEQ ID NO: 1, or N-terminal truncations of a variant which is at least 90% identical, at least 95%, at least 96%, at least 97%, at least 98% or at least 99% identical to SEQ ID NO: 1.

### LK03 serotype

The AAV vector particle may comprise an LK03 capsid protein. Suitably, the AAV vector particle may be encapsidated by LK03 capsid proteins.

The AAV-LK03 cap sequence consists of fragments from seven different wild-type serotypes (AAV1, 2, 3B, 4, 6, 8, 9) and is described in Lisowski, L., et al., 2014. Nature, 506(7488), pp.382-386. The present inventors have demonstrated that AAV-LK03 vectors can achieve high transduction of close to 100% in human podocytes *in vitro.*

The AAV vector particle may comprise an LK03 VP1 capsid protein, an LK03 VP2 capsid protein, and/or an LK03 VP3 capsid protein. Suitably, the AAV vector particle may be encapsidated by LK03 VP1 capsid proteins, LK03 VP2 capsid proteins, and/or LK03 VP3 capsid proteins. Suitably, the AAV vector particle may be encapsidated by LK03 VP1, VP2, and VP3 capsid proteins.

Suitably, the LK03 VP1 capsid protein may comprise or consist of the amino acid sequence shown as SEQ ID NO: 2, or a variant which is at least 90% identical to SEQ ID NO: 2.

### Illustrative LK03 VP1 capsid protein (SEQ ID NO: 2):

MAADGYLPDWLEDNLSEGIREWWALQPGAPKPKANQQHQDNARGLVLPGYKYLGPGNGL
DKGEPVNAADAAALEHDKAYDQQLKAGDNPYLKYNHADAEFQERLKEDTSFGGNLGRAVF
QAKKRLLEPLGLVEEAAKTAPGKKRPVDQSPQEPDSSSGVGKSGKQPARKRLNFGQTGD
SESVPDPQPLGEPPAAPTSLGSNTMASGGGAPMADNNEGADGVGNSSGNWHCDSQWL
GDRVITTSTRTWALPTYNNHLYKQISSQSGASNDNHYFGYSTPWGYFDFNRFHCHFSPRD
WQRLINNNWGFRPKKLSFKLFNIQVKEVTQNDGTTTIANNLTSTVQVFTDSEYQLPYVLGSA
HQGCLPPFPADVFMVPQYGYLTLNNGSQAVGRSSFYCLEYFPSQMLRTGNNFQFSYTFED
VPFHSSYAHSQSLDRLMNPLIDQYLYYLNRTQGTTSGTTNQSRLLFSQAGPQSMSLQARN
WLPGPCYRQQRLSKTANDNNNSNFPWTAASKYHLNGRDSLVNPGPAMASHKDDEEKFFP
MHGNLIFGKEGTTASNAELDNVMITDEEEIRTTNPVATEQYGTVANNLQSSNTAPTTRTVND
QGALPGMVWQDRDVYLQGPIWAKIPHTDGHFHPSPLMGGFGLKHPPPQIMIKNTPVPANP

PTTFSPAKFASFITQYSTGQVSVEIEWELQKENSKRWNPEIQYTSNYNKSVNVDFTVDTNG
VYSEPRPIGTRYLTRPL

Suitably, the variant may be at least 95%, at least 96%, at least 97%, at least 98% or at least 99% identical to SEQ ID NO: 2.

Suitably, the LK03 VP2 and VP3 capsid proteins may be N-terminal truncations of SEQ ID NO: 2, or N-terminal truncations of a variant which is at least 90% identical, at least 95%, at least 96%, at least 97%, at least 98% or at least 99% identical to SEQ ID NO: 2.

### AAV9 serotype

The AAV vector particle may comprise an AAV9 capsid protein. Suitably, the AAV vector particle may be encapsidated by AAV9 capsid proteins.

The present inventors have demonstrated that AAV9 vectors can achieve high transduction in human podocytes *in vitro.*

The AAV vector particle may comprise an AAV9 VP1 capsid protein, an AAV9 VP2 capsid protein, and/or an AAV9 VP3 capsid protein. Suitably, the AAV vector particle may be encapsidated by AAV9 VP1 capsid proteins, AAV9 VP2 capsid proteins, and/or AAV9 VP3 capsid proteins. Suitably, the AAV vector particle may be encapsidated by AAV9 VP1, VP2, and VP3 capsid proteins.

Suitably, the AAV9 VP1 capsid protein may comprise or consist of the amino acid sequence shown as SEQ ID NO: 3, or a variant which is at least 90% identical to SEQ ID NO: 3.

### Illustrative AAV9 VP1 capsid protein (SEQ ID NO: 3):

MAADGYLPDWLEDNLSEGIREWWALKPGAPQPKANQQHQDNARGLVLPGYKYLGPGNGL
DKGEPVNAADAAALEHDKAYDQQLKAGDNPYLKYNHADAEFQERLKEDTSFGGNLGRAVF
QAKKRLLEPLGLVEEAAKTAPGKKRPVEQSPQEPDSSAGIGKSGAQPAKKRLNFGQTGDT
ESVPDPQPIGEPPAAPSGVGSLTMASGGGAPVADNNEGADGVGSSSGNWHCDSQWLGD
RVITTSTRTWALPTYNNHLYKQISNSTSGGSSNDNAYFGYSTPWGYFDFNRFHCHFSPRD
WQRLINNNWGFRPKRLNFKLFNIQVKEVTDNNGVKTIANNLTSTVQVFTDSDYQLPYVLGS
AHEGCLPPFPADVFMIPQYGYLTLNDGSQAVGRSSFYCLEYFPSQMLRTGNNFQFSYEFE
NVPFHSSYAHSQSLDRLMNPLIDQYLYYLSKTINGSGQNQQTLKFSVAGPSNMAVQGRNYI
PGPSYRQQRVSTTVTQNNNSEFAWPGASSWALNGRNSLMNPGPAMASHKEGEDRFFPL
SGSLIFGKQGTGRDNVDADKVMITNEEEIKTTNPVATESYGQVATNHQSAQAQAQTGWVQ
NQGILPGMVWQDRDVYLQGPIWAKIPHTDGNFHPSPLMGGFGMKHPPPQILIKNTPVPADP

PTAFNKDKLNSFITQYSTGQVSVEIEWELQKENSKRWNPEIQYTSNYYKSNNVEFAVNTEG
VYSEPRPIGTRYLTRNL

Suitably, the variant may be at least 95%, at least 96%, at least 97%, at least 98% or at least 99% identical to SEQ ID NO: 3.

Suitably, the AAV9 VP2 and VP3 capsid proteins may be N-terminal truncations of SEQ ID NO: 3, or N-terminal truncations of a variant which is at least 90% identical, at least 95%, at least 96%, at least 97%, at least 98% or at least 99% identical to SEQ ID NO: 3.

### Other viral vectors

### Retroviral and lentiviral vectors

In embodiments not forming part of the claimed invention, the vector may be a retroviral vector or a lentiviral vector. The vector may be a retroviral vector particle or a lentiviral vector particle.

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV).

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses.

The basic structure of retrovirus and lentivirus genomes share many common features such as a 5' LTR and a 3' LTR. Between or within these are located a packaging signal to enable the genome to be packaged, a primer binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective. Portions of the viral genome may also be replaced by a library encoding candidate modulating moieties operably linked to a regulatory control region and a reporter moiety in the vector genome in order to generate a vector comprising candidate modulating moieties which is capable of transducing a target host cell and/or integrating its genome into a host genome.

Lentivirus vectors are part of the larger group of retroviral vectors. In brief, lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritis-encephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells. In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans).

Examples of non-primate lentiviruses may be any member of the family of lentiviridae which does not naturally infect a primate.

As examples of lentivirus-based vectors, HIV-1- and HIV-2-based vectors are described below.

The HIV-1 vector contains cis-acting elements that are also found in simple retroviruses. It has been shown that sequences that extend into the gag open reading frame are important for packaging of HIV-1. Therefore, HIV-1 vectors often contain the relevant portion of gag in which the translational initiation codon has been mutated. In addition, most HIV-1 vectors also contain a portion of the env gene that includes the RRE. Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat.

Most HIV-2-based vectors are structurally very similar to HIV-1 vectors. Similar to HIV-1-based vectors, HIV-2 vectors also require RRE for efficient transport of the full-length or singly spliced viral RNAs.

Preferably, the viral vector used in the present invention has a minimal viral genome.

By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably, the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably, the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

The vectors may be self-inactivating (SIN) vectors in which the viral enhancer and promoter sequences have been deleted. SIN vectors can be generated and transduce non-dividing cells in vivo with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR.

The vectors may be integration-defective. Integration defective lentiviral vectors (IDLVs) can be produced, for example, either by packaging the vector with catalytically inactive integrase (such as an HIV integrase bearing the D64V mutation in the catalytic site) or by modifying or deleting essential att sequences from the vector LTR, or by a combination of the above.

### Adenoviral vectors

In embodiments not forming part of the claimed invention, the vector may be an adenoviral vector. The vector may be an adenoviral vector particle.

The adenovirus is a double-stranded, linear DNA virus that does not go through an RNA intermediate. There are over 50 different human serotypes of adenovirus divided into 6 subgroups based on the genetic sequence homology. The natural targets of adenovirus are the respiratory and gastrointestinal epithelia, generally giving rise to only mild symptoms. Serotypes 2 and 5 (with 95% sequence homology) are most commonly used in adenoviral vector systems and are normally associated with upper respiratory tract infections in the young.

Adenoviruses have been used as vectors for gene therapy and for expression of heterologous genes. The large (36 kb) genome can accommodate up to 8 kb of foreign insert DNA and is able to replicate efficiently in complementing cell lines to produce very high titres of up to 10¹². Adenovirus is thus one of the best systems to study the expression of genes in primary non-replicative cells.

The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, they function episomally (independently from the host genome) as a linear genome in the host nucleus. Hence the use of recombinant adenovirus alleviates the problems associated with random integration into the host genome.

### Herpes simplex viral vector

In embodiments not forming part of the claimed invention, the vector may be a herpes simplex viral vector. The vector may be a herpes simplex viral vector particle.

Herpes simplex virus (HSV) is a neurotropic DNA virus with favorable properties as a gene delivery vector. HSV is highly infectious, so HSV vectors are efficient vehicles for the delivery of exogenous genetic material to cells. Viral replication is readily disrupted by null mutations in immediate early genes that in vitro can be complemented in trans, enabling straightforward production of high-titre pure preparations of non-pathogenic vector. The genome is large (152 Kb) and many of the viral genes are dispensable for replication in vitro, allowing their replacement with large or multiple transgenes. Latent infection with wild-type virus results in episomal viral persistence in sensory neuronal nuclei for the duration of the host lifetime. The vectors are non-pathogenic, unable to reactivate and persist long-term. The latency active promoter complex can be exploited in vector design to achieve long-term stable transgene expression in the nervous system. HSV vectors transduce a broad range of tissues because of the wide expression pattern of the cellular receptors recognized by the virus. Increasing understanding of the processes involved in cellular entry has allowed targeting the tropism of HSV vectors.

### Vaccinia virus vectors

In embodiments not forming part of the claimed invention, the vector may be a vaccinia viral vector. The vector may be a vaccinia viral vector particle.

Vaccinia virus is large enveloped virus that has an approximately 190 kb linear, double-stranded DNA genome. Vaccinia virus can accommodate up to approximately 25 kb of foreign DNA, which also makes it useful for the delivery of large genes.

A number of attenuated vaccinia virus strains are known in the art that are suitable for gene therapy applications, for example the MVA and NYVAC strains.

### Regulatory elements

The vector of the invention may comprise one or more regulatory sequences which may act pre- or post-transcriptionally. Suitably, the nucleotide sequence encoding a complement protein (e.g. an inhibitor of the complement system) may be operably linked to one or more regulatory sequences. The one or more regulatory sequences may facilitate expression of the complement protein (e.g. an inhibitor of the complement system) in podocytes.

"Regulatory sequences" are any sequences which facilitate expression of the polypeptides, e.g. act to increase expression of a transcript or to enhance mRNA stability. Suitable regulatory sequences include for example promoters, enhancer elements, post-transcriptional regulatory elements and polyadenylation sites.

### Promoters

The vector of the invention comprises a promoter, operably linked to the nucleotide sequence encoding an inhibitor of the complement system. The promoter facilitates expression of the inhibitor of the complement system in podocytes.

A "promoter" is a region of DNA that leads to initiation of transcription of a gene. Promoters are located near the transcription start sites of genes, upstream on the DNA (towards the 5' region of the sense strand). Any suitable promoter may be used, the selection of which may be readily made by the skilled person.

The promoter is a tissue-specific promoter. In an embodiment not forming part of the invention, the promoter may be a constitutive promoter.

Suitable constitutive promoters will be known to the skilled person. For example, in one embodiment the promoter is a CMV promoter.

The vector of the invention comprises a podocyte-specific promoter. The nucleotide sequence encoding an inhibitor of the complement system is operably linked to the podocyte-specific promoter.

As used herein, a "podocyte-specific promoter" is a promoter which preferentially facilitates expression of a gene in podocyte cells. Suitably, a podocyte-specific promoter may facilitate higher expression of a gene in podocytes as compared to other cell-types. Higher expression in podocytes may be measured for example by measuring the expression of a transgene, e.g. GFP, operably linked to the promoter, wherein expression of the transgene in podocytes correlates with the ability of the promoter to facilitate expression of a gene in podocytes. For example, a podocyte-specific promoter may be a promoter which facilitates gene expression levels at least 10% higher, at least 20% higher, at least 30% higher, at least 40% higher, at least 50% higher, at least 100% higher, at least 200% higher, at least 300% higher, at least 400% higher, at least 500% higher, or at least 1000% higher in podocytes compared to expression levels in other cell types.

Suitable podocyte-specific promoters will be well known to those of skill in the art.

The podocyte-specific promoter may be or may be derived from a promoter associated with a gene with selective expression in human podocytes. Genes selectively expressed in podocytes will be known to those of skill in the art and selective gene expression in podocytes can be readily determined by methods know to those of skill in the art, for instance with microarrays. Genes selectively expressed in podocytes include NPHS1, NPHS2, WT1, FOXC2, ABCA9, ACPP, ACTN4, ADM, ANGPTL2, ANXA1, ASB15, ATP8B1, B3GALT2, BB014433, BMP7, C1QTNF1, CAR13, CD2AP, CD55, CD59A, CD59B, CDC14A, CDH3, CDKN1B, CDKN1C, CEP85L, CLIC3, CLIC5, COL4A1, COL4A2, COL4A3, COL4A4, COL4A5, COLEC12, CRIM1, CST12, DEGS1, DOCK4, DOCK5, EGF, ENPEP, EPHX1, FAM81A, FAT1, FGFBP1, FOXD1, FRYL, GABRB1, GALC, GM10554, H2-D1, H2-Q7, H2BC4, H3C15, HS3ST3A1, HTRA1, IFNGR1, IL18, ILDR2, ITGB5, ITGB8, KIRREL, LAMA1, LAMA5, LAMB1, LAMB2, LMX1B, MAFB, MAGI2, MELA, MERTK, MGAT4A, MYO1D, MYO1E, MYOM2, MYZAP, NEBL, NES, NOD1, NPR3, NR2F2, NUPR1, OPTN, P3H2, PAK1, PARD3B, PDPN, PLAT, PLCE1, PLSCR2, PODXL, PROS1, PTPRO, RAB3B, RDH1, RDH9, SDC4, SEMA3E, SERPINB6B, SH3BGRL2, SLC41A2, SLCO2A1, ST3GAL6, SYNPO, TDRD5, THSD7A, TIMP3, TJP1, TLR7, TM4SF1, TMEM108, TMEM54, TMTC1, TOP1MT, TRAV10, TRAV10N, TRAV5-4, TSHB, UACA, UBA1Y, UPRT, VEGFA, VTCN1, ZBTB20, and 5730407I07RIK.

Methods to identify the promoter regions associated with genes will be well known to those of skill in the art. The promoter is usually located just proximal to or overlapping the transcription initiation site and contains several sequence motifs with which transcription factors (TFs) interact in a sequence-specific manner.

The podocyte-specific promoter is selected from a NPHS1 promoter and a NPHS2 promoter. In embodiments not forming part of the claimed invention the podocyte-specific promoter may be selected from a WT1 promoter, a FOXC2 promoter, a ABCA9 promoter, a ACPP promoter, a ACTN4 promoter, a ADM promoter, a ANGPTL2 promoter, a ANXA1 promoter, a ASB15 promoter, a ATP8B1 promoter, a B3GALT2 promoter, a BB014433 promoter, a BMP7 promoter, a C1QTNF1 promoter, a CAR13 promoter, a CD2AP promoter, a CD55 promoter, a CD59A promoter, a CD59B promoter, a CDC14A promoter, a CDH3 promoter, a CDKN1B promoter, a CDKN1C promoter, a CEP85L promoter, a CLIC3 promoter, a CLIC5 promoter, a COL4A1 promoter, a COL4A2 promoter, a COL4A3 promoter, a COL4A4 promoter, a COL4A5 promoter, a COLEC12 promoter, a CRIM1 promoter, a CST12 promoter, a DEGS1 promoter, a DOCK4 promoter, a DOCK5 promoter, a EGF promoter, a ENPEP promoter, a EPHX1 promoter, a FAM81A promoter, a FAT1 promoter, a FGFBP1 promoter, a FOXD1 promoter, a FRYL promoter, a GABRB1 promoter, a GALC promoter, a GM10554 promoter, a H2-D1 promoter, a H2-Q7 promoter, a H2BC4 promoter, a H3C15 promoter, a HS3ST3A1 promoter, a HTRA1 promoter, a IFNGR1 promoter, a IL18 promoter, a ILDR2 promoter, a ITGB5 promoter, a ITGB8 promoter, a KIRREL promoter, a LAMA1 promoter, a LAMA5 promoter, a LAMB1 promoter, a LAMB2 promoter, a LMX1B promoter, a MAFB promoter, a MAGI2 promoter, a MELA promoter, a MERTK promoter, a MGAT4A promoter, a MYO1D promoter, a MYO1E promoter, a MYOM2 promoter, a MYZAP promoter, a NEBL promoter, a NES promoter, a NOD1 promoter, a NPR3 promoter, a NR2F2 promoter, a NUPR1 promoter, a OPTN promoter, a P3H2 promoter, a PAK1 promoter, a PARD3B promoter, a PDPN promoter, a PLAT promoter, a PLCE1 promoter, a PLSCR2 promoter, a PODXL promoter, a PROS1 promoter, a PTPRO promoter, a RAB3B promoter, a RDH1 promoter, a RDH9 promoter, a SDC4 promoter, a SEMA3E promoter, a SERPINB6B promoter, a SH3BGRL2 promoter, a SLC41A2 promoter, a SLCO2A1 promoter, a ST3GAL6 promoter, a SYNPO promoter, a TDRD5 promoter, a THSD7A promoter, a TIMP3 promoter, a TJP1 promoter, a TLR7 promoter, a TM4SF1 promoter, a TMEM108 promoter, a TMEM54 promoter, a TMTC1 promoter, a TOP1MT promoter, a TRAV10 promoter, a TRAV10N promoter, a TRAV5-4 promoter, a TSHB promoter, a UACA promoter, a UBA1Y promoter, a UPRT promoter, a VEGFA promoter, a VTCN1 promoter, a ZBTB20 promoter, and a 5730407I07RIK promoter, or a fragment or derivative thereof.

Preferably, the podocyte-specific promoter is a NPHS1 or a NPHS2 promoter, or a fragment or derivative thereof. More preferably, the podocyte-specific promoter is a NPHS1 promoter, or a fragment or derivative thereof.

The podocyte-specific promoter may be a minimal podocyte-specific promoter. As used, herein, a "minimal podocyte-specific promoter" means the minimal sequence that can act as a podocyte-specific promoter.

Preferably, the podocyte-specific promoter is a minimal NPHS1 or a minimal NPHS2 promoter, or a fragment or derivative thereof. More preferably, the podocyte-specific promoter is a minimal NPHS1 promoter, or a fragment or derivative thereof.

Preferably, the promoter is a human promoter, e.g. a minimal human NPHS1 promoter.

### NPHS1 promoter

The vector of the invention may comprise a NPHS1 promoter, or a fragment or derivative thereof. Suitably, the NPHS1 promoter, or fragment or derivative thereof, may be operably linked to the nucleotide sequence encoding a complement protein (e.g. an inhibitor of the complement system).

The NPHS1 gene encodes nephrin, which is selectively expressed in podocytes.

The NPHS1 promoter may be a minimal NPHS1 promoter. For example, the NPHS1 promoter may have a length of 1.2 kb or less.

A minimal human NPHS1 promoter has been described in Moeller et al. 2002 J Am Soc Nephrol, 13(6):1561-7 and Wong MA et al. 2000 Am J Physiol Renal Physiol, 279(6):F1027-32. This minimal NPHS1 is a 1.2kb fragment and appears to be podocyte-specific. The 1.2kb promoter region lacks a TATA box, but has recognition motifs for other transcription factors e.g. PAX-2 binding element, E-box and GATA consensus sequences.

Suitably, the NPHS1 promoter may comprise or consist of the nucleotide sequence shown as SEQ ID NO: 4, or a variant which is at least 70% identical to SEQ ID NO: 4.

### Illustrative minimal NPHS1 promoter (SEQ ID NO: 4):

cacctgaggtcaggagttcgagaccagcgtggccaacatgatgaaaccccgtctctagtaaaaatacaaaaattagccaggc
atggtgctatatacctgtagcaccagctacttgggagacagaggtgggagaattacttgaacctgggaggttcaagccatggga
ggtggaagttgcagtgagccgagatgccactgcactccagcctgagcaacagagcaagactatctcaagaaaagaaagaaa
gaaagaaagagacttgccaaggtcatgtatcagggcaaggaagagctgggggcccagctggctgctcccctgctgagctggg
agaccaccttgatctgacttctcccatcttcccagcctaagccaggccctggggtcacggaggctggggaggcaccgaggaac
gcgcctggcatgtgctgacaggggattttatgctccagctgggccagctgggaggagcctgctgggcagaggccagagctggg
ggctctggaaggtacctgggggaggttgcactgtgagaatgagctcaagctgggtcagagagcagggctgactctgccagtgc
ctgcatcagcctcatcgctctcctaggctcctggcctgctggactctgggctgcaggtccttcttgaaaggctgtgagtagtgagac
aaggagcaggagtgaggggtggcaggagagaagatagagattgagagagagagagagagagacagagagagagg
aagagacagagacaaaaggagagagaacggcttagacaaggagagaaagatggaaagataaagagactgggcgcagt
ggctcacgcctgtaatcccaacacttggggaggccaaggtgggaggatggcttgaaggaaagagtctgagatcaacctggcc
aacatagtgagaccccgtctctaaaaaaaaaagaaaaaaaaaagaaaaaagaaaaaaaagtttttttaaagagacagaga
aagagactcagagattgagactgagagcaagacagagagagatactcacagggaagaggggaagaggaaaacgagaa
agggaggagagtaacggaaagagataaaaaagaaaagcaggtggcagagacacacagagagggacccagagaaagc
cagacagacgcaggtggctggcagcgggcgctgtgggggtcacagtaggggacctgtg

Suitably, the variant may be at least 75%, at least 80%, at least 85%, at least 90%, at least 95%, at least 98% or at least 99% identical to SEQ ID NO: 4.

In some embodiments, the NPHS1 promoter may comprise or consist of the nucleotide sequence shown as SEQ ID NO: 27, or a variant which is at least 70% identical to SEQ ID NO: 27.

### Illustrative minimal NPHS1 promoter - 265 bp (SEQ ID NO: 27)

GGCCCTGGGGTCACGGAGGCTGGGGAGGCACCGAGGAACGCGCCTGGCATGTGCTG
ACAGGGGATTTTATGCTCCAGGAGCAAGACAGAGAGAGATACTCACAGGGAAGAGGG
GAAGAGGAAAACGAGAAAGGGAGGAGAGTAACGGAAAGAGATAAAAAAGAAAAGCAG
GTGGCAGAGACACACAGAGAGGGACCCAGAGAAAGCCAGACAGACGCAGGTGGCTGG
CAGCGGGCGCTGTGGGGGTCACAGTAGGGGGACCTGTG

Suitably, the variant may be at least 75%, at least 80%, at least 85%, at least 90%, at least 95%, at least 98% or at least 99% identical to SEQ ID NO: 27. The NPHS1 promoter may comprise or consist of a variant of SEQ ID NO: 27 shown as SEQ ID NO: 28 or SEQ ID NO: 29.

### Exemplary minimal nephrin promoter - 265 bp (SEQ ID NO: 28)

GGCCCTGGGGTCACGGAGGCTGGGGAGGCACCGAGGAACGCGCCTGGCATGTGCTG
ACAGGGAATTTTATGCTCCAGGAGCAAGACAGAGAGAGACACTCACAGGGAAGAGGG
GAAGAGGAAAACGAGAAAGGGAGGAGAGTAACGGAAAGAGATAAAAAAGAAAAGCAG
GTGGCAGAGACACAGAGAGAGGGACCCAGAGAAAGCCAGACAGACGCAGGTGGCTG
GCAGCGGGCGCTGTGGGGGTCACAGTAGGGGGACCTGTC

### Exemplary minimal nephrin promoter variant - 265 bp (SEQ ID NO: 29)

GGCCCTGGGGTCACGGAGGCTGGGGAGGCACCGAGGAACGCGCCTGGCATGTGCTG
ACAGGGGATTTTATGCTCCAGGAGCAAGACAGAGAGAGATACTCACAGGGAAGAGGG
GAAGAGGAAAACGAGAAAGGGAGGAGAGTAACGGAAAGAGATAAAAAAGAAAAGCAG
GTGGCAGAGACACAGAGAGAGGGACCCAGAGAAAGCCAGACAGACGCAGGTGGCTG
GCAGCGGGCGCTGTGGGGGTCACAGTAGGGGGACCTGTC

In some embodiments, the NPHS1 promoter may comprise or consist of the nucleotide sequence shown as SEQ ID NO: 28 or 29, or a variant which is at least 70% identical to SEQ ID NO: 28 or 29. Suitably, the variant may be at least 75%, at least 80%, at least 85%, at least 90%, at least 95%, at least 98% or at least 99% identical to SEQ ID NO: 28 or 29.

### NPHS2 promoter

The vector of the invention may comprise a NPHS2 promoter, or a fragment or derivative thereof. Suitably, the NPHS2 promoter, or fragment or derivative thereof, may be operably linked to the nucleotide sequence encoding a complement protein (e.g. an inhibitor of the complement system).

The NPHS2 gene encodes podocin, which is selectively expressed in podocytes.

The NPHS2 promoter may be a minimal NPHS2 promoter. For example, the NPHS1 promoter may have a length of 0.6 kb or less.

A minimal human NPHS2 promoter has been described in Oleggini R, et al., 2006. Gene Expr. 13(1):59-66. This minimal NPHS2 is a 630bp fragment which has shown expression in podocytes *in vitro.*

Suitably, the NPHS2 promoter may comprise or consist of the nucleotide sequence shown as SEQ ID NO: 5, or a variant which is at least 70% identical to SEQ ID NO: 5.

### Illustrative minimal NPHS2 promoter (SEQ ID NO: 5):

ggaaagttggggatgaggcgaaatttctgattttaccttaaagtgaccctaattcgatgaccttttgtggtttttttctttttctttttctttttta
cttggccctgcccaagcaggacctaaaaacaaacagacaaaaaaggttactaacaactgttcctctccacgaaatctgcagt
aaaaggtaaaagatgtattcgttttgaagagaaaccagagcttgcgatgagcttctgtatctccgtcagccctctagcatgacatta
ggaaccctccaggagatgagtcttcacagcccgggttggcacctgcagacacgcacttttcaacgcccgcaccctgcccgggg
ccggctctcccacccaggcctctctgcttcagcgccgccccggccgtgggagtcggcgggcgcagtccacagctccaccaa
gacacagctgtcggggttccgggtgcgccccgcccgcggccccggtgtcccgcccctcgccctcagcccccacccgacggtct
ttagggtcccccgggcacgccacgcggacccgcagcgactccacagggactgcgctcccgtgcccctagcgctcccgcgctg
ctgctccagccgcccggcagctctgacc

Suitably, the variant may be at least 75%, at least 80%, at least 85%, at least 90%, at least 95%, at least 98% or at least 99% identical to SEQ ID NO: 5.

### Enhancers

The vector of the invention may comprise an enhancer. Suitably, the enhancer may be operably linked to the nucleotide sequence encoding a complement protein (e.g. an inhibitor of the complement system). The enhancer may facilitate expression of the complement protein (e.g. an inhibitor of the complement system) in podocytes.

An "enhancer" is a region of DNA that can be bound by proteins (activators) to increase the likelihood that transcription of a particular gene will occur. Enhancers are cis-acting. They can be located up to 1 Mbp (1,000,000 bp) away from the gene, upstream or downstream from the start site. Any suitable enhancer may be used, the selection of which may be readily made by the skilled person.

The vector of the invention may comprise a podocyte-specific enhancer. Suitably, the enhancer may be operably linked to the nucleotide sequence encoding a complement protein (e.g. an inhibitor of the complement system).

As used herein, a "podocyte-specific enhancer" is an enhancer which preferentially facilitates expression of a gene in podocyte cells. Suitably, a podocyte-specific enhancer may facilitate higher expression of a gene in podocytes as compared to other cell-types. Higher expression in podocytes may be measured for example by measuring the expression of a transgene, e.g. GFP, operably linked to the enhancer, wherein expression of the transgene in podocytes correlates with the ability of the enhancer to facilitate expression of a gene in podocytes. For example, a podocyte-specific enhancer may be an enhancer which facilitates gene expression levels at least 10% higher, at least 20% higher, at least 30% higher, at least 40% higher, at least 50% higher, at least 100% higher, at least 200% higher, at least 300% higher, at least 400% higher, at least 500% higher, or at least 1000% higher in podocytes compared to expression levels in other cell types.

Suitable podocyte-specific enhancer will be well known to those of skill in the art.

Suitably, the podocyte-specific enhancer may be or may be derived from an enhancer associated with a gene with selective expression in human podocytes. Methods to identify the enhancer regions associated with genes will be well known to those of skill in the art.

Preferably, the podocyte-specific enhancer is a NPHS1 or a NPHS2 enhancer, or a fragment or derivative thereof. More preferably, the podocyte-specific enhancer is a NPHS1 enhancer, or a fragment or derivative thereof.

Preferably, the enhancer is a human enhancer, e.g. a human NPHS1 enhancer.

The enhancer may be used with the corresponding promoter, for example the NPHS1 enhancer may be used with the NPHS1 promoter. Alternatively, the enhancer may be used with a different promoter, for example a promoter which is not podocyte-specific e.g. hsp promoter.

The vector of the invention may comprise a promoter-enhancer. Suitably, the promoter-enhancer may be operably linked to the nucleotide sequence encoding a complement protein (e.g. an inhibitor of the complement system). The promoter-enhancer may facilitate expression of the complement protein (e.g. an inhibitor of the complement system) in podocytes. The promoter-enhancer may be a podocyte-specific promoter-enhancer. The promoter-enhancer may be a NPHS1 promoter-enhancer or a NPHS2 promoter-enhancer, or a fragment or derivative thereof.

### NPHS1 enhancer

A NPHS1 enhancer has been described in Guo, G., et al., 2004. Journal of the American Society of Nephrology, 15(11), pp.2851-2856. A 186-bp fragment from the human NPHS1 promoter was capable of directing podocyte-specific expression of a β-galactosidase transgene when placed in front of a heterologous minimal promoter in transgenic mice.

Suitably, the NPHS1 enhancer may comprise or consist of the nucleotide sequence shown as SEQ ID NO: 6, or a variant which is at least 70% identical to SEQ ID NO: 6.

### Illustrative NPHS1 enhancer (SEQ ID NO: 6):

ctgctgagctgggagaccaccttgatctgacttctcccatcttcccagcctaagccaggccctggggtcacggaggctggggagg
caccgaggaacgcgcctggcatgtgctgacaggggattttatgctccagctgggccagctgggaggagcctgctgggcagag
gccagagctgggggctctg

Suitably, the variant may be at least 75%, at least 80%, at least 85%, at least 90%, at least 95%, at least 98% or at least 99% identical to SEQ ID NO: 6.

### Kozak sequence

The vector of the invention may comprise a Kozak sequence. Suitably, the Kozak sequence may be operably linked to the nucleotide sequence encoding a complement protein (e.g. an inhibitor of the complement system). A Kozak sequence may be inserted before the start codon of the complement protein (e.g. an inhibitor of the complement system) to improve the initiation of translation.

Suitable Kozak sequences will be well known to those of skill in the art.

Suitably, the Kozak sequence may comprise or consist of the nucleotide sequence shown as SEQ ID NO: 7, or a variant which is at least 65% identical to SEQ ID NO: 7.

*Illustrative Kozak sequence (SEQ ID NO: 7):*
GCCGCCACCAUGG

Suitably, the variant may be at least 75%, at least 85%, or at least 90% identical to SEQ ID NO: 7.

### Post-transcriptional regulatory elements

The vector of the invention may comprise a post-transcriptional regulatory element. Suitably, the post-transcriptional regulatory element may be operably linked to the nucleotide sequence encoding a complement protein (e.g. an inhibitor of the complement system). The post-transcriptional regulatory element may improve gene expression.

The vector may comprise a Woodchuck Hepatitis Virus Post-transcriptional Regulatory Element (WPRE). Suitably, the WPRE may be operably linked to the nucleotide sequence encoding a complement protein (e.g. an inhibitor of the complement system).

The WPRE sequence may have mutations within the X-antigen promoter and/or the initiation codon of the X-antigen. This may prevent the production of a functional X-antigen.

Suitably, the WPRE may comprise or consist of the nucleotide sequence shown as SEQ ID NO: 8, or a variant which is at least 70% identical to SEQ ID NO: 8.

### Illustrative WPRE (SEQ ID NO: 8):

aatcaacctctggattacaaaatttgtgaaagattgactggtattcttaactatgttgctccttttacgctatgtggatacgctgctttaat
gcctttgtatcatgctattgcttcccgtatggctttcattttctcctccttgtataaatcctggttgctgtctctttatgaggagttgtggcccgtt
gtcaggcaacgtggcgtggtgtgcactgtgtttgctgacgcaacccccactggttggggcattgccaccacctgtcagctcctttcc
gggactttcgctttccccctccctattgccacggcggaactcatcgccgcctgccttgcccgctgctggacaggggctcggctgttg
ggcactgacaattccgtggtgttgtcggggaaatcatcgtcctttccttggctgctcgcctgtgttgccacctggattctgcgcgggac
gtccttctgctacgtcccttcggccctcaatccagcggaccttccttcccgcggcctgctgccggctctgcggcctcttccgcgtcttc
gccttcgccctcagacgagtcggatctccctttgggccgcctccccgc

Suitably, the variant may be at least 75%, at least 80%, at least 85%, at least 90%, at least 95%, at least 98%, at least 99% identical to SEQ ID NO: 8.

### Polyadenylation signal

The vector of the invention may comprise a polyadenylation signal. Suitably, the polyadenylation signal may be operably linked to the nucleotide sequence encoding a complement protein (e.g. an inhibitor of the complement system). The polyadenylation signal may improve gene expression.

Suitable polyadenylation signals include the early SV40 polyadenylation signal (SV40pA),a bovine growth hormone polyadenylation signal (bGH), or a soluble neuropilin-1 polyadenylation signal. Preferably, the polyadenylation signal is a bGH polyadenylation signal or a soluble neuropilin-1 polyadenylation signal.

Suitably, the polyadenylation signal may comprise or consist of the nucleotide sequence shown as SEQ ID NO: 9, or a variant which is at least 70% identical to SEQ ID NO: 9.

### Illustrative bGH poly(A) signal sequence (SEQ ID NO: 9):

ctgtgccttctagttgccagccatctgttgtttgcccctcccccgtgccttccttgaccctggaaggtgccactcccactgtcctttccta
ataaaatgaggaaattgcatcgcattgtctgagtaggtgtcattctattctggggggtggggtggggcaggacagcaaggggga
ggattgggaagacaatagcaggcatgctggggatgcggtgggctctatgg

Suitably, the variant may be at least 75%, at least 80%, at least 85%, at least 90%, at least 95%, at least 98%, at least 99% identical to SEQ ID NO: 9.

Suitably, the polyadenylation signal may comprise or consist of the nucleotide sequence shown as SEQ ID NO: 10, or a variant which is at least 70% identical to SEQ ID NO: 10.

*Illustrative soluble neuropilin-1 polyadenylation signal* (SEQ ID NO: 10):
aaataaaatacgaaatg

Suitably, the variant may be at least 75%, at least 80%, at least 85%, at least 90%, at least 95%, at least 98%, at least 99% identical to SEQ ID NO: 10.

### Complement proteins

The vector of the present invention comprises a nucleotide sequence encoding an inhibitor of the complement system..

The vector may comprise multiple copies (e.g., 2, 3 etc.) of the nucleotide sequence. The nucleotide sequence may be codon-optimised.

The complement system, also known as complement cascade, is a central part of the innate immunity that serves as a first line of defence against foreign and altered host cells. The complement system is composed of plasma proteins produced mainly by the liver or membrane proteins expressed on cell surface. Complement operates in plasma, in tissues, or within cells. Complement proteins collaborate as a cascade to opsonize pathogens and induce a series of inflammatory responses helping immune cells to fight infection and maintain homeostasis (Merle, N.S., et al., 2015. Frontiers in immunology, 6, 262).

There are three pathways of complement activation: the classical, the alternative, and the lectin pathways. The three complement pathways differ in their mechanisms of target recognition but converge in the activation of the central component C3. After this activation, C5 is cleaved, and the assembly of the membrane attack complex (MAC) is initiated. The enzymatic cleavage of C3 and C5 leads to the production and release of anaphylotoxins C3a and C5a.

As used herein, an "inhibitor of the complement system" or "complement inhibitor" is a protein which prevents activation of the complement system. Complement is tightly controlled by these inhibitors, which naturally protect self cells and tissues from unwanted complement activation. Complement inhibitors can regulate complement activation in different stages of the classical, lectin, and alternative pathways. Complement inhibitors are grouped into two categories: soluble inhibitors and membrane-bound inhibitors. Preferably, the inhibitor of the complement system is a soluble complement inhibitor.

Suitably, the complement inhibitor is a naturally-occurring complement inhibitor, or a fragment or derivative thereof.

Soluble complement inhibitors include C1 inhibitor (C1INH), complement factor I (CFI), complement factor H (CFH), complement factor H-like protein 1 (FHL-1), C4 binding protein (C4BP), clusterin and vitronectin.

Membrane-bound regulators include CD46, CD55, CD59, CD35 and CUB and Sushi multiple domain 1 (CSMD1).

The inhibitor of the complement system may be selected from: CFI, CFH, FHL-1, C1INH, C4BP, CD46, CD55, CD59, CD35, vitronectin and clusterin, or fragments or derivatives thereof. In an embodiment not forming part of the claimed invention the inhibitor of the complement system is CSMD1
Preferably, the inhibitor of the complement system is selected from: CFI, CFH, and FHL-1, or fragments or derivatives thereof.

Preferably, the inhibitor of the complement system is an inhibitor of the complement system in humans.

### CFI

The vector of the present invention may comprise a nucleotide sequence encoding CFI, or a fragment or derivative thereof.

Complement factor I (CFI) is a trypsin-like serine protease that inhibits the complement system by cleaving three peptide bonds in the alpha-chain of C3b and two bonds in the alpha-chain of C4b thereby inactivating these proteins.

CFI is a glycoprotein heterodimer consisting of a disulfide linked heavy chain and light chain. The heavy chain has four domains: an FI membrane attack complex (FIMAC) domain, CD5 domain, and low density lipoprotein receptor 1 and 2 (LDLr1 and LDLr2) domains. The heavy chain plays an inhibitory role in maintaining the enzyme inactive until it meets the complex formed by the substrate (either C3b or C4b) and a cofactor protein (Factor H, C4b-binding protein, complement receptor 1, and membrane cofactor protein). Upon binding of the enzyme to the substrate:cofactor complex, the heavy:light chain interface is disrupted, and the enzyme activated by allostery. The light chain contains only the serine protease domain. This domain contains the catalytic triad His-362, Asp-411, and Ser-507, which is responsible for specific cleavage of C3b and C4b.

The CFI or a fragment or derivative thereof may be capable of cleaving C3b into iC3b and/or may be capable of cleaving iC3b into C3d,g.

The fragment or derivative of CFI may retain at least 50%, 60%, 70%, 80%, 90%, 95% or 100% of the C3b-inactivating and iC3b-degradation activity of native CFI. The C3b-inactivating and iC3b-degradation activity of the fragment or derivative of CFI and native CFI, may be determined using any suitable method known to those of skill in the art. For example, using a proteolytic assay.

Preferably, the CFI is a human CFI. An example human CFI is the CFI having the UniProtKB accession number P05156.

Suitably, the CFI may comprise or consist of the polypeptide sequence shown as SEQ ID NO: 11, or a variant which is at least 70% identical to SEQ ID NO: 11.

### Illustrative CFI polypeptide sequence (SEQ ID NO: 11):

MKLLHVFLLFLCFHLRFCKVTYTSQEDLVEKKCLAKKYTHLSCDKVFCQPWQRCIEGTCVC
KLPYQCPKNGTAVCATNRRSFPTYCQQKSLECLHPGTKFLNNGTCTAEGKFSVSLKHGNT
DSEGIVEVKLVDQDKTMFICKSSWSMREANVACLDLGFQQGADTQRRFKLSDLSINSTECL
HVHCRGLETSLAECTFTKRRTMGYQDFADVVCYTQKADSPMDDFFQCVNGKYISQMKAC
DGINDCGDQSDELCCKACQGKGFHCKSGVCIPSQYQCNGEVDCITGEDEVGCAGFASVT
QEETEILTADMDAERRRIKSLLPKLSCGVKNRMHIRRKRIVGGKRAQLGDLPWQVAIKDASG
ITCGGIYIGGCWILTAAHCLRASKTHRYQIWTTVVDWIHPDLKRIVIEYVDRIIFHENYNAGTY
QNDIALIEMKKDGNKKDCELPRSIPACVPWSPYLFQPNDTCIVSGWGREKDNERVFSLQW
GEVKLISNCSKFYGNRFYEKEMECAGTYDGSIDACKGDSGGPLVCMDANNVTYVWGVVS
WGENCGKPEFPGVYTKVANYFDWISYHVGRPFISQYNV

Suitably, the variant may be at least 75%, at least 80%, at least 85%, at least 90%, at least 95%, at least 98%, at least 99% identical to SEQ ID NO: 11.

An example nucleotide sequence encoding CFI is NM_000204.5. Suitably, the nucleotide sequence encoding CFI may comprise or consist of the polynucleotide sequence shown as SEQ ID NO: 12, or a variant which is at least 70% identical to SEQ ID NO: 12.

### Illustrative CFI polynucleotide sequence (SEQ ID NO: 12):

atgaagcttcttcatgttttcctgttatttctgtgcttccacttaaggttttgcaaggtcacttatacatctcaagaggatctggtggagaaa
aagtgcttagcaaaaaatatactcacctctcctgcgataaagtcttctgccagccatggcagagatgcattgagggcacctgtgt
ttgtaaactaccgtatcagtgcccaaagaatggcactgcagtgtgtgcaactaacaggagaagcttcccaacatactgtcaaca
aaagagtttggaatgtcttcatccagggacaaagtttttaaataacggaacatgcacagccgaaggaaagtttagtgtttccttgaa
gcatggaaatacagattcagagggaatagttgaagtaaaacttgtggaccaagataagacaatgttcatatgcaaaagcagct
ggagcatgagggaagccaacgtggcctgccttgaccttgggtttcaacaaggtgctgatactcaaagaaggtttaagttgtctgat
ctctctataaattccactgaatgtctacatgtgcattgccgaggattagagaccagtttggctgaatgtacttttactaagagaagaa
ctatgggttaccaggatttcgctgatgtggtttgttatacacagaaagcagattctccaatggatgacttctttcagtgtgtgaatggga
aatacatttctcagatgaaagcctgtgatggtatcaatgattgtggagaccaaagtgatgaactgtgttgtaaagcatgccaaggc
aaaggcttccattgcaaatcgggtgtttgcattccaagccagtatcaatgcaatggtgaggtggactgcattacaggggaagatg
aagttggctgtgcaggctttgcatctgtgactcaagaagaaacagaaattttgactgctgacatggatgcagaaagaagacggat
aaaatcattattacctaaactatcttgtggagttaaaaacagaatgcacattcgaaggaaacgaattgtgggaggaaagcgagc
acaactgggagacctcccatggcaggtggcaattaaggatgccagtggaatcacctgtgggggaatttatattggtggctgttgg
attctgactgctgcacattgtctcagagccagtaaaactcatcgttaccaaatatggacaacagtagtagactggatacaccccg
accttaaacgtatagtaattgaatacgtggatagaattattttccatgaaaactacaatgcaggcacttaccaaaatgacatcgcttt
gattgaaatgaaaaaagacggaaacaaaaaagattgtgagctgcctcgttccatccctgcctgtgtcccctggtctccttacctatt
ccaacctaatgatacatgcatcgtttctggctggggacgagaaaaagataacgaaagagtcttttcacttcagtggggtgaagtta
aactaataagcaactgctctaagttttacggaaatcgtttctatgaaaaagaaatggaatgtgcaggtacatatgatggttccatcg
atgcctgtaaaggggactctggaggccccttagtctgtatggatgccaacaatgtgacttatgtctggggtgttgtgagttgggggg
aaaactgtggaaaaccagagttcccaggtgtttacaccaaagtggccaattattttgactggattagctaccatgtaggaaggcct
tttatttctcagtacaatgtataa

Suitably, the variant may be at least 75%, at least 80%, at least 85%, at least 90%, at least 95%, at least 98%, at least 99% identical to SEQ ID NO: 12.

### CFH

The vector of the present invention may comprise a nucleotide sequence encoding CFH, or a fragment or derivative thereof.

Complement factor H (CFH) regulates complement activation on self cells and surfaces. CFH competes for binding of complement factor B (CFB) to C3b, acts as a cofactor for CFI-catalysed proteolytic cleavage of C3b, and accelerates the irreversible dissociation of C3bBb and C3b2Bb into their separate components. Thus, CFH not only inhibits formation of the convertases but it also shortens the lifespan of any convertase complex that forms.

CFH is a large (155 kDa) soluble glycoprotein. CFH is composed from a total of 20 domains, each containing approximately 60 amino acid residues and termed complement control protein modules (CCPs) or short consensus repeats that are joined by short linkers consisting of 3-8 residues. The CCP modules are numbered from 1-20 (from the N-terminus of the protein): CCPs 1-4 and CCPs 19-20 engage with C3b while CCPs 7 and CCPs 19-20 bind to GAGs and sialic acid.

The CFH or a fragment or derivative thereof may be capable of binding C3b and/or C3d; and/or acting as a cofactor for the CFI-catalysed proteolytic cleavage of C3b; and/or increasing the irreversible dissociation of C3bBb and C3b2Bb into their separate components. The fragment or derivative of CFH may retain at least 50%, 60%, 70%, 80%, 90%, 95% or 100% of the activity of native CFH. The activity of the fragment or derivative of CFH and native CFH may be determined using any suitable method known to those of skill in the art.

Preferably, the CFH is a human CFH. An example human CFH is the CFH having the UniProtKB accession number P08603.

Suitably, the CFH may comprise or consist of the polypeptide sequence shown as SEQ ID NO: 13, or a variant which is at least 70% identical to SEQ ID NO: 13.

### Illustrative CFH polypeptide sequence (SEQ ID NO: 13):

MRLLAKIICLMLWAICVAEDCNELPPRRNTEILTGSWSDQTYPEGTQAIYKCRPGYRSLGNVI
MVCRKGEWVALNPLRKCQKRPCGHPGDTPFGTFTLTGGNVFEYGVKAVYTCNEGYQLLG
EINYRECDTDGWTNDIPICEVVKCLPVTAPENGKIVSSAMEPDREYHFGQAVRFVCNSGYKI
EGDEEMHCSDDGFWSKEKPKCVEISCKSPDVINGSPISQKIIYKENERFQYKCNMGYEYSE
RGDAVCTESGWRPLPSCEEKSCDNPYIPNGDYSPLRIKHRTGDEITYQCRNGFYPATRGN
TAKCTSTGWIPAPRCTLKPCDYPDIKHGGLYHENMRRPYFPVAVGKYYSYYCDEHFETPS
GSYWDHIHCTQDGWSPAVPCLRKCYFPYLENGYNQNYGRKFVQGKSIDVACHPGYALPK
AQTTVTCMENGWSPTPRCIRVKTCSKSSIDIENGFISESQYTYALKEKAKYQCKLGYVTADG
ETSGSITCGKDGWSAQPTCIKSCDIPVFMNARTKNDFTWFKLNDTLDYECHDGYESNTGST
TGSIVCGYNGWSDLPICYERECELPKIDVHLVPDRKKDQYKVGEVLKFSCKPGFTIVGPNSV
QCYHFGLSPDLPICKEQVQSCGPPPELLNGNVKEKTKEEYGHSEVVEYYCNPRFLMKGPN
KIQCVDGEWTTLPVCIVEESTCGDIPELEHGWAQLSSPPYYYGDSVEFNCSESFTMIGHRSI
TCIHGVWTQLPQCVAIDKLKKCKSSNLIILEEHLKNKKEFDHNSNIRYRCRGKEGWIHTVCIN
GRWDPEVNCSMAQIQLCPPPPQIPNSHNMTTTLNYRDGEKVSVLCQENYLIQEGEEITCKD

GRWQSIPLCVEKIPCSQPPQIEHGTINSSRSSQESYAHGTKLSYTCEGGFRISEENETTCYM
GKWSSPPQCEGLPCKSPPEISHGVVAHMSDSYQYGEEVTYKCFEGFGIDGPAIAKCLGEK
WSHPPSCIKTDCLSLPSFENAIPMGEKKDVYKAGEQVTYTCATYYKMDGASNVTCINSRWT
GRPTCRDTSCVNPPTVQNAYIVSRQMSKYPSGERVRYQCRSPYEMFGDEEVMCLNGNWT
EPPQCKDSTGKCGPPPPIDNGDITSFPLSVYAPASSVEYQCQNLYQLEGNKRITCRNGQW
SEPPKCLHPCVISREIMENYNIALRWTAKQKLYSRTGESVEFVCKRGYRLSSRSHTLRTTC
WDGKLEYPTCAKR

Suitably, the variant may be at least 75%, at least 80%, at least 85%, at least 90%, at least 95%, at least 98%, at least 99% identical to SEQ ID NO: 13.

An example nucleotide sequence encoding CFH is NM_000186.4. Suitably, the nucleotide sequence encoding CFH may comprise or consist of the polynucleotide sequence shown as SEQ ID NO: 14, or a variant which is at least 70% identical to SEQ ID NO: 14.

### Illustrative CFH polynucleotide sequence (SEQ ID NO: 14):

atgagacttctagcaaagattatttgccttatgttatgggctatttgtgtagcagaagattgcaatgaacttcctccaagaagaaatac
agaaattctgacaggttcctggtctgaccaaacatatccagaaggcacccaggctatctataaatgccgccctggatatagatctc
ttggaaatgtaataatggtatgcaggaagggagaatgggttgctcttaatccattaaggaaatgtcagaaaaggccctgtggaca
tcctggagatactccttttggtacttttacccttacaggaggaaatgtgtttgaatatggtgtaaaagctgtgtatacatgtaatgaggg
gtatcaattgctaggtgagattaattaccgtgaatgtgacacagatggatggaccaatgatattcctatatgtgaagttgtgaagtgtt
taccagtgacagcaccagagaatggaaaaattgtcagtagtgcaatggaaccagatcgggaataccattttggacaagcagta
cggtttgtatgtaactcaggctacaagattgaaggagatgaagaaatgcattgttcagacgatggtttttggagtaaagagaaacc
aaagtgtgtggaaatttcatgcaaatccccagatgttataaatggatctcctatatctcagaagattatttataaggagaatgaacg
atttcaatataaatgtaacatgggttatgaatacagtgaaagaggagatgctgtatgcactgaatctggatggcgtccgttgccttca
tgtgaagaaaaatcatgtgataatccttatattccaaatggtgactactcacctttaaggattaaacacagaactggagatgaaatc
acgtaccagtgtagaaatggtttttatcctgcaacccggggaaatacagcaaaatgcacaagtactggctggatacctgctccga
gatgtaccttgaaaccttgtgattatccagacattaaacatggaggtctatatcatgagaatatgcgtagaccatactttccagtagc
tgtaggaaaatattactcctattactgtgatgaacattttgagactccgtcaggaagttactgggatcacattcattgcacacaagat
ggatggtcgccagcagtaccatgcctcagaaaatgttattttccttatttggaaaatggatataatcaaaatcatggaagaaagtttg
tacagggtaaatctatagacgttgcctgccatcctggctacgctcttccaaaagcgcagaccacagttacatgtatggagaatgg
ctggtctcctactcccagatgcatccgtgtcaaaacatgttccaaatcaagtatagatattgagaatgggtttatttctgaatctcagta
tacatatgccttaaaagaaaaagcgaaatatcaatgcaaactaggatatgtaacagcagatggtgaaacatcaggatcaattac
atgtgggaaagatggatggtcagctcaacccacgtgcattaaatcttgtgatatcccagtatttatgaatgccagaactaaaaatg
acttcacatggtttaagctgaatgacacattggactatgaatgccatgatggttatgaaagcaatactggaagcaccactggttcc
atagtgtgtggttacaatggttggtctgatttacccatatgttatgaaagagaatgcgaacttcctaaaatagatgtacacttagttcct
gatcgcaagaaagaccagtataaagttggagaggtgttgaaattctcctgcaaaccaggatttacaatagttggacctaattccgt

tcagtgctaccactttggattgtctcctgacctcccaatatgtaaagagcaagtacaatcatgtggtccacctcctgaactcctcaat
gggaatgttaaggaaaaaacgaaagaagaatatggacacagtgaagtggtggaatattattgcaatcctagatttctaatgaag
ggacctaataaaattcaatgtgttgatggagagtggacaactttaccagtgtgtattgtggaggagagtacctgtggagatatacct
gaacttgaacatggctgggcccagctttcttcccctccttattactatggagattcagtggaattcaattgctcagaatcatttacaatg
attggacacagatcaattacgtgtattcatggagtatggacccaacttccccagtgtgtggcaatagataaacttaagaagtgcaa
atcatcaaatttaattatacttgaggaacatttaaaaaacaagaaggaattcgatcataattctaacataaggtacagatgtagag
gaaaagaaggatggatacacacagtctgcataaatggaagatggatccagaagtgaactgctcaatggcacaaatacaatt
atgcccacctccacctcagattcccaattctcacaatatgacaaccacactgaattatcgggatggagaaaaagtatctgttctttg
ccaagaaaattatctaattcaggaaggagaagaaattacatgcaaagatggaagatggcagtcaataccactctgtgttgaaa
aaattccatgttcacaaccacctcagatagaacacggaaccattaattcatccaggtcttcacaagaaagttatgcacatgggac
taaattgagttatacttgtgagggtggtttcaggatatctgaagaaaatgaaacaacatgctacatgggaaaatggagttctccac
ctcagtgtgaaggccttccttgtaaatctccacctgagatttctcatggtgttgtagctcacatgtcagacagttatcagtatggagaa
gaagttacgtacaaatgttttgaaggttttggaattgatggcctgcaattgcaaaatgcttaggagaaaaatggtctcaccctccat
catgcataaaaacagattgtctcagtttacctagctttgaaaatgccatacccatgggagagaagaaggatgtgtataaggcggg
tgagcaagtgacttacacttgtgcaacatattacaaaatggatggagccagtaatgtaacatgcattaatagcagatggacagga
aggccaacatgcagagacacctcctgtgtgaatccgcccacagtacaaaatgcttatatagtgtcgagacagatgagtaaatat
ccatctggtgagagagtacgttatcaatgtaggagcccttatgaaatgtttggggatgaagaagtgatgtgtttaaatggaaactgg
acggaaccacctcaatgcaaagattctacaggaaaatgtgggcccccctccacctattgacaatggggacattacttcattcccgtt
gtcagtatatgctccagcttcatcagttgagtaccaatgccagaacttgtatcaacttgagggtaacaagcgaataacatgtagaa
atggacaatggtcagaaccaccaaaatgcttacatccgtgtgtaatatcccgagaaattatggaaaattataacatagcattaag
gtggacagccaaacagaagctttattcgagaacaggtgaatcagttgaatttgtgtgtaaacggggatatcgtctttcatcacgttct
cacacattgcgaacaacatgttgggatgggaaactggagtatccaacttgtgcaaaaagatag

Suitably, the variant may be at least 75%, at least 80%, at least 85%, at least 90%, at least 95%, at least 98%, at least 99% identical to SEQ ID NO: 14.

The CFH fragment may be a splice variant. For example, complement factor H-like protein 1 (FHL-1) is a CFH gene splice variant, which is almost identical to the N-terminal 7 domains of CFH (CCPs 1-7).

### FHL-1

The vector of the present invention may comprise a nucleotide sequence encoding FHL-1, or a fragment or derivative thereof. FHL-1 or a fragment or derivative thereof may be capable of binding C3b and/or C3d. The fragment or derivative of FHL-1 may retain at least 50%, 60%, 70%, 80%, 90%, 95% or 100% of the activity of native FHL-1. The activity of the fragment or derivative of FHL-1 and native FHL-1 may be determined using any suitable method known to those of skill in the art.

Preferably, the FHL-1 is a human FHL-1. An example human FHL-1 is the FHL-1 having the NCBI Reference Sequence: NP_001014975.1.

Suitably, the FHL-1 may comprise or consist of the polypeptide sequence shown as SEQ ID NO: 15, or a variant which is at least 70% identical to SEQ ID NO: 15.

### Illustrative FHL-1 polypeptide sequence (SEQ ID NO: 15):

MRLLAKIICLMLWAICVAEDCNELPPRRNTEILTGSWSDQTYPEGTQAIYKCRPGYRSLGNVI
MVCRKGEWVALNPLRKCQKRPCGHPGDTPFGTFTLTGGNVFEYGVKAVYTCNEGYQLLG
EINYRECDTDGWTNDIPICEVVKCLPVTAPENGKIVSSAMEPDREYHFGQAVRFVCNSGYKI
EGDEEMHCSDDGFWSKEKPKCVEISCKSPDVINGSPISQKIIYKENERFQYKCNMGYEYSE
RGDAVCTESGWRPLPSCEEKSCDNPYIPNGDYSPLRIKHRTGDEITYQCRNGFYPATRGN
TAKCTSTGWIPAPRCTLKPCDYPDIKHGGLYHENMRRPYFPVAVGKYYSYYCDEHFETPS
GSYWDHIHCTQDGWSPAVPCLRKCYFPYLENGYNQNHGRKFVQGKSIDVACHPGYALPK
AQTTVTCMENGWSPTPRCIRVSFTL

Suitably, the variant may be at least 75%, at least 80%, at least 85%, at least 90%, at least 95%, at least 98%, at least 99% identical to SEQ ID NO: 15.

An example nucleotide sequence encoding FHL-1 is NM_001014975.2. Suitably, the nucleotide sequence encoding FHL-1 may comprise or consist of the polynucleotide sequence shown as SEQ ID NO: 16, or a variant which is at least 70% identical to SEQ ID NO: 16.

### Illustrative FHL-1 polynucleotide sequence (SEQ ID NO: 16):

atgagacttctagcaaagattatttgccttatgttatgggctatttgtgtagcagaagattgcaatgaacttcctccaagaagaaatac
agaaattctgacaggttcctggtctgaccaaacatatccagaaggcacccaggctatctataaatgccgccctggatatagatctc
ttggaaatgtaataatggtatgcaggaagggagaatgggttgctcttaatccattaaggaaatgtcagaaaaggccctgtggaca
tcctggagatactccttttggtacttttacccttacaggaggaaatgtgtttgaatatggtgtaaaagctgtgtatacatgtaatgaggg
gtatcaattgctaggtgagattaattaccgtgaatgtgacacagatggatggaccaatgatattcctatatgtgaagttgtgaagtgtt
taccagtgacagcaccagagaatggaaaaattgtcagtagtgcaatggaaccagatcgggaataccattttggacaagcagta
cggtttgtatgtaactcaggctacaagattgaaggagatgaagaaatgcattgttcagacgatggtttttggagtaaagagaaacc
aaagtgtgtggaaatttcatgcaaatccccagatgttataaatggatctcctatatctcagaagattatttataaggagaatgaacg
atttcaatataaatgtaacatgggttatgaatacagtgaaagaggagatgctgtatgcactgaatctggatggcgtccgttgccttca
tgtgaagaaaaatcatgtgataatccttatattccaaatggtgactactcacctttaaggattaaacacagaactggagatgaaatc
acgtaccagtgtagaaatggtttttatcctgcaacccggggaaatacagcaaaatgcacaagtactggctggatacctgctccga
gatgtaccttgaaaccttgtgattatccagacattaaacatggaggtctatatcatgagaatatgcgtagaccatactttccagtagc
tgtaggaaaatattactcctattactgtgatgaacattttgagactccgtcaggaagttactgggatcacattcattgcacacaagat
ggatggtcgccagcagtaccatgcctcagaaaatgttattttccttatttggaaaatggatataatcaaaatcatggaagaaagtttg

tacagggtaaatctatagacgttgcctgccatcctggctacgctcttccaaaagcgcagaccacagttacatgtatggagaatgg
ctggtctcctactcccagatgcatccgtgtcagctttaccctctga

Suitably, the variant may be at least 75%, at least 80%, at least 85%, at least 90%, at least 95%, at least 98%, at least 99% identical to SEQ ID NO: 16.

### Other complement inhibitors

### C11NH

The vector of the present invention may comprise a nucleotide sequence encoding C1INH, or a fragment or derivative thereof.

C1-inhibitor (C1INH) irreversibly binds to and inactivates C1r and C1s proteases in the C1 complex of classical pathway of complement.

Preferably, the C1INH is a human C1INH. An example human C1INH is the C1INH having the UniProtKB accession number P05155. Suitably, the C1INH may comprise or consist of the polypeptide sequence shown as SEQ ID NO: 17, or a variant which is at least 70% identical to SEQ ID NO: 17.

### Illustrative C1INH polypeptide sequence (SEQ ID NO: 17):

Suitably, the variant may be at least 75%, at least 80%, at least 85%, at least 90%, at least 95%, at least 98%, at least 99% identical to SEQ ID NO: 17.

### C4BP

The vector of the present invention may comprise a nucleotide sequence encoding C4BP, or a fragment or derivative thereof.

C4b-binding protein (C4BP) inhibits the action the classical and the lectin pathways, more specifically C4. It also has ability to bind C3b. C4BP accelerates decay of C3-convertase and is a cofactor for CFI which cleaves C4b and C3b. The main form of C4BP in human blood is composed of 7 identical alpha-chains and one unique beta-chain.

Preferably, the C4BP is a human C4BP.

An example human C4BP alpha chain is the C4BP alpha chain having the UniProtKB accession number P04003. Suitably, the C4BP may comprise the polypeptide sequence shown as SEQ ID NO: 18, or a variant which is at least 70% identical to SEQ ID NO: 18.

### Illustrative C4BP alpha chain polypeptide sequence (SEQ ID NO: 18):

MHPPKTPSGALHRKRKMAAWPFSRLWKVSDPILFQMTLIAALLPAVLGNCGPPPTLSFAAP
MDITLTETRFKTGTTLKYTCLPGYVRSHSTQTLTCNSDGEWVYNTFCIYKRCRHPGELRNG
QVEIKTDLSFGSQIEFSCSEGFFLIGSTTSRCEVQDRGVGWSHPLPQCEIVKCKPPPDIRNG
RHSGEENFYAYGFSVTYSCDPRFSLLGHASISCTVENETIGVWRPSPPTCEKITCRKPDVS
HGEMVSGFGPIYNYKDTIVFKCQKGFVLRGSSVIHCDADSKWNPSPPACEPNSCINLPDIPH
ASWETYPRPTKEDVYVVGTVLRYRCHPGYKPTTDEPTTVICQKNLRWTPYQGCEALCCPE
PKLNNGEITQHRKSRPANHCVYFYGDEISFSCHETSRFSAICQGDGTWSPRTPSCGDICNF
PPKIAHGHYKQSSSYSFFKEEIIYECDKGYILVGQAKLSCSYSHWSAPAPQCKALCRKPELV
NGRLSVDKDQYVEPENVTIQCDSGYGVVGPQSITCSGNRTWYPEVPKCEWETPEGCEQV
LTGKRLMQCLPNPEDVKMALEVYKLSLEIEQLELQRDSARQSTLDKEL

Suitably, the variant may be at least 75%, at least 80%, at least 85%, at least 90%, at least 95%, at least 98%, at least 99% identical to SEQ ID NO: 18.

An example human C4BP beta chain is the C4BP alpha chain having the UniProtKB accession number P20851. Suitably, the C4BP may comprise the polypeptide sequence shown as SEQ ID NO: 19, or a variant which is at least 70% identical to SEQ ID NO: 19.

### Illustrative C4BP beta chain polypeptide sequence (SEQ ID NO: 19):

MFFWCACCLMVAWRVSASDAEHCPELPPVDNSIFVAKEVEGQILGTYVCIKGYHLVGKKTL
FCNASKEWDNTTTECRLGHCPDPVLVNGEFSSSGPVNVSDKITFMCNDHYILKGSNRSQC
LEDHTWAPPFPICKSRDCDPPGNPVHGYFEGNNFTLGSTISYYCEDRYYLVGVQEQQCVD
GEWSSALPVCKLIQEAPKPECEKALLAFQESKNLCEAMENFMQQLKESGMTMEELKYSLEL
KKAELKAKLL

Suitably, the variant may be at least 75%, at least 80%, at least 85%, at least 90%, at least 95%, at least 98%, at least 99% identical to SEQ ID NO: 19.

### CD46

The vector of the present invention may comprise a nucleotide sequence encoding CD46, or a fragment or derivative thereof.

CD46 (also known as Membrane Cofactor Protein) acts as a cofactor for CFI.

Preferably, the CD46 is a human CD46. An example human CD46 is the CD46 having the UniProtKB accession number P15529. Suitably, the CD46 may comprise or consist of the polypeptide sequence shown as SEQ ID NO: 20, or a variant which is at least 70% identical to SEQ ID NO: 20.

### Illustrative CD46 polypeptide sequence (SEQ ID NO: 20):

Suitably, the variant may be at least 75%, at least 80%, at least 85%, at least 90%, at least 95%, at least 98%, at least 99% identical to SEQ ID NO: 20.

### CD55

The vector of the present invention may comprise a nucleotide sequence encoding CD55, or a fragment or derivative thereof.

CD55 (also known as DAF) inhibits formation of the C4b2b and C3bBb.

Preferably, the CD55 is a human CD55. An example human CD55 is the CD55 having the UniProtKB accession number P08174. Suitably, the CD55 may comprise or consist of the polypeptide sequence shown as SEQ ID NO: 21, or a variant which is at least 70% identical to SEQ ID NO: 21.

### Illustrative CD55 polypeptide sequence (SEQ ID NO: 21):

MTVARPSVPAALPLLGELPRLLLLVLLCLPAVWGDCGLPPDVPNAQPALEGRTSFPEDTVIT
YKCEESFVKIPGEKDSVICLKGSQWSDIEEFCNRSCEVPTRLNSASLKQPYITQNYFPVGTV
VEYECRPGYRREPSLSPKLTCLQNLKWSTAVEFCKKKSCPNPGEIRNGQIDVPGGILFGATI
SFSCNTGYKLFGSTSSFCLISGSSVQWSDPLPECREIYCPAPPQIDNGIIQGERDHYGYRQS

VTYACNKGFTMIGEHSIYCTVNNDEGEWSGPPPECRGKSLTSKVPPTVQKPTTVNVPTTEV
SPTSQKTTTKTTTPNAQATRSTPVSRTTKHFHETTPNKGSGTTSGTTRLLSGHTCFTLTGLL
GTLVTMGLLT

Suitably, the variant may be at least 75%, at least 80%, at least 85%, at least 90%, at least 95%, at least 98%, at least 99% identical to SEQ ID NO: 21.

### CD59

The vector of the present invention may comprise a nucleotide sequence encoding CD59, or a fragment or derivative thereof.

CD59 (also known as MAC-IP or MIRL) can prevent C9 from polymerizing and forming the complement membrane attack complex. CD59 may also signal the cell to perform active measures such as endocytosis of the CD59-CD9 complex.

Preferably, the CD59 is a human CD59. An example human CD59 is the CD59 having the UniProtKB accession number P13987. Suitably, the CD59 may comprise or consist of the polypeptide sequence shown as SEQ ID NO: 22, or a variant which is at least 70% identical to SEQ ID NO: 22.

### Illustrative CD59 polypeptide sequence (SEQ ID NO: 22):

MGIQGGSVLFGLLLVLAVFCHSGHSLQCYNCPNPTADCKTAVNCSSDFDACLITKAGLQVY
NKCWKFEHCNFNDVTTRLRENELTYYCCKKDLCNFNEQLENGGTSLSEKTVLLLVTPFLAA
AWSLHP

Suitably, the variant may be at least 75%, at least 80%, at least 85%, at least 90%, at least 95%, at least 98%, at least 99% identical to SEQ ID NO: 22.

### CD35

The vector of the present invention may comprise a nucleotide sequence encoding CD35, or a fragment or derivative thereof.

CD35 (also known as Complement receptor type 1 (CR1)) serves as the main system for processing and clearance of complement opsonized immune complexes. It has been shown that CR1 can act as a negative regulator of the complement cascade, mediate immune adherence and phagocytosis and inhibit both the classic and alternative pathways.

Preferably, the CD35 is a human CD35. An example human CD35 is the CD35 having the UniProtKB accession number P17927. Suitably, the CD35 may comprise or consist of the polypeptide sequence shown as SEQ ID NO: 23, or a variant which is at least 70% identical to SEQ ID NO: 23.

### Illustrative CD35 polypeptide sequence (SEQ ID NO: 23):

MGASSPRSPEPVGPPAPGLPFCCGGSLLAVVVLLALPVAWGQCNAPEWLPFARPTNLTDE
FEFPIGTYLNYECRPGYSGRPFSIICLKNSVWTGAKDRCRRKSCRNPPDPVNGMVHVIKGI
QFGSQIKYSCTKGYRLIGSSSATCIISGDTVIWDNETPICDRIPCGLPPTITNGDFISTNRENF
HYGSVVTYRCNPGSGGRKVFELVGEPSIYCTSNDDQVGIWSGPAPQCIIPNKCTPPNVENG
ILVSDNRSLFSLNEVVEFRCQPGFVMKGPRRVKCQALNKWEPELPSCSRVCQPPPDVLHA
ERTQRDKDNFSPGQEVFYSCEPGYDLRGAASMRCTPQGDWSPAAPTCEVKSCDDFMGQ
LLNGRVLFPVNLQLGAKVDFVCDEGFQLKGSSASYCVLAGMESLWNSSVPVCEQIFCPSP
PVIPNGRHTGKPLEVFPFGKTVNYTCDPHPDRGTSFDLIGESTIRCTSDPQGNGVWSSPAP
RCGILGHCQAPDHFLFAKLKTQTNASDFPIGTSLKYECRPEYYGRPFSITCLDNLVWSSPKD
VCKRKSCKTPPDPVNGMVHVITDIQVGSRINYSCTTGHRLIGHSSAECILSGNAAHWSTKPP
ICQRIPCGLPPTIANGDFISTNRENFHYGSVVTYRCNPGSGGRKVFELVGEPSIYCTSNDDQ
VGIWSGPAPQCIIPNKCTPPNVENGILVSDNRSLFSLNEVVEFRCQPGFVMKGPRRVKCQA
LNKWEPELPSCSRVCQPPPDVLHAERTQRDKDNFSPGQEVFYSCEPGYDLRGAASMRCT
PQGDWSPAAPTCEVKSCDDFMGQLLNGRVLFPVNLQLGAKVDFVCDEGFQLKGSSASYC
VLAGMESLWNSSVPVCEQIFCPSPPVIPNGRHTGKPLEVFPFGKAVNYTCDPHPDRGTSFD
LIGESTIRCTSDPQGNGVWSSPAPRCGILGHCQAPDHFLFAKLKTQTNASDFPIGTSLKYEC
RPEYYGRPFSITCLDNLVWSSPKDVCKRKSCKTPPDPVNGMVHVITDIQVGSRINYSCTTG
HRLIGHSSAECILSGNTAHWSTKPPICQRIPCGLPPTIANGDFISTNRENFHYGSVVTYRCNL
GSRGRKVFELVGEPSIYCTSNDDQVGIWSGPAPQCIIPNKCTPPNVENGILVSDNRSLFSLN
EVVEFRCQPGFVMKGPRRVKCQALNKWEPELPSCSRVCQPPPEILHGEHTPSHQDNFSP
GQEVFYSCEPGYDLRGAASLHCTPQGDWSPEAPRCAVKSCDDFLGQLPHGRVLFPLNLQ
LGAKVSFVCDEGFRLKGSSVSHCVLVGMRSLWNNSVPVCEHIFCPNPPAILNGRHTGTPS
GDIPYGKEISYTCDPHPDRGMTFNLIGESTIRCTSDPHGNGVWSSPAPRCELSVRAGHCKT
PEQFPFASPTIPINDFEFPVGTSLNYECRPGYFGKMFSISCLENLVWSSVEDNCRRKSCGP
PPEPFNGMVHINTDTQFGSTVNYSCNEGFRLIGSPSTTCLVSGNNVTWDKKAPICEIISCEP
PPTISNGDFYSNNRTSFHNGTVVTYQCHTGPDGEQLFELVGERSIYCTSKDDQVGVWSSP
PPRCISTNKCTAPEVENAIRVPGNRSFFSLTEIIRFRCQPGFVMVGSHTVQCQTNGRWGPK
LPHCSRVCQPPPEILHGEHTLSHQDNFSPGQEVFYSCEPSYDLRGAASLHCTPQGDWSPE
APRCTVKSCDDFLGQLPHGRVLLPLNLQLGAKVSFVCDEGFRLKGRSASHCVLAGMKALW
NSSVPVCEQIFCPNPPAILNGRHTGTPFGDIPYGKEISYACDTHPDRGMTFNLIGESSIRCTS
DPQGNGVWSSPAPRCELSVPAACPHPPKIQNGHYIGGHVSLYLPGMTISYICDPGYLLVGK
GFIFCTDQGIWSQLDHYCKEVNCSFPLFMNGISKELEMKKVYHYGDYVTLKCEDGYTLEGS

PWSQCQADDRWDPPLAKCTSRTHDALIVGTLSGTIFFILLIIFLSWIILKHRKGNNAHENPKEV
AIHLHSQGGSSVHPRTLQTNEENSRVLP

Suitably, the variant may be at least 75%, at least 80%, at least 85%, at least 90%, at least 95%, at least 98%, at least 99% identical to SEQ ID NO: 23.

### Vitronectin

The vector of the present invention may comprise a nucleotide sequence encoding vitronectin, or a fragment or derivative thereof.

Vitronectin inhibits the membrane-damaging effect of the terminal cytolytic complement pathway.

Preferably, the vitronectin is a human vitronectin. An example human vitronectin is the vitronectin having the UniProtKB accession number P04004. Suitably, the vitronectin may comprise or consist of the polypeptide sequence shown as SEQ ID NO: 24, or a variant which is at least 70% identical to SEQ ID NO: 24.

### Illustrative vitronectin polypeptide sequence (SEQ ID NO: 24):

MAPLRPLLILALLAWVALADQESCKGRCTEGFNVDKKCQCDELCSYYQSCCTDYTAECKP
QVTRGDVFTMPEDEYTVYDDGEEKNNATVHEQVGGPSLTSDLQAQSKGNPEQTPVLKPE
EEAPAPEVGASKPEGIDSRPETLHPGRPQPPAEEELCSGKPFDAFTDLKNGSLFAFRGQYC
YELDEKAVRPGYPKLIRDVWGIEGPIDAAFTRINCQGKTYLFKGSQYWRFEDGVLDPDYPR
NISDGFDGIPDNVDAALALPAHSYSGRERVYFFKGKQYWEYQFQHQPSQEECEGSSLSAV
FEHFAMMQRDSWEDIFELLFWGRTSAGTRQPQFISRDWHGVPGQVDAAMAGRIYISGMA
PRPSLAKKQRFRHRNRKGYRSQRGHSRGRNQNSRRPSRATWLSLFSSEESNLGANNYDD
YRMDWLVPATCEPIQSVFFFSGDKYYRVNLRTRRVDTVDPPYPRSIAQYWLGCPAPGHL

Suitably, the variant may be at least 75%, at least 80%, at least 85%, at least 90%, at least 95%, at least 98%, at least 99% identical to SEQ ID NO: 24.

### Clusterin

The vector of the present invention may comprise a nucleotide sequence encoding clusterin, or a fragment or derivative thereof.

Clusterin inhibits the membrane-damaging effect of the terminal cytolytic complement pathway.

Preferably, the clusterin is a human clusterin. An example human clusterin is the clusterin having the UniProtKB accession number P10909. Suitably, the clusterin may comprise or consist of the polypeptide sequence shown as SEQ ID NO: 25, or a variant which is at least 70% identical to SEQ ID NO: 25.

### Illustrative clusterin polypeptide sequence (SEQ ID NO: 25):

MMKTLLLFVGLLLTWESGQVLGDQTVSDNELQEMSNQGSKYVNKEIQNAVNGVKQIKTLIE
KTNEERKTLLSNLEEAKKKKEDALNETRESETKLKELPGVCNETMMALWEECKPCLKQTC
MKFYARVCRSGSGLVGRQLEEFLNQSSPFYFWMNGDRIDSLLENDRQQTHMLDVMQDHF
SRASSIIDELFQDRFFTREPQDTYHYLPFSLPHRRPHFFFPKSRIVRSLMPFSPYEPLNFHA
MFQPFLEMIHEAQQAMDIHFHSPAFQHPPTEFIREGDDDRTVCREIRHNSTGCLRMKDQCD
KCREILSVDCSTNNPSQAKLRRELDESLQVAERLTRKYNELLKSYQWKMLNTSSLLEQLNE
QFNWVSRLANLTQGEDQYYLRVTTVASHTSDSDVPSGVTEVVVKLFDSDPITVTVPVEVSR
KNPKFMETVAEKALQEYRKKHREE

Suitably, the variant may be at least 75%, at least 80%, at least 85%, at least 90%, at least 95%, at least 98%, at least 99% identical to SEQ ID NO: 25.

### CSMD1

In an embodiment not forming part of the claimed invention the vector may comprise a nucleotide sequence encoding CSMD1, or a fragment or derivative thereof.

CUB and Sushi multiple domains 1 (CSMD1) can inhibit complement activation by promoting CFI-mediated C4b/C3b degradation and by inhibiting the MAC assembly.

Preferably, the CSMD1 is a human CSMD1. An example human CSMD1 is the CSMD1 having the UniProtKB accession number Q96PZ7. Suitably, the CSMD1 may comprise or consist of the polypeptide sequence shown as SEQ ID NO: 26, or a variant which is at least 70% identical to SEQ ID NO: 26.

### Illustrative CSMD1 polypeptide sequence (SEQ ID NO: 26):

MTAWRRFQSLLLLLGLLVLCARLLTAAKGQNCGGLVQGPNGTIESPGFPHGYPNYANCTWI
IITGERNRIQLSFHTFALEEDFDILSVYDGQPQQGNLKVRLSGFQLPSSIVSTGSILTLWFTTD
FAVSAQGFKALYEVLPSHTCGNPGEILKGVLHGTRFNIGDKIRYSCLPGYILEGHAILTCIVSP
GNGASWDFPAPFCRAEGACGGTLRGTSSSISSPHFPSEYENNADCTWTILAEPGDTIALVF
TDFQLEEGYDFLEISGTEAPSIWLTGMNLPSPVISSKNWLRLHFTSDSNHRRKGFNAQFQV
KKAIELKSRGVKMLPSKDGSHKNSVLSQGGVALVSDMCPDPGIPENGRRAGSDFRVGANV

QFSCEDNYVLQGSKSITCQRVTETLAAWSDHRPICRARTCGSNLRGPSGVITSPNYPVQYE
DNAHCVWVITTTDPDKVIKLAFEEFELERGYDTLTVGDAGKVGDTRSVLYVLTGSSVPDLIV
SMSNQMWLHLQSDDSIGSPGFKAVYQEIEKGGCGDPGIPAYGKRTGSSFLHGDTLTFECP
AAFELVGERVITCQQNNQWSGNKPSCVFSCFFNFTASSGIILSPNYPEEYGNNMNCVWLIIS
EPGSRIHLIFNDFDVEPQFDFLAVKDDGISDITVLGTFSGNEVPSQLASSGHIVRLEFQSDHS
TTGRGFNITYTTFGQNECHDPGIPINGRRFGDRFLLGSSVSFHCDDGFVKTQGSESITCILQ
DGNVVWSSTVPRCEAPCGGHLTASSGVILPPGWPGYYKDSLHCEWIIEAKPGHSIKITFDR
FQTEVNYDTLEVRDGPASSSPLIGEYHGTQAPQFLISTGNFMYLLFTTDNSRSSIGFLIHYES
VTLESDSCLDPGIPVNGHRHGGDFGIRSTVTFSCDPGYTLSDDEPLVCERNHQWNHALPS
CDALCGGYIQGKSGTVLSPGFPDFYPNSLNCTWTIEVSHGKGVQMIFHTFHLESSHDYLLIT
EDGSFSEPVARLTGSVLPHTIKAGLFGNFTAQLRFISDFSISYEGFNITFSEYDLEPCDDPGV
PAFSRRIGFHFGVGDSLTFSCFLGYRLEGATKLTCLGGGRRVWSAPLPRCVAECGASVKG
NEGTLLSPNFPSNYDNNHECIYKIETEAGKGIHLRTRSFQLFEGDTLKVYDGKDSSSRPLGT
FTKNELLGLILNSTSNHLWLEFNTNGSDTDQGFQLTYTSFDLVKCEDPGIPNYGYRIRDEGH
FTDTVVLYSCNPGYAMHGSNTLTCLSGDRRVWDKPLPSCIAECGGQIHAATSGRILSPGYP
APYDNNLHCTWIIEADPGKTISLHFIVFDTEMAHDILKVWDGPVDSDILLKEWSGSALPEDIH
STFNSLTLQFDSDFFISKSGFSIQFSTSIAATCNDPGMPQNGTRYGDSREAGDTVTFQCDP
GYQLQGQAKITCVQLNNRFFWQPDPPTCIAACGGNLTGPAGVILSPNYPQPYPPGKECDW
RVKVNPDFVIALIFKSFNMEPSYDFLHIYEGEDSNSPLIGSYQGSQAPERIESSGNSLFLAFR
SDASVGLSGFAIEFKEKPREACFDPGNIMNGTRVGTDFKLGSTITYQCDSGYKILDPSSITCV
IGADGKPSWDQVLPSCNAPCGGQYTGSEGVVLSPNYPHNYTAGQICLYSITVPKEFVVFGQ
FAYFQTALNDLAELFDGTHAQARLLSSLSGSHSGETLPLATSNQILLRFSAKSGASARGFHF
VYQAVPRTSDTQCSSVPEPRYGRRIGSEFSAGSIVRFECNPGYLLQGSTALHCQSVPNALA
QWNDTIPSCVVPCSGNFTQRRGTILSPGYPEPYGNNLNCIWKIIVTEGSGIQIQVISFATEQN
WDSLEIHDGGDVTAPRLGSFSGTTVPALLNSTSNQLYLHFQSDISVAAAGFHLEYKTVGLAA
CQEPALPSNSIKIGDRYMVNDVLSFQCEPGYTLQGRSHISCMPGTVRRWNYPSPLCIATCG
GTLSTLGGVILSPGFPGSYPNNLDCTWRISLPIGYGAHIQFLNFSTEANHDFLEIQNGPYHTS
PMIGQFSGTDLPAALLSTTHETLIHFYSDHSQNRQGFKLAYQAYELQNCPDPPPFQNGYMI
NSDYSVGQSVSFECYPGYILIGHPVLTCQHGINRNWNYPFPRCDAPCGYNVTSQNGTIYSP
GFPDEYPILKDCIWLITVPPGHGVYINFTLLQTEAVNDYIAVWDGPDQNSPQLGVFSGNTAL
ETAYSSTNQVLLKFHSDFSNGGFFVLNFHAFQLKKCQPPPAVPQAEMLTEDDDFEIGDFVK
YQCHPGYTLVGTDILTCKLSSQLQFEGSLPTCEAQCPANEVRTGSSGVILSPGYPGNYFNS
QTCSWSIKVEPNYNITIFVDTFQSEKQFDALEVFDGSSGQSPLLVVLSGNHTEQSNFTSRSN
QLYLRWSTDHATSKKGFKIRYAAPYCSLTHPLKNGGILNRTAGAVGSKVHYFCKPGYRMVG
HSNATCRRNPLGMYQWDSLTPLCQAVSCGIPESPGNGSFTGNEFTLDSKVVYECHEGFKL
ESSQQATAVCQEDGLWSNKGKPPTCKPVACPSIEAQLSEHVIWRLVSGSLNEYGAQVLLS
CSPGYYLEGWRLLRCQANGTWNIGDERPSCRVISCGSLSFPPNGNKIGTLTVYGATAIFTC

NTGYTLVGSHVRECLANGLWSGSETRCLAGHCGSPDPIVNGHISGDGFSYRDTVVYQCNP
GFRLVGTSVRICLQDHKWSGQTPVCVPITCGHPGNPAHGFTNGSEFNLNDVVNFTCNTGY
LLQGVSRAQCRSNGQWSSPLPTCRVVNCSDPGFVENAIRHGQQNFPESFEYGMSILYHCK
KGFYLLGSSALTCMANGLWDRSLPKCLAISCGHPGVPANAVLTGELFTYGAVVHYSCRGS
ESLIGNDTRVCQEDSHWSGALPHCTGNNPGFCGDPGTPAHGSRLGDDFKTKSLLRFSCE
MGHQLRGSPERTCLLNGSWSGLQPVCEAVSCGNPGTPTNGMIVSSDGILFSSSVIYACWE
GYKTSGLMTRHCTANGTWTGTAPDCTIISCGDPGTLANGIQFGTDFTFNKTVSYQCNPGYV
MEAVTSATIRCTKDGRWNPSKPVCKAVLCPQPPPVQNGTVEGSDFRWGSSISYSCMDGY
QLSHSAILSCEGRGVWKGEIPQCLPVFCGDPGIPAEGRLSGKSFTYKSEVFFQCKSPFILVG
SSRRVCQADGTWSGIQPTCIDPAHNTCPDPGTPHFGIQNSSRGYEVGSTVFFRCRKGYHI
QGSTTRTCLANLTWSGIQTECIPHACRQPETPAHADVRAIDLPTFGYTLVYTCHPGFFLAGG
SEHRTCKADMKWTGKSPVCKSKGVREVNETVTKTPVPSDVFFVNSLWKGYYEYLGKRQP
ATLTVDWFNATSSKVNATFSEASPVELKLTGIYKKEEAHLLLKAFQIKGQADIFVSKFENDN
WGLDGYVSSGLERGGFTFQGDIHGKDFGKFKLERQDPLNPDQDSSSHYHGTSSGSVAAAI
LVPFFALILSGFAFYLYKHRTRPKVQYNGYAGHENSNGQASFENPMYDTNLKPTEAKAVRF
DTTLNTVCTVV

Suitably, the variant may be at least 75%, at least 80%, at least 85%, at least 90%, at least 95%, at least 98%, at least 99% identical to SEQ ID NO: 26.

### Variants, derivatives, analogues, homologues and fragments

In addition to the specific proteins and nucleotides mentioned herein, the invention also encompasses variants, derivatives, homologues and fragments thereof.

In the context of the invention, a "variant" of any given sequence is a sequence in which the specific sequence of residues (whether amino acid or nucleic acid residues) has been modified in such a manner that the polypeptide or polynucleotide in question retains at least one of its endogenous functions. For example, a variant of a complement inhibitor may retain the ability to inhibit the complement system. A variant sequence can be obtained by addition, deletion, substitution, modification, replacement and/or variation of at least one residue present in the naturally occurring polypeptide or polynucleotide.

The term "derivative" as used herein in relation to proteins or polypeptides of the invention includes any substitution of, variation of, modification of, replacement of, deletion of and/or addition of one (or more) amino acid residues from or to the sequence, providing that the resultant protein or polypeptide retains at least one of its endogenous functions. For example, a derivative of a complement inhibitor may retain the ability to inhibit the complement system.

Typically, amino acid substitutions may be made, for example from 1, 2 or 3, to 10 or 20 substitutions, provided that the modified sequence retains the required activity or ability. Amino acid substitutions may include the use of non-naturally occurring analogues.

Proteins used in the invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" as used herein means a variant having a certain homology with the wild type amino acid sequence or the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 96% or 97% or 98% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 96% or 97% or 98% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye, or more usually, with the aid of readily available sequence comparison programs. These commercially available computer programs can calculate percent homology or identity between two or more sequences.

Percent homology may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, USA; Devereux et al. (1984) Nucleic Acids Research 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410), EMBOSS Needle (Madeira, F., et al., 2019. Nucleic acids research, 47(W1), pp.W636-W641) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, BLAST 2 Sequences, is also available for comparing protein and nucleotide sequences (FEMS Microbiol. Lett. (1999) 174(2):247-50; FEMS Microbiol. Lett. (1999) 177(1):187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix (the default matrix for the BLAST suite of programs). GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result. The percent sequence identity may be calculated as the number of identical residues as a percentage of the total residues in the SEQ ID NO referred to.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants, derivatives, homologues and fragments may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

### Codon optimisation

The polynucleotides used in the invention may be codon-optimised.

Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells (e.g. humans), as well as for a variety of other organisms.

### Cells

In one aspect, the present invention provides a cell comprising the vector of the invention. The cell may be an isolated cell. The cell may be a human cell, suitably an isolated human cell.

Vectors comprising polynucleotides used in the invention may be introduced into cells using a variety of techniques known in the art, such as transfection, transduction and transformation. Suitably, the vector of the present invention is introduced into the cell by transfection or transduction.

The cell may be any cell type known in the prior art.

Suitably, the cell may be a producer cell. The term "producer cell" includes a cell that produces viral particles, after transient transfection, stable transfection or vector transduction of all the elements necessary to produce the viral particles or any cell engineered to stably comprise the elements necessary to produce the viral particles. Suitable producer cells will be well known to those of skill in the art. Suitable producer cell lines include HEK 293 (e.g. HEK 293T), HeLa, and A549 cell lines.

Suitably, the cell may be a packaging cell. The term "packaging cell" includes a cell which contains some or all of the elements necessary for packaging an infectious recombinant virus.

The packaging cell may lack a recombinant viral vector genome. Typically, such packaging cells contain one or more vectors which are capable of expressing viral structural proteins. Cells comprising only some of the elements required for the production of enveloped viral particles are useful as intermediate reagents in the generation of viral particle producer cell lines, through subsequent steps of transient transfection, transduction or stable integration of each additional required element. These intermediate reagents are encompassed by the term "packaging cell". Suitable packaging cells will be well known to those of skill in the art.

Suitably, the cell may be a kidney cell, for example a podocyte. Suitably, the cell may be an immortalized kidney cell, for example an immortalized podocyte. Suitable podocyte cell lines will be well known to those of skill in the art, for example CIHP-1. Methods to generate immortalized podocytes will be well known to those of skill in the art. Suitable methods are described in Ni, L., et al., 2012. Nephrology, 17(6), pp.525-531.

### Pharmaceutical compositions

In one aspect, the present invention provides pharmaceutical composition comprising the vector of the invention or the cell of the invention.

A pharmaceutical composition is a composition that comprises or consists of a therapeutically effective amount of a pharmaceutically active agent i.e. the vector. It preferably includes a pharmaceutically acceptable carrier, diluent or excipient (including combinations thereof).

By "pharmaceutically acceptable" is included that the formulation is sterile and pyrogen free. The carrier, diluent, and/or excipient must be "acceptable" in the sense of being compatible with the vector and not deleterious to the recipients thereof. Typically, the carriers, diluents, and excipients will be saline or infusion media which will be sterile and pyrogen free, however, other acceptable carriers, diluents, and excipients may be used.

Acceptable carriers, diluents, and excipients for therapeutic use are well known in the pharmaceutical art. The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s) or solubilising agent(s).

Examples of pharmaceutically acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

The vector, cell, or pharmaceutical composition according to the present invention may be administered in a manner appropriate for treating and/or preventing the diseases described herein. The quantity and frequency of administration will be determined by such factors as the condition of the subject, and the type and severity of the subject's disease, although appropriate dosages may be determined by clinical trials. The pharmaceutical composition may be formulated accordingly.

The vector, cell or pharmaceutical composition according to the present invention may be administered parenterally, for example, intravenously, or by infusion techniques. The vector, cell or pharmaceutical composition may be administered in the form of a sterile aqueous solution which may contain other substances, for example, enough salts or glucose to make the solution isotonic with blood. The aqueous solution may be suitably buffered (preferably to a pH of from 3 to 9). The pharmaceutical composition may be formulated accordingly. The preparation of suitable parenteral formulations under sterile conditions is readily accomplished by standard pharmaceutical techniques well-known to those skilled in the art.

The vector, cell or pharmaceutical composition according to the present invention may be administered systemically, for example by intravenous injection.

The vector, cell or pharmaceutical composition according to the present invention may be administered locally, for example by targeting administration to the kidney. Suitably, the vector, cell or pharmaceutical composition may be administered by injection into the renal artery or by ureteral or subcapsular injection.

The pharmaceutical compositions may comprise vectors or cells of the invention in infusion media, for example sterile isotonic solution. The pharmaceutical composition may be enclosed in ampoules, disposable syringes or multiple dose vials made of glass or plastic.

The vector, cell or pharmaceutical composition may be administered in a single or in multiple doses. Particularly, the vector, cell or pharmaceutical composition may be administered in a single, one off dose. The pharmaceutical composition may be formulated accordingly.

The vector, cell or pharmaceutical composition may be administered at varying doses (e.g. measured in vector genomes (vg) per kg). The physician in any event will determine the actual dosage which will be most suitable for any individual subject and it will vary with the age, weight and response of the particular subject. Typically, however, for the AAV vectors of the invention, doses of 10¹⁰ to 10¹⁴ vg/kg, or 10¹¹ to 10¹³ vg/kg may be administered.

The pharmaceutical composition may further comprise one or more other therapeutic agents.

The invention further includes the use of kits comprising the vector, cells and/or pharmaceutical composition of the present invention. Preferably said kits are for use in the methods and used as described herein, e.g., the therapeutic methods as described herein. Preferably said kits comprise instructions for use of the kit components.

In one aspect, the present invention provides the vector, cell or pharmaceutical composition according to the present invention for use as a medicament.

In a related aspect not forming part of the claimed invention, is provided the use of the vector, cell or pharmaceutical composition according to the present invention in the manufacture of a medicament.

In a related aspect not forming part of the claimed invention, is provided a method of administering the vector, cell or pharmaceutical composition according to the present invention to a subject in need thereof.

### Methods for treating and/or preventing disease

The vector, cell or pharmaceutical composition according to the present invention may be used to treat complement-mediated kidney diseases in a subject. Suitably, the subject is a human subject.

In one aspect, the present invention provides the vector, cell or pharmaceutical composition according to the present invention for use in preventing or treating a complement-mediated kidney disease.

In a related aspect not forming part of the claimed invention, is provided the use of the vector, cell or pharmaceutical composition according to the present invention for the manufacture of a medicament for preventing or treating a complement-mediated kidney disease.

In a related aspect not forming part of the claimed invention, is provided a method of preventing or treating a complement-mediated kidney disease comprising administering the vector, cell or pharmaceutical composition according to the present invention to a subject in need thereof.

### Complement-mediated kidney disease

As used herein a "complement-mediated kidney disease" is a disease of the kidney which is caused by dysregulation of the complement system. The complement system can cause kidney injury in a variety of different diseases. Suitably, the complement-mediated kidney disease is caused by excessive activation of the complement system.

Exemplary complement-mediated kidney diseases include IgA nephropathy, C3 glomerulopathy, atypical hemolytic uremic syndrome (aHUS), typical hemolytic-uremic syndrome, lupus nephritis, cryoglobulinemia, anti-GBM disease, Antineutrophil cytoplasmic antibody (ANCA)-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III.

The vector, cell or pharmaceutical composition according to the present invention may be administered to a subject with a complement-mediated kidney disease in order to reverse the rejection or slow down progression of the complement-mediated kidney disease, or to lessen, reduce, or improve at least one symptom of the complement-mediated kidney disease.

### IgA Nephropathy

In one aspect, the present invention provides the vector, cell or pharmaceutical composition according to the present invention for use in preventing or treating IgA Nephropathy.

In a related aspect not forming part of the claimed invention, is provided the use of the vector, cell or pharmaceutical composition according to the present invention for the manufacture of a medicament for preventing or treating IgA Nephropathy.

In a related aspect not forming part of the claimed invention, is provided a method of preventing or treating IgA Nephropathy comprising administering the vector, cell or pharmaceutical composition according to the present invention to a subject in need thereof.

IgA nephropathy (IgAN), also known as Berger's disease, or synpharyngitic glomerulonephritis, is a disease of the kidney (or nephropathy) and the immune system; specifically it is a form of glomerulonephritis or an inflammation of the glomeruli of the kidney. IgA nephropathy is the most common glomerulonephritis worldwide

IgA nephropathy is associated with aberrant glycosylation of IgA1 molecules, and the development of autoantibodies specific for the altered IgA1. IgA1-containing immune complexes deposit within the mesangium, and likely initiate glomerular injury. IgA activates the complement system through either the alternative or mannose binding lectin pathway. Secretion of complement inhibitors from podocytes may help locally regulate the complement system in subjects with IgA nephropathy.

The vector, cell or pharmaceutical composition according to the present invention may be administered to a subject with IgA Nephropathy in order to reverse the rejection or slow down progression of IgA Nephropathy, or to lessen, reduce, or improve at least one symptom of IgA Nephropathy such as hematuria and proteinuria. Administration of the vector, cell or pharmaceutical composition may remove IgA from the glomerulus and/or prevent further IgA deposition.

### C3 glomerulopathy

In one aspect, the present invention provides the vector, cell or pharmaceutical composition according to the present invention for use in preventing or treating C3 glomerulopathy.

In a related aspect not forming part of the claimed invention, is provided use of the vector, cell or pharmaceutical composition according to the present invention for the manufacture of a medicament for preventing or treating C3 glomerulopathy.

In a related aspect not forming part of the claimed invention, is provided a method of preventing or treating C3 glomerulopathy comprising administering the vector, cell or pharmaceutical composition according to the present invention to a subject in need thereof.

C3 glomerulopathy is a group of related conditions that includes two over-lapping pathologies: dense deposit disease and C3 glomerulonephritis. The major features of C3 glomerulopathy include high levels of protein in the urine (proteinuria), blood in the urine (hematuria), reduced amounts of urine, low levels of protein in the blood, and swelling in many areas of the body. Electron microscopy is necessary to distinguish the two major subtypes of C3 glomerulopathy, dense deposit disease and C3 glomerulonephritis.

C3 glomerulopathy is diagnosed by detection of prominent glomerular C3 in the relative absence of immunoglobulin, C1q, or C4d. C3 glomerulopathy is caused by excessive activation of the alternative complement pathway due to a genetic or acquired defect in complement regulation. Activated C3 fragments (including C3b, iC3b, C3dg and C3d) are deposited in the glomerular basement membrane, disrupting membrane function and causing an inflammatory response that leads to glomerular damage.

The vector, cell or pharmaceutical composition according to the present invention may be administered to a subject with C3 glomerulopathy in order to reverse the rejection or slow down progression of C3 glomerulopathy, or to lessen, reduce, or improve at least one symptom of C3 glomerulopathy such as hematuria and proteinuria. Administration of the vector, cell or pharmaceutical composition may remove activated C3 fragments from the glomerular basement membrane and/or prevent further deposition of activated C3 fragments.

### Dense deposit disease

**In** one aspect, the present invention provides the vector, cell or pharmaceutical composition according to the present invention for use in preventing or treating dense deposit disease.

**In** a related aspect not forming part of the claimed invention, is provided the use of the vector, cell or pharmaceutical composition according to the present invention for the manufacture of a medicament for preventing or treating dense deposit disease.

**In** a related aspect not forming part of the claimed invention, is provided a method of preventing or treating dense deposit disease comprising administering the vector, cell or pharmaceutical composition according to the present invention to a subject in need thereof.

Dense deposit disease is a C3 glomerulopathy that has been historically classified as membranoproliferative glomerulonephritis type 2.

**In** dense deposit disease, electron microscopy reveals highly electron-dense, osmiophilic deposits with a 'sausage-shaped' or 'Chinese calligraphy-like' appearance that thicken and transform the lamina densa of the glomerular basement membrane (GBM).

### C3 glomerulonephritis

**In** one aspect, the present invention provides the vector, cell or pharmaceutical composition according to the present invention for use in preventing or treating C3 glomerulonephritis.

**In** a related aspect not forming part of the claimed invention, is provided the use of the vector, cell or pharmaceutical composition according to the present invention for the manufacture of a medicament for preventing or treating C3 glomerulonephritis.

In a related aspect not forming part of the claimed invention, is provided a method of preventing or treating C3 glomerulonephritis comprising administering the vector, cell or pharmaceutical composition according to the present invention to a subject in need thereof.

C3 glomerulonephritis is a C3 glomerulopathy that has been historically classified as atypical membranoproliferative glomerulonephritis type 1 and type 3.

In C3 glomerulonephritis, the electron density of deposits approaches that of the glomerular matrix components. These deposits often have an amorphous cloudy appearance within the mesangium and can appear as ill-defined, subendothelial (intramembranous and/or subepithelial) inclusions.

### Other complement-mediated kidney diseases

In one aspect, the present invention provides the vector, cell or pharmaceutical composition according to the present invention for use in preventing or treating atypical hemolytic uremic syndrome (aHUS), typical hemolytic-uremic syndrome, lupus nephritis, cryoglobulinemia, anti-GBM disease, Antineutrophil cytoplasmic antibody (ANCA)-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III.

In a related aspect not forming part of the claimed invention, is provided the use of the vector, cell or pharmaceutical composition according to the present invention for the manufacture of a medicament for preventing or treating atypical hemolytic uremic syndrome (aHUS), typical hemolytic-uremic syndrome, lupus nephritis, cryoglobulinemia, anti-GBM disease, Antineutrophil cytoplasmic antibody (ANCA)-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III.

In a related aspect not forming part of the claimed invention, is provided a method of preventing or treating atypical hemolytic uremic syndrome (aHUS), typical hemolytic-uremic syndrome, lupus nephritis, cryoglobulinemia, anti-GBM disease, Antineutrophil cytoplasmic antibody (ANCA)-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III, the method comprising administering the vector, cell or pharmaceutical composition according to the present invention to a subject in need thereof.

Atypical hemolytic-uremic syndrome is a disease which causes abnormal blood clots (thrombi) to form in small blood vessels in the kidneys. These clots can cause serious medical problems if they restrict or block blood flow. Atypical hemolytic-uremic syndrome is characterized by three major features related to abnormal clotting: hemolytic anemia, thrombocytopenia, and kidney failure. aHUS is usually caused by chronic, uncontrolled activation of the complement system.

Typical hemolytic-uremic syndrome occurs in 5 to 15 percent of individuals, especially children, who are infected by the Escherichia coli. E. coli releases Stx toxins into the gut that are absorbed into the bloodstream and may be transported to the kidneys. This can result in acute renal injury, damage to the brain, the pancreas, and other organs. There is growing evidence for a role for activation of complement in typical hemolytic-uremic syndrome.

Lupus nephritis is an inflammation of the kidneys caused by systemic lupus erythematosus (SLE), an autoimmune disease. Complement activation mediates glomerular injury in lupus nephritis.

Cryoglobulinemia is a medical condition in which the blood contains large amounts of pathological cold sensitive antibodies called cryoglobulins. Cryoglobulins can deposit on the epithelium of blood vessels and activate the blood complement system to form pro-inflammatory elements such as C5a thereby initiating the systemic vascular inflammatory reaction termed cryoglobulinemic vasculitis.

Anti-glomerular basement membrane (GBM) disease, also known as Goodpasture's disease, is a rare condition that causes inflammation of the small blood vessels in the kidneys and lungs. GPS is caused by abnormal plasma cell production of anti-GBM antibodies. The anti-GBM antibodies attack the alveoli and glomeruli basement membranes. These antibodies bind their reactive epitopes to the basement membranes and activate the complement cascade, leading to the death of tagged cells.

Antineutrophil cytoplasmic antibody (ANCA)-associated vasculitis (AAV) is a group of diseases (granulomatosis with polyangiitis, eosinophilic granulomatosis with polyangiitis and microscopic polyangiitis), characterized by destruction and inflammation of small vessels. Activation of the complement system is crucial for the development of AAV, and that the complement activation product C5a has a central role.

Bacterial endocarditis is a bacterial infection of the inner layer of the heart or the heart valves. Patients with bacterial endocarditis can develop several forms of kidney disease including a bacterial infection-related immune complex-mediated glomerulonephritis.

Post-infectious glomerulonephritis (PIGN) is an immune complex-mediated glomerular injury that can occur as a consequence of an infection. Co-deposition of immunoglobulin (Ig) G and C3 is commonly observed in PIGN.

Antibody-mediated rejection is caused by binding of antibodies to human leukocyte antigens (HLA) expressed on endothelial cells of the transplanted organ. The antibodies activate the classical pathway of complement.

Membranous nephropathy (MN) describes a histopathologic pattern of injury marked by glomerular subepithelial immune deposits. There is much circumstantial evidence for a prominent role of complement in human MN because C3 and C5b-9 are found consistently within immune deposits.

Membranoproliferative glomerulonephritis (MPGN) is a type of glomerulonephritis caused by deposits in the kidney glomerular mesangium and basement membrane (GBM) thickening, activating complement and damaging the glomeruli.

There are three types of MPGN. Type I, the most common by far, is caused by immune complexes depositing in the kidney. It is characterised by subendothelial and mesangial immune deposits. It is believed to be associated with the classical complement pathway MPGN type II is now preferably known as dense deposit disease.

Type III is very rare, it is characterized by a mixture of subepithelial and subendothelial immune and/or complement deposits. These deposits elicit an immune response, causing damage to cells and structures within their vicinity.

### EXAMPLES

The invention will now be further described by way of Examples, which are meant to serve to assist one of ordinary skill in the art in carrying out the invention and are not intended in any way to limit the scope of the invention.

### Example 1 - AAV serotype 9 and LK03 transduction and expression in podocytes

### Tail vein injection of AAV serotype 9 demonstrates transduction of kidney cells and expression in the podocyte

At 8 weeks of age, mice were administered 1.5 x 10¹² vg via tail vein of either AAV2/9 hNPHS1.mpod or AAV2/9 mNPHS1.mpod, or saline. 6 weeks later, AAV ITRs were detected in the kidney cortex of mice injected with AAV (AAV 2/9 hNPHS1.mpod=39,067±13,285 copies ssDNA, AAV 2/9 mNPHS1mpod=76,533.33±32047 copies ssDNA, n=5-6/group) (**Figure 1C**). HA-tagged podocin was shown to co-localise with podocyte markers nephrin and podocin **(****Figure 1D****).**

### AAV2/9 expressing wild type podocin reduces albuminuria in iPod NPHS2^{fl/fl} mice

Vector treated groups showed a reduction in urinary albumin:creatinine ratio (ACR) (**Figure 2A****, 2B).** The effect of tail vein injection of AAV 2/9 expressing podocin on urinary ACR yielded an F ratio of F (2, 24) = 9.61, p<0.001 (n=9/group). At 14 days post-doxycycline, urinary ACR was higher in the saline group than either of the vector treated groups (AAV 2/9 hNPHS1.mpod = 758.1±488.1 mg/mmol, AAV 2/9 mNPHS1.mpod = 59.8±28.0 mg/mmol, saline = 3,770.1±1337.6 mg/mmol, AAV 2/9 hNPHS1.mpod vs saline p=0.40, AAV 2/9 mNPHS1.mpod vs saline p=0.25). There was a significant reduction in urinary ACR in the vector treated groups at day 28 (AAV 2/9 hNPHS1.mpod = 3,083.0±932.8 mg/mmol, AAV 2/9 mNPHS1.mpod = 2,195.1±778.9 mg/mmol, saline = 10,198±3,189.5 mg/mmol, AAV 2/9 hNPHS1.mpod vs saline p=0.008, AAV 2/9 mNPHS1.mpod vs saline p=0.002) and day 42 (AAV 2/9 hNPHS1.mpod = 3,266.8±1,212.2 mg/mmol, AAV 2/9 mNPHS1.mpod = 3,553.3±1,477.87mg/mmol, saline = 13,488.8±3,877.3 mg/mmol, AAV 2/9 hNPHS1.mpod vs saline p<0.001, AAV 2/9 mNPHS1.mpod vs saline p<0.001). In the vector treated groups, 2 of 9 mice in AAV 2/9 hNPHS1.mpod group and 1 of 9 mice in AAV 2/9 mNPHS1.mpod group had urinary ACRs of less than 30mg/mmol at day 42.

Although the mice in vector treated groups showed an improvement, there was a degree of variation within the groups which we hypothesised might be attributable to amount of vector that reached the kidney after a systemic injection. The amount of viral DNA detected in kidney cortex showed an inverse correlation with the degree of albuminuria at day 42 (Spearman r= -0.4596, p=0.0477) (**Figure 2D**).

### AAV2/9 expressing wild type podocin partially rescues the phenotype in iPod NPHS2^{fl/fl} mice

Vector treated mice showed a reduction in creatinine (saline = 39.0±8.5 µmol/L, AAV 2/9 hNPHS1.mpod = 27.3±7.9 µmol/L, AAV 2/9 mNPHS1.mpod = 18.6±4.4 mmol/L, p=0.1622), a reduction in urea (saline = 39.4±17.6 mmol/L, AAV 2/9 hNPHS1.mpod = 12.0±2.0 mmol/L, AAV 2/9 mNPHS1.mpod = 11.6±1.6 mmol/L, p=0.058), an increase in albumin (saline = 10.5±5.4 g/L, AAV 2/9 hNPHS1.mpod 17.1=4.8 ± g/L, AAV 2/9 mNPHS1.mpod = 17.1±3.6 g/L, p=0.5602) and a significant reduction in cholesterol (saline = 15.76±1.75 mmol/L, AAV 2/9 hNPHS1.mpod = 2.64±0.60 mmol/L, AAV 2/9 mNPHS2.mpod = 4.86±0.76 mmol/L, p=0009) (**Figure 2E**).

Saline treated mice showed histological features of focal segmental glomerulosclerosis (FSGS) by 6 weeks. Vector treated mice did not show histological features of FSGS on light microscopy, but demonstrated a range of histological findings from completely normal glomeruli to pseudo-crescents or mesangial hypercellularity. (**Figure 2F**).

These mice also showed prolonged survival (n=3-4/group), with a median survival of 75.5 days (range 38 to 111 days) in the saline group, compared to median survival of 192 days (range 74 to still alive at 206 days) in AAV 2/9 hNPHS1.mpod and median survival of 192 days (range 131 to still alive at 206 days) in AAV 2/9 mNPHS1.mpod (p=0.049) (**Figure 2C**).

Untreated mice show loss of expression of podocin with a change in pattern of expression of nephrin to a diffuse pattern (**Figure 2G**). This is a stark contrast to the predominantly membranous pattern of expression of nephrin and podocin seen in vector treated mice (**Figure 1D**).

### AAV LK03 transduces human podocytes

AAV LK03 with CMV GFP and AAV LK03 hNPHS1 GFP were used to transduce human podocytes, glomerular endothelial cells and proximal tubular epithelial cells at a MOI of 5x105. Flow cytometry (n=3) showed that AAV LK03 CMV GFP had highly efficient transduction of the podocyte (% GFP expression=98.83±0.84), AAV LK03 hNPHS1 GFP had good transduction (% GFP expression=71.3±3.39) and untransduced cells had unremarkable expression (% GFP expression=0.89±0.36) (**Figure 3D**). This is reflected on immunofluorescence (**Figure 3A****,** **3C****,** **3E**) and western blot (**Figure 3B**). Although the proportion of cells positive for GFP expression is high in podocytes transduced with AAV LK03 hNPHS1 GFP, the cells have lower fluorescence intensity than those transduced with AAV LK03 CMV GFP (**Figure 3F**).

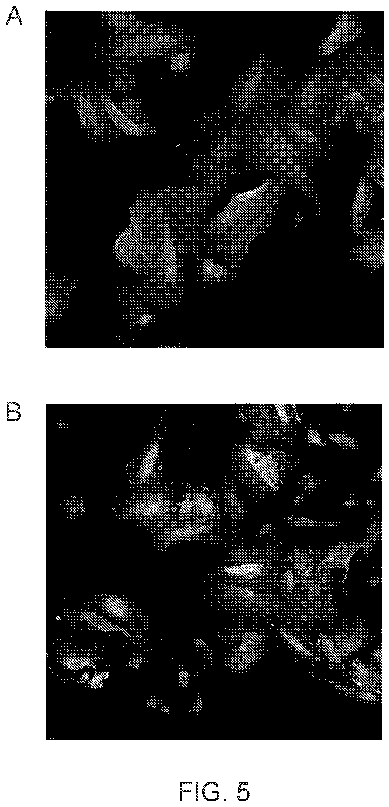

Interestingly, AAV LK03 CMV GFP showed much lower transduction in glomerular endothelial cells (% GFP expression=7.35±0.19). AAV LK03 hNPHS1 GFP showed minimal transduction in glomerular endothelial cells (% GFP expression=0.59±0.10), on a similar level to untransduced glomerular endothelial cells (% GFP expression=0.23±0.02). As AAV 2/9 has been the serotype which has seen the best transduction in kidney cells in vivo in rodent kidneys, we tested the expression of AAV 2/9 CMV GFP on human kidney cell lines. AAV 2/9 CMV GFP showed low transduction efficiency in both podocytes (% GFP expression= 13.9±1.98) and glomerular endothelial cells (% GFP expression=21.99±4.35) (**Figure 3D**). AAV LK03 with AAV LK03 hNPHS1 HAVDR and AAV LK03 hNPHS1 hSmad7 were used to transduce human podocytes showing good expression of both proteins (**Figure 5**)

### AAV LK03 expressing human podocin under the minimal nephrin promoter shows functional rescue in mutant podocin R138Q podocyte cell line.

The R138Q podocin mutant results in mislocalisation of podocin from the plasma membrane to the endoplasmic reticulum. The mutant podocin R138Q podocyte cell line was acquired from a patient kidney and conditionally immortalised using temperature sensitive SV40 T antigen. AAV LK03 hNPHS1 hpod transduces R138Q podocytes and expresses HA-tagged podocin (**Figure 4A, 4B**). HA-tagged podocin is seen at the plasma membrane on confocal microscopy and colocalises with Caveolin-1, a lipid raft protein, as seen on TIRF microscopy (**Figure 4B****,** **4E**). Untransduced R138Q podocytes do not show any podocin expression at the plasma membrane (**Figure 4B**). HA-tagged podocin does not colocalise with Calnexin, an endoplasmic reticulum marker (**Figure 4D**).

Podocytes show a decrease or increase in adhesion in diseased states. Our previous work has shown that the R138Q mutation causes a decrease in podocyte adhesion. AAV transduction causes a decrease in podocyte adhesion but the R138Q podocytes still show reduced adhesion compared to wild type podocytes, and transduction with AAV LK03 hNPHS1 hpod results in the rescue of the adhesional function of R138Q podocytes (**Figure 4C**).

### Adding WPRE to a construct using the minimal human nephrin promoter increases gene expression in human podocytes in vitro

WPRE has been previously shown to increase gene expression in certain circumstances, but there have also been concerns that it might potentially contribute to the pathogenesis of hepatocellular carcinoma so potentially limiting its use for gene therapy applications. Our WPRE sequence has mutations within the X-antigen promoter, and the initiation codon of the X-antigen has also been mutated, which prevents the production of a functional X-antigen. ssAAV LK03 hNPHS1.GFP.WPRE.bGH was compared to ssAAV.LK03 hNPHS1.GFP.bGH. Flow cytometry showed an increase in % GFP expression where the construct with WPRE showed % GFP expression of 71.30±3.39 versus 45.93±4.34 (n=3, p<0.0001) in the construct without WPRE (**Figure 4F**).

### Discussion

Here we have successfully targeted the podocyte with AAV 2/9 using a minimal nephrin promoter to express mouse podocin in a conditional mouse knock-out model, with partial rescue of the phenotype and improvements in albuminuria seen in vector treated mice. As a first proof of principle study, we have chosen to inject the vector prior to doxycycline induction, so that effective rescue by the vector is in place when podocin is knocked out. The effect of doxycycline induction is rapid, and the progression to severe nephrosis (8-14 days) and FSGS is relative quick (about 6 weeks). We have shown here that in vitro, introducing wild type human podocin to R138Q podocytes enables expression of podocin that reaches the plasma membrane, and rescues podocyte adhesion.

AAV LK03 has shown high transduction of close to 100% in human podocytes in vitro, which is reduced to 72.3% when using the minimal human nephrin promoter. We have shown that we can use this serotype to transduce podocytes specifically in vitro, and that expression of wild type podocin in R138Q mutant podocytes show functional rescue. Using AAV LK03 has implications on translation as such effective transduction of human podocytes might enable a significant reduction in effective dose in humans. A recent UK study has shown low anti AAV LK03 neutralising antibody seroprevalence of 23%, with a nadir in late childhood (Perocheau, D. P. et al., 2019. Human gene therapy, 30(1), pp.79-87), which makes this particular serotype a promising candidate for translational studies.

By testing ssAAV LK03 hNPHS1.GFP.WPRE.bGH against ssAAV LK03 hNPHS1.GFP.bGH in the human podocyte, GFP expression was improved in podocytes when using the WPRE containing construct. It was important that this was tested in this particular context as the effect of WPRE on improving gene expression is promoter and cell line specific.

We describe a proof of principle study that demonstrates AAV transduction of podocytes with a podocyte-specific promoter ameliorates albuminuria in the iPod NPHS2^{fl/fl} mouse model. We also show that a synthetic capsid, AAV LK03, shows highly efficient transduction of human podocytes. In combination, this work is a first step towards translation of AAV gene therapy targeting monogenic disease of the podocyte.

### Materials and methods

### Vector production

We prepared pAV.hNPHS1.mpodHA.WPRE.bGH, pAV.mNPHS1.mpodHA.WPRE.bGH and pAV.hNPHS1.hpodHA.WPRE.bGH (**Figure 1A**) pAV.mNPHS1.hHAVDR.WPRE.bGH and pAV.mNPHS1.hHASmad7.WPRE.bGH in our laboratory from a CMV eGFP L22Y pUC-AV2 construct using human and mouse podocin cDNA (Origene, Herford, Germany) and human VDR and Smad7 cDNA. Human embryonic kidney 293T cells were transfected with a capsid plasmid (pAAV9 from Penn Vector Core), a helper plasmid with adenoviral genes and the transgene plasmid using polyethyleneimine. Cells and supernatant were harvested at 72 hours post-transfection. Cells underwent 5 freeze-thaw cycles, while the supernatant underwent PEG precipitation (8% PEG 0.5N NaCl). These were combined and incubated with 0.25% sodium deoxycholic acid and 70units/ml Benzonase for 30 minutes at 37°C. The vector was purified by iodixanol gradient ultracentrifugation, and subsequently concentrated in PBS. Vectors were titrated by qPCR using the standard curve method using the following primers:
ITR F GGAACCCCTAGTGATGGAGTT,
ITR R CGGCCTCAGTGAGCGA,
ITR probe FAM-5'-CACTCCCTCTCTGCGCGCTCG-3'-TAMRA.

### Animals

All animal experiments and procedures were approved by the UK Home Office in accordance with the Animals (Scientific Procedures) Act 1986, and the Guide for the Care and Use of Laboratory Animals was followed during experiments. NPHS2^{flox/flox} mice were bred with NPHS2-rtTA/ Tet-On Cre mice to generate offspring with NPHS2-rtTA/ Tet-On Cre/ NPHS2^{flox/flox}. These mice develop a podocyte-specific knockout of podocin when exposed to doxycycline. These will be called iPod NPHS2^{fl/fl} from hereon. Mice were on a mixed background and equal numbers of each sex were used. Mice were administered AAV via tail vein injection at 8 weeks of age. (**Figure 1B**) 10 to 14 days later, mice were provided with drinking water supplemented with doxycycline 2mg/ml and 5% sucrose for 3 weeks. Urine was taken weekly. Mice were culled by Schedule 1 methods at 6 weeks post initiation of doxycycline. A small number of mice were kept beyond 6 weeks to test for effect on survival. All mice were re-genotyped from tissue taken at death.

### Cell Culture

Conditionally immortalised human podocytes (Pod) were cultured in RPMI with L-glutamine and NaHCO₃ with 10% Fetal Bovine Serum (Sigma Aldrich, Gillingham, UK). Conditionally immortalised human glomerular endothelial cells (GEnC) were cultured in EBMTM-2 Endothelial Cell Growth Basal Medium-2 supplemented with EGMTM-2 Endothelial Cell Growth Medium-2 BulletKit^{™} (Lonza, Basel, Switzerland). Immortalised proximal tubule epithelial cells (ATCC, Teddington, UK) (PTEC) were cultured in DMEM/F12 supplemented with Insulin, Transferrin and Selenium, Hydrocortisone and 10% FBS.

Cells were transduced with AAV at a MOI of 5 x 10⁵. For GFP expression, cells were used at 5-7 days post transduction to allow comparisons across different cell lines. For podocin, VDR and Smad7 expression, cells were used at 10-14 days post transduction when podocytes are maximally differentiated.

### Quantitative PCR

DNA was extracted using DNeasy Blood and Tissue Kit (Qiagen, Manchester, UK) from mouse kidney cortex. AAV DNA was detected using the primers above for viral titration and normalised against mouse beta-actin.

RNA was extracted using RNeasy Mini Kit with RNase-Free DNase set (Qiagen, Manchester, UK).

### Immunofluorescence

5µm sections were fixed using 4% PFA and blocked with 3% BSA 0.3% Triton X- 100 and 5% of either goat or donkey serum. Primary antibodies were anti-HA High Affinity from rat IgG1 (Roche, Basel, Switzerland), Guinea Pig anti-Nephrin (1243-1256) Antibody (Origene, Herford, Germany), and Rabbit anti-NPHS2 Antibody (Proteintech, Manchester, UK).

Cells were fixed with either 4% PFA and or ice cold methanol, incubated for 5 minutes with 0.03M glycine, permeabilised with 0.3% Triton then blocked with 3% BSA. Primary antibodies were mouse HA.11 Epitope Tag Antibody (Biolegend, San Diego, USA), mouse anti-GFP (Roche, Basel, Switzerland), rabbit anti-Calnexin (Merck Millipore, Darmstadt, Germany) and rabbit anti-Caveolin 1 (Cell Signaling, Danvers, USA).

Secondary antibodies were AlexaFluor 488 donkey anti-mouse, AlexaFluor 488 donkey anti-rabbit, AlexaFluor 488 goat-anti guinea pig, AlexaFluor 555 goat anti-rabbit and AlexaFluor 633 goat anti-rat, and AlexaFluor 633 Phalloidin (Invitrogen, Thermo Fisher Scientific, Waltham, USA). Sections were counterstained with DAPI and mounted with Mowiol. Images were taken on a Leica SPE single channel confocal laser scanning microscope attached to a Leica DMi8 inverted epifluorescence microscope, or Leica SP5-II confocal laser scanning microscope attached to a Leica DMI 6000 inverted epifluorescence microscope, or Leica AM TIRF MC (multi-colour) system attached to a Leica DMI 6000 inverted epifluorescence microscope using LAS (Leica Application Suite) X Software.

### Western Blotting

Cells were extracted in SDS lysis buffer. Samples were run on a 12.5% gel and transferred to PVDF membrane. Membranes were blocked in 5% milk in TBST 0.1%. Primary antibodies used were mouse HA.11 Epitope Tag Antibody (Biolegend, San Diego, USA), mouse anti-GFP (Roche, Basel, Switzerland) in 3% BSA in TBST 0.1%, or rabbit anti-NPHS2 antibody (Proteintech, Manchester, UK). Secondary antibodies were anti-rabbit or anti-mouse IgG Peroxidase (Sigma Aldrich, Gillingham, UK) in 3% BSA in TBST 0.1%. Membranes were imaged on Amersham Imager 600.

### Flow Cytometry

Live cells were stained with propidium iodide and only live single cells were included in the analysis. Flow cytometry was carried out on the NovoCyte Flow Cytometer.

### Adhesion assay

Cells were trypsinised and resuspended at 105/ml and allowed to recover for 10 30 minutes before plating 50µl of cells diluted 1 in 2 with PBS in a 96 well plate. Technical triplicates were used. Cells were left to adhere for about 1 hour at 37°C. Cells were washed with PBS to wash away non adherent cells, then fixed with 4% PFA for 20 minutes. Cells were washed with distilled water then stained with 0.1% crystal violet in 2% ethanol for 60 minutes at room temperature. Cells were washed and incubated with 10% acetic acid on a shaker for 5 minutes. Absorbance was measured at 570nm and results were normalised against the wild type cell line transduced with AAV LK03 CMV GFP.

### Urine

Albumin levels were measured using a mouse albumin 5 ELISA kit (Bethyl Laboratories Inc, Montgomery, USA) and Creatinine levels were measured on the Konelab Prime 60i Analyzer.

### Blood tests

Mouse plasma was processed either using the Konelab Prime 60i analyser or the Roche Cobas system with reagents and protocols supplied by the manufacturer.

### Statistical analysis

All data is presented as mean±SEM unless stated otherwise. Statistical analyses were performed in GraphPad Prism (Graphpad softward, La Jolla, USA). Statistical tests used include two-tailed t-test, one-way ANOVA with Tukey's multiple comparison posthoc analysis, two-way ANOVA with Tukey's multiple comparison posthoc analysis, and Logrank (Mantel-Cox) test for survival analysis.

### Example 2 - Podocyte production and secretion of complement proteins

### Human podocytes express and secrete complement factors C3 and CFH in vitro

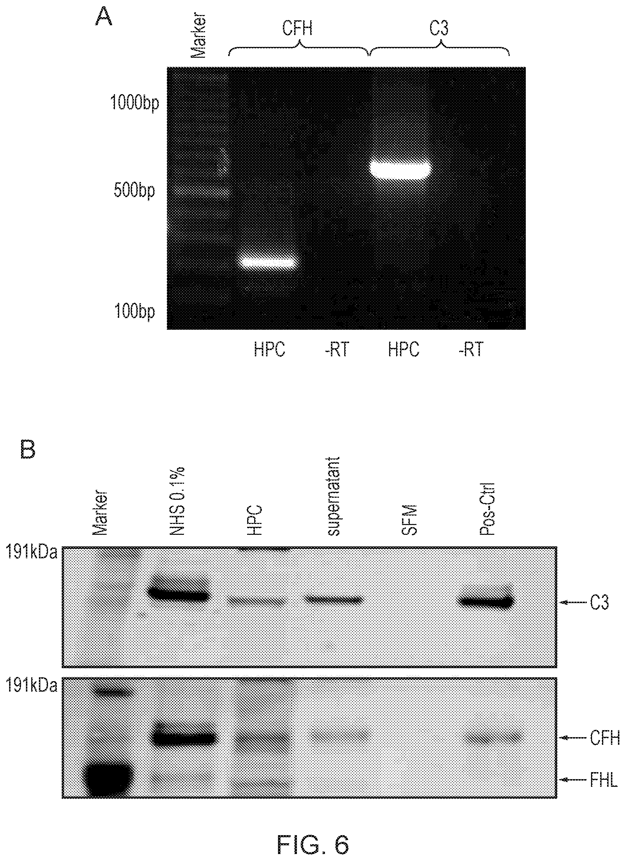

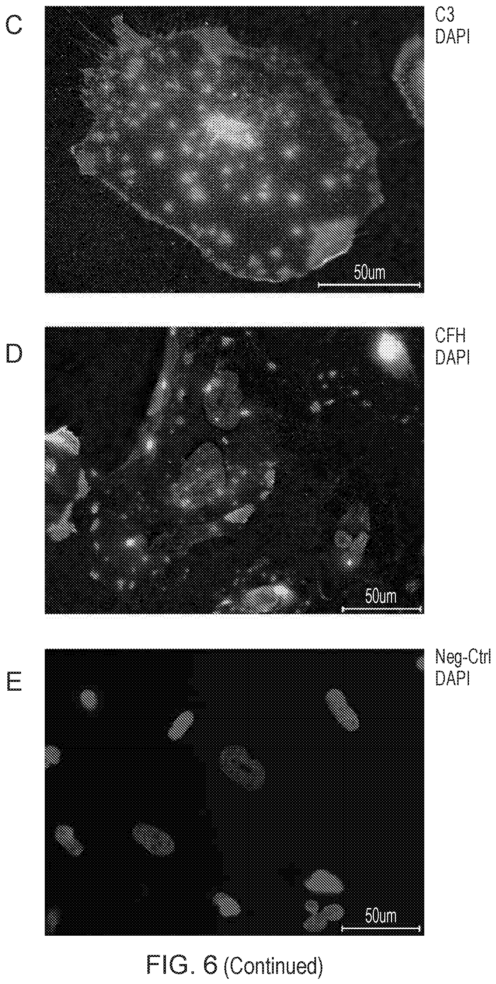

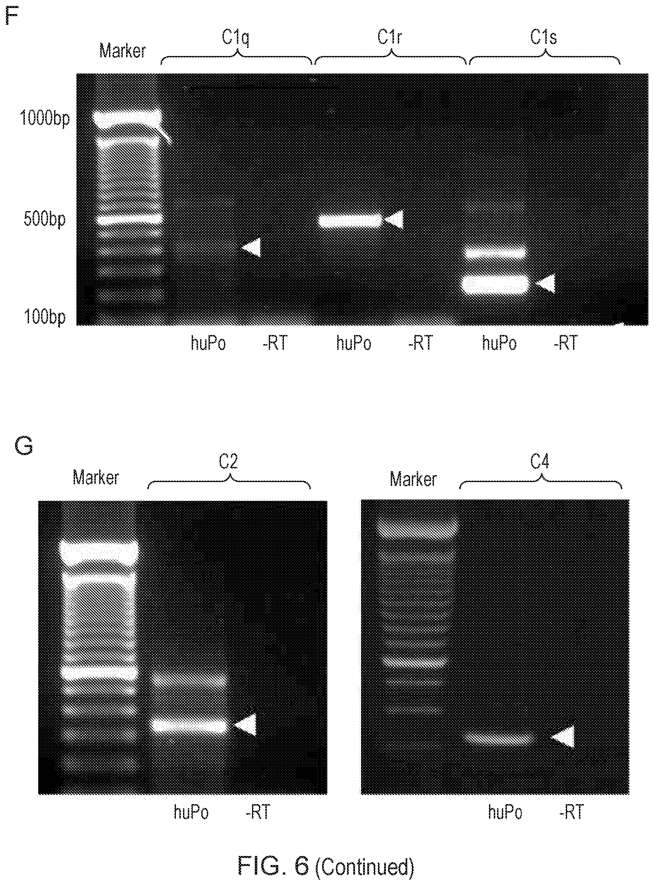

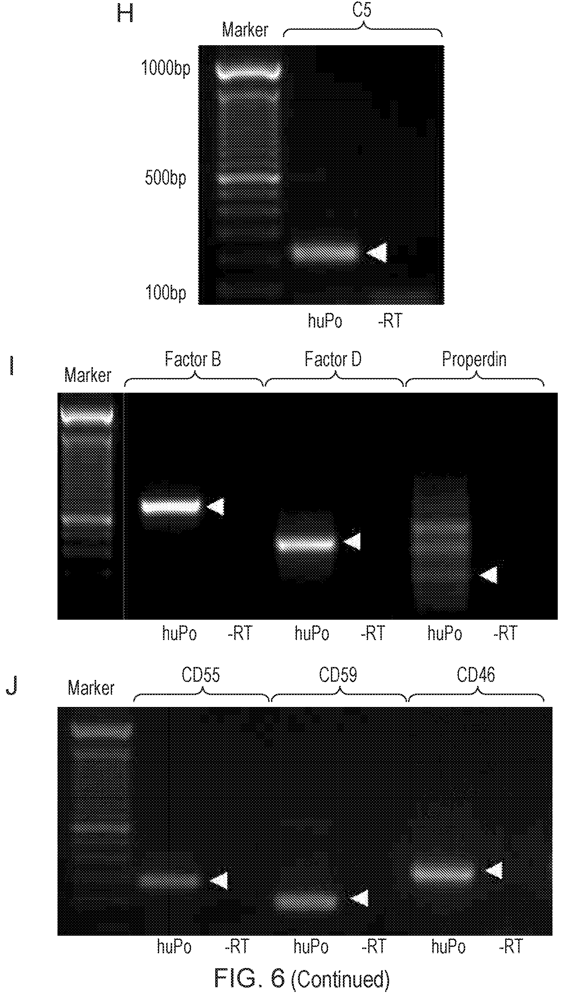

To evaluate the potential of podocytes to build complement components we analyzed conditionally immortalized human podocytes. Conventional reverse transcriptase PCR identified mRNA for the activating key component C3 (PCR product 783 bp) and also for CFH (320 bp) (**Figure 6A**)**.**

RNA was also identified for the early activating complement proteins C1q, C1r, C1s, C2, C4, C5; the alternative pathway activators factor B, factor D, properdin and the regulators CD55, CD59 and CD46 (MCP) (**Figure 6F to 6J**).

As CFH is one of the most important soluble inhibitors of the alternative pathway, and C3 is a key component in early complement activation, we focused on these two proteins. After 24 hours of incubation in SFM, C3 and CFH were detected with Western blots of the whole cell lysates and in the cell culture-supernatant. Podocytes showed the expression of two products of the CFH-gene: CFH and factor H-like protein 1 (FHL-1) (**Figure 6B**). Complement proteins for C2, C5 and the regulatory components CFH, CD46, CD55 and CD59 were also detected. The expression of C3 and CFH could also be determined in immunofluorescence (**Figure 6C** and **6D**). Both proteins were detected on the surface of non-permeabilized podocytes.

### Production and secretion of podocyte complement components is an active process

Secreted CFH circulates throughout the body and can bind to most cells by binding to the cellular glycocalyx. This regulates uncontrolled complement activation directly on the cell surface. CFH glycocalyx binding sites can be degraded temporarily by treatment with low dose trypsin. To see whether the podocytes are capable of replacing removed CFH from the surface we treated differentiated podocytes with low dose trypsin to remove surface-bound CFH. Cells were then allowed to recover in SFM. CFH was detected again on the surface of the podocytes 24 hours later, showing that these cells can produce and replace CFH (**Figure 7A**).

IFNg has previously been shown to increase cellular synthesis of complement proteins in a variety of cell lines. Therefore, human podocytes were treated with IFNg at different time points and concentrations. Stimulation with IFNg significantly enhanced human podocyte mRNA expression of C3 and CFH (**Figure 7B**). It also increased the expression of both proteins in whole cell lysates. Furthermore, secreted C3 and CFH were increased after stimulation with IFNg in a time- and dose-dependent manner (Figure 7C**-E**). This suggests that podocytes are capable of producing complement proteins as part of a pro-inflammatory response.

### Expression of complement factor C3 and CFH varies in cultured human podocytes and glomerular endothelial cells

Podocytes are always affected in proteinuric glomerulopathies. Nevertheless, within the glomerulus there are other cell types, which can contribute to the local complement production. Glomerular endothelial cells have direct contact with serum-based complement activation and complement products, and we have previously shown that podocyte-derived VEGF regulated expression of protective complement regulators on glomerular endothelial cells. To determine the cell specific production of complement proteins, we compared the expression of CFH and C3 in conditionally immortalized human glomerular endothelial cells (CiGenC) (Satchell et al., 2006. Kidney Int 69(9), 1633-1640) and podocytes (Saleem et al., 2002. J Am Soc Nephrol 13(3)). Cultivated podocytes produced significantly more C3 mRNA, but less CFH mRNA compared to endothelial cells (**Figure 8A** and **B**). At the protein level for C3 and CFH, there was a slightly higher expression of C3 in podocytes (**Figure 8C** and **D**). The expression of CFH was significantly lower in podocytes according to protein quantification (**Figure 8C** and **E**).

Complement activation may happen on any glomerular cell. Hence, the secretion of produced complement products is important. From the results in mRNA production, a comparison of secretion of complement proteins C3 and CFH showed a significant higher secretion of C3 (**Figure 8F** and **G**) and a lower secretion of CFH (**Figure 8F** and **H**) in podocytes compared to endothelial cells. Therefore, we could show that complement production and secretion profiles may differ in different intraglomerular cell types.

### Podocyte-derived CFH is efficient in complement control

Complement activation on normal human podocytes and podocytes isolated from a patient with diagnosed aHUS was compared. aHUS is a rare disorder of complement regulation, which results in kidney impairment, thrombocytopenia and anemia. A mutation in regulatory complement genes is found in many patients with this disease. We used podocytes from a patient with a known Arg1182Ser (G3546T) CFH mutation (as described in Mühlig, A.K., et al., 2020. Frontiers in Immunology, 11, p.1833). This mutation prevents CFH from binding to the cell surface and partially to C3b (Kajander et al., 2011. PNAS 108(7), 2897-2902). This is why cells from this patient cannot regulate complement activation on the surface. In a complement challenge assay, there was significantly more C5b-9 on the surface of cells containing the CFH mutation, compared to not diseased podocytes. This suggests that the secreted podocyte CFH contributed to complement regulation in the normal podocyte cell line but the mutated CFH from the aHUS cell line reduced the cells' ability to regulate complement (**Figure 9A and B**).

### Example 3 - The role of podocytes in complement-mediated kidney disease

### Generation of a mouse model with podocytes expressing Gb3

Chronic, uncontrolled, and excessive activation of the complement system has been implicated in atypical HUS (Noris, M. and Remuzzi, G., 2009. NEJM, 361(17), pp.1676-1687). Shiga toxin-producing E. coli HUS (STEC-HUS) occurs after ingestion of a strain of bacteria expressing Shiga toxin such as enterohemorrhagic Escherichia coli (EHEC). Shiga toxin acts via the podocyte Gb3 receptor to reduce local VEGF-A secretion (Keir, L.S. and Saleem, M.A., 2014. Pediatric Nephrology, 29(10), pp.1895-1902). Loss of podocyte VEGF-A increases glomerular endothelial cell susceptibility to complement attack resulting in haemolytic uremic syndrome (Eremina, V., et al., 2008. New England Journal of Medicine, 358(11), pp.1129-1136; Keir, L.S., et al., 2017. The Journal of clinical investigation, 127(1), pp.199-214).

Human podocytes express Gb3, whilst mouse podocytes do not. We have generated a mouse model with podocytes expressing Gb3 on Gb3 null background, using tetracycline-controlled transcription activation of Gb3 synthase. This mouse model can be used to investigate the role of podocytes in STEC-HUS.

### Gb3 KO mice do not develop HUS phenotype when injected with Shiga Toxin

We injected WT mice and Gb3 synthase KO mice with 10 ng/g of Shiga toxin (N=4 for each genotype). Results are shown in **Figure 10****.** All WT mice died by day 5 from injection of Shiga toxin. In contrast, all Gb3 synthase KO mice survived until the end of the monitoring period (day 15) (**Figure 10A**). WT mice showed clear evidence of acute tubular necrosis with oedematous tubules and vacuolations. In contrast, Gb3 synthase KO mice showed no changes in the tubular or glomerular morphology (**Figure 10B**).

We injected Gb3 synthase KO mice with 10-fold Shiga toxin (100 ng/g) (N=2). Results are shown in **Figure 11****.** Gb3 synthase KO mice survived until the end of the monitoring period (day 9) and showed no changes in the tubular or glomerular morphology.

### Podocyte Gb3 expressing mice on Gb3 null background develop HUS phenotype when injected with Shiga Toxin

Gb3 synthase KO mice were crossed with podocyte Gb3 expressing mice (Pod rtTA TetOGb3 synthase mice) to produce podocyte Gb3 expressing mice on Gb3 null background (Pod rtTA TetOGb3 Gb3^{null} mice). These mice were then injected with Shiga toxin (Stx) (**Figure 12**).

Podocyte Gb3 expressing mice on Gb3 null background develop HUS phenotype when given IP Shiga toxin (10 ng/g). They had significantly lower platelet counts (unpaired T test, p=0.004) and haemoglobin levels (unpaired T test p=0.041) as measured day 10 post Stx injection than the control mice (Pod rtTA TetOWT Gb3^{null} mice) (**Figures 13A and 13B**) and had significantly higher plasma urea concentration (unpaired T test p=0.0052) in samples pooled from days 12-16 post Stx injection (**Figure 13C**). Analysis of blood films showed evidence of HUS phenotype (**Figure 13D**). Fibrinogen levels were significantly higher (N=3, 30 glomeruli per mouse, unpaired T test p<0.001) in the podocyte Gb3 expressing mice on Gb3 null background (**Figure 13E**).

### Complement inhibitor rescue of the HUS phenotype

C3b levels were more than 2-fold higher (N=3, 30 glomeruli per mouse, unpaired T test p<0.0001) in the podocyte Gb3 expressing mice on Gb3 null background (**Figure 14A** **and** **Figure 14B**). C3b is an important component of the complement system. C3b is potent in opsonisation and plays a role in forming C3 convertase and C5 convertase.

To assess the role of complement in the HUS phenotype in the mice, BB5.1 (a C5 inhibitor) was injected into the mice following the development of HUS symptoms (**Figure 12**).

Pod rtTA TetOGb3 Gb3^{null} mice were injected with Shiga toxin on day 0, as before. On day 7, the mice were injected with saline, or BB5.1 (C5 inhibitor). Mice injected with the C5 inhibitor had significantly higher platelet count than the mice injected with saline (unpaired T test p<0.0005) (**Figure 14C**). Mice injected with the C5 inhibitor had significantly higher haemoglobin than the mice injected with saline (unpaired T test p<0.005) (**Figure 14D**).

These results show that podocytes may play an important role in complement-mediated kidney diseases. Targeting podocytes with complement inhibitors may provide an effective treatment for complement-mediated kidney diseases.

### Example 4 - Podocyte-targeted AAV encoding a complement inhibitor

### Vector production

**Figure 15** shows exemplary AAV constructs which are capable of transducing podocytes and inducing expression and secretion of complement inhibitors from the podocytes. The AAV constructs may be packaged with AAV3B, LK03, or AAV9 serotypes to effectively transduce podocytes.

AAV constructs pAAV.NPHS1.CFI.WPRE.bGH, pAAV.NPHS1.CFH.WPRE.bGH, pAAV.265.CFH.WPRE.bGH and pAAV.NPHS1.FHL-1.WPRE.bGH may be prepared using suitable CFI, CFH, and FHL-1 cDNA. Human embryonic kidney 293T cells may be transfected with a capsid plasmid, a helper plasmid with adenoviral genes and the transgene plasmid using polyethyleneimine. Cells and supernatant may be harvested at 72 hours post-transfection. Cells may undergo 5 freeze-thaw cycles, while the supernatant may undergo PEG precipitation (8% PEG 0.5N NaCl). These may be combined and incubated with 0.25% sodium deoxycholic acid and 70units/ml Benzonase for 30 minutes at 37°C. The vector may be purified by iodixanol gradient ultracentrifugation, and subsequently concentrated in PBS. Vectors may be titrated by qPCR using the standard curve method using suitable primers.

### Cell culture

Immortalised human podocytes may be transduced with the packaged AAV constructs and expression of the complement inhibitor and complement activity may be measured.

Conditionally immortalised human podocytes (Pod) may be cultured in RPMI with L-glutamine and NaHCO₃ with 10% Fetal Bovine Serum (Sigma Aldrich, Gillingham, UK). Cells may be transduced with AAV at a MOI of 5 x 10⁵. Cells may be used at 10-14 days post transduction when podocytes are maximally differentiated.

### Mouse models

The packaged AAV constructs may be administered to suitable mouse models (e.g. Pod rtTA TetOGb3 Gb3^{null} mice following Shiga Toxin injection) via tail vein injection. Expression of the complement inhibitor, complement activity and rescue of complement-mediated disease may be measured.

### Example 5 - Vector production

Plasmids were prepared encoding Complement Factor H (CFH), Complement Factor I (CFI) or Complement Factor H Like-1 (FHL-1), under control of a 265bp minimal nephrin promoter variant: pAAV.MCS.NPHS1(265).CFH.WPRE.bGH, pAAV.MCS.NPHS1(FL).CFI.WPRE.bGH and pAAV.MCS.NPHS1(FL).CFHL1.WPRE.bGH (Figures 16A**-C**).

Complement Factor H, Complement Factor I and Complement Factor H Like-1 were PCR amplified from Origene plasmids and cloned into the pAAV.MCS.NPHS1(FL)/NPHS1(265) backbone. A PCR-based molecular cloning approach was used. The primers used are described below.

| **Target** | **Primer** | **Sequence** | **Expected Amplicon** |
|---|---|---|---|
| Agel-CFH-MYC-FLAG-Sbfl | Forward | TAATAAaccggtcgccaCCATGAGACTTCTAGCAA AG (SEQ ID NO: 30) | 3822bp |
| | Reverse | TAATAAcctgcaggTTAAACCTTATCGTCGTCATC C (SEQ ID NO: 31) | |
| Agel-CFI-MYC-FLAG-Sbfl | Forward | TAATAAaccggtcgccaCCATGAAGCTTCTTCATG TT (SEQ ID NO: 32) | 1878bp |
| | Reverse | TAATAAcctgcaggTTAAACCTTATCGTCGTCATC C (SEQ ID NO: 33) | |
| Agel-CFHL1-MYC-FLAG-Sbfl | Forward | TAATAAaccggtcgccaCCATGTGGCTCCTGGTC AGTGTAATT (SEQ ID NO: 34) | 1119bp |
| | Reverse | TAATAAcctgcaggTTAAACCTTATCGTCGTCATC C (SEQ ID NO: 35) | |

Each PCR product was then ligated into the pAAV.MCS.NPHS1(FL)/NPHS1(265) backbone at a ratio of 1:3 vector:insert and transformed into *E. coli* stable competent cells. Colonies were grown and digested with Smal to confirm the presence of insert/ITRs. Each plasmid was sent for sequencing.

AAV purification was performed using iodixanol gradient ultracentrifugation. Alkaline gel electrophoresis demonstrated that intact virus was identified following ultracentrifugation **(****Figure 16D****).**

### Example 6 - Expression of CFH, CFI and CFHL1 in HEK cells

### Materials and methods

60-80% confluent 293T Human Embryonic Kidney cells grown in DMEM supplemented with 10% FBS were triple transfected with pHelper (HGTI1), one of the two pAAV Rep-Cap (LK03 or 2/9) and one of the three ITR-expression plasmids containing 1) CFH under the 265bp mini nephrin promoter (pAAV-265-CFH), 2) CFI under the 1249 bp full-length minimal nephrin promoter (pAAV-FL-CFI) or 3) CFHL1 under the 1249bp full-length minimal nephrin promoter (pAAV-FL-CFHL1). All constructs were tagged with MYC and FLAG.

Transfection was carried out on a 150mm culture dish in serum-free media in the presence of polyethylenimine (PEI). Media was changed to DMEM with FBS the following day after transfection. On Day 4 post-transfection, media and cells were collected and processed separately. Media was frozen and stored at -80°C. Cells were lysed with RIPA buffer supplemented with proteinase inhibitors and stored at -80°C.

Protein concentration in the cell lysates was measured using Pierce BCA protein assay and 10ug of total protein from each sample was loaded onto a 4-15% polyacrylamide Tris-Glycine gel. A total of 2.6ul of media from each sample was loaded onto the gel. Protein was transferred to a nitrocellulose membrane using the iBlot2 dry blotting system. The following primary antibodies were used for protein detection: anti-Factor H (Abcam, cat. ab124769), anti-Factor I (Abcam, ab278524), anti-MYC-tag (CST, cat.2276S), anti-FLAG-tag (CST, cat. 14793S) and anti-GAPDH (Millipore, cat. MAB374).

### Results

Factor H was expressed in both the cell lysates and the media from 293T HEK cells transfected with the CFH expression plasmid (**Figure 17**). Expression was detected using a Factor H-specific antibody. Factor H was not detected in the cell lysates or media of untransfected cells or cells transfected with the CFI or CFHL1 expression plasmids.

Factor I was expressed in both the cell lysates and the media from 293T HEK cells transfected with the CFI expression plasmid (**Figure 17**). Expression was detected using a Factor I-specific antibody which was able to detect Factor I in the cell lysates and the media. Anti-MYC- and anti-FLAG-tag antibodies detected uncleaved Factor I (~88kDa) in the cell lysates, but not in the media. Lack of MYC-tag and FLAG-tag detection in the media is believed to be due to Factor I undergoing post-translation processing where it is cleaved and secreted from the cell without the MYC or FLAG tag. In support of this, we detected a cleaved form of Factor I with Factor I-specific antibody in the media at the expected size (~50kDa). We could not detect the cleaved form in the media of untransfected cells or cells transfected with CFH or CFHL1 expression plasmids, indicating a specific staining.

Factor H-like 1 was expressed in the media from 293T HEK cells transfected with the CFHL1 expression plasmid, but was not detected in the cell lysates (**Figure 17**). Expression was detected using anti-MYC and anti-FLAG antibodies. Factor H-like 1 was not detected in the cell lysates or cell media of untransfected cells or cells transfected with the CFI or CFH expression plasmids. Two bands were detected due to different glycosylated forms of the protein being recognised.

In conclusion, transfection of 293T HEK cells with pHelper (HGTI1), a pAAV Rep-Cap plasmid (LK03 or 2/9) and one of three ITR-expression plasmids containing CFH, CFI or CFHL1 leads to expression of each of these transgenes.

### Example 7 - Transduction of Factor H mutated podocytes with AAV2/9 265-CFH or plasmid encoding CFH

### Transduction of Factor H mutated podocytes with AAV2/9 265-CFH

Conditionally immortalised human podocytes with a mutation in endogenous Factor H (Muehlig et al, 2020) grown in RPMI supplemented with 10% FBS and 1% ITS were seeded in 6-well culture plates and grown at 33°C until 70-80% confluency. Cells were incubated with AAV2/9 containing a CFH transgene under the control of the 265bp minimal nephrin promoter (SEQ ID NO: 27). Cells incubated without the virus were used as a non-transduced (NT) control. On the same day following viral transduction, cells were transferred to a non-permissive temperature of 37°C to allow for cell differentiation and transgene expression. Media was changed twice on the subsequent days following transduction. On Day 10 posttransduction, cell media was collected and the concentration of human Factor H was measured by ELISA using an anti-Factor H antibody (Abcam, cat. ab252359).

Human podocytes with a mutation in Factor H were transduced with AAV2/9 virus containing a CFH transgene under the control of the 265 bp promoter demonstrated higher concentrations of human Factor H in the culture media than untransduced cells (**Figures 18A and 18B**). This demonstrates that a Factor H transgene delivered by AAV can be expressed in human podocytes.

### Transduction of Factor H mutated podocytes with plasmid encoding CFH

Conditionally immortalised human podocytes with a mutation in endogenous Factor H (Muehlig et al, 2020) grown in RPMI supplemented with 10% FBS and 1% ITS were seeded in 6-well culture plates and grown at 33°C until 70-80% confluency. For plasmid transfection, cells were incubated with 1.5ug of expression plasmid in serum-free media in the presence of polyethylenimine. Cells where no plasmid was added were used as a non-transfected (NT) control. The media was changed the following day to media containing FBS. On Day 3 post-transfection, media was collected and analysed by ELISA (Abcam, cat. ab252359).

Podocytes transfected with a plasmid expressing the CFH transgene under the control of the 265bp minimal nephrin promoter demonstrated higher concentrations of human Factor H than the non-transfected control (**Figure 18C**).

### Example 8 - Complement inhibition on glomerular endothelial cells with human CFH

### Materials and methods

Conditionally immortalised glomerular endothelial cells were grown at 33°C in a fully supplemented EGM-2 MV Endothelial Media from Lonza. Cells were seeded on a 96-well culture plate and transferred to a non-permissive temperature of 37°C to differentiate. Media was changed after 3 days. On Day 6 of differentiation, cell media was removed and cells were incubated for 20min at 37°C with a mixture of preactivated Zymosan (189ug/ml) and MgEGTA (10mM) in Gelatin Veronal Buffer (GVB) with or without purified Factor H. Following incubation, human Factor H-depleted serum was added to each well to a 10-fold final dilution. GVB was used instead of the serum for the negative control. Cells were further incubated at 37°C for 30min and then fixed. Assay plate with fixed cells was used for a cell-ELISA method using rabbit anti-C5b-9 primary antibody and anti-rabbit-HRP secondary antibody to detect MAC complexes in the cell membrane (as described previously; Jeon et al. 2014). As a read-out, optical density was measured using Promega Glomax Discover Microplate Reader with a 450nm filter.

### Results

Treating an in vitro model of complement activation with increasing concentrations of human Factor H leads to an increase in inhibition of complement activation as determined by measuring C5b9 on human glomerular endothelial cells (**Figure 19**).

Factor H inhibits C3bBb convertase, and as a cofactor to Factor I, it also cleaves C3b into its inactive form. The presence of Factor H inhibits the alternative pathway upstream to C5b-9 decreasing the amount of MAC complex formed on the surface of endothelial cells. Quantification of the MAC complex on the cell surface by ELISA is used here as an indirect method for measuring Factor H activity.

### Example 9 - Inhibition of complement in 293T HEK cells

The same method outlined in Example 8 was used in this Example. However, the following additional steps were performed: protein concentration of the media from NT and transfected HEK cells using Amicon Ultra-4 100K Centrifugal Filters. GenC cells were first pre-incubated for 1h at 37°C with 293T HEK culture media with or without purified Factor H or the concentrated media from transfected or non-transfected HEK cells. Following the incubation step, the media was removed and a mixture of 150ug/ml Zymosan and 10mM MgEGTA in plain RPMI was added. To activate the alternative pathway, human Factor H-Depleted serum was added for a final 1:10 dilution. Plain RPMI was added as a negative control. Cells were incubated for 45min at 37°C followed by the cell-ELISA method.

Media from cells transfected with a CMV-CFH plasmid demonstrated expression of Factor H compared to media from the non-transfected cells (**Figure 20A**). This expressed Factor H was able to inhibit a complement activation assay to the same extent as purified Factor H, whereas non-transfected cells which did not express Factor H were not able to inhibit the complement activation assay at all and demonstrated the same level of complement activation as the positive control (**Figure 20B**).

### Example 10 - Expression of CFH in the kidney in WT mice

### Materials and methods

100 µl of AAV2/9 gene therapy product (pAAV.NPHS1(265).hCFH.WPRE.bGH) or saline was administered to wild-type C57BL6 mice by IV tail vein injection. AAV expressed tagged wild-type human CFH transgene under a podocyte-specific promoter. AAV was harvested and purified by ultracentrifugation 3 days later and titrated (~1.5x10e13/ml) in PBS. All animals completed the study on Day 21 and culled. Kidneys were snap frozen in liquid nitrogen and used for RNA extraction by RNeasy Micro Kit (Qiagen Cat. No. / ID: 74004) as per the manufacturers' protocol. RNA was then converted into cDNA using High-Capacity RNA-to-cDNA^{™} Kit (4387406) prior to qPCR analysis. The following primers were used:

| | |
|---|---|
| **mHPRT-F** | tcagtcaacgggggacataaa (SEQ ID NO: 36) |
| **mHPRT-R** | ggggctgtactgcttaaccag (SEQ ID NO: 37) |
| **18s-F** | cccagtaagtgcgggtcataa (SEQ ID NO: 38) |
| **18s-R** | ccgagggcctcactaaacc (SEQ ID NO: 39) |
| **hCFH-F** | CTGATCGCAAGAAAGACCAGTA (SEQ ID NO: 40) |
| **hCFH-R** | TGGTAGCACTGAACGGAATTAG (SEQ ID NO: 41) |

Quantitative qPCR was performed on DNA samples from kidneys of pAAV_CFH injected mice. Standard curve qPCR with SYBR green reagents and passive ROX method was used to detect ITR presence in the kidney DNA samples. Viral genomes per pg of DNA were calculated using a standard curve of known quantities of an ITR amplicon. Finally, viral genomes per cell were calculated based on the assumption that diploid mouse cells have 6pg of DNA.

Immunofluorescence staining was performed on the frozen kidney sections 5 microns thick using an anti-nephrin antibody (PROGEN) and an anti-CFH antibody (ab124767).

Tissue was embedded in OCT compound (VWR, cat. number 361603E) and snap frozen in liquid nitrogen. 10uM sections were cut using a cryostat (Thermo, Cryostar NX270). Tissue sections were fixed with 4% Paraformaldehyde for 20 minutes at room temperature, permeabilized with 0.3% Triton-X for 15 minutes at room temperature and blocked with 5% BSA for 30 minutes at room temperature.

Primary antibodies (Rabbit monoclonal [EPR6226] to Factor H, Abcam cat. number ab124769 and Nephrin (NPHS1) (1243-1256) Guinea Pig Polyclonal Antibody, Origene cat. number BP5030) were diluted in 5% BSA. (1:100 for anti-factor H and 1:300 for anti-nephrin). Incubation was done at room temperature for 1 hour.

Secondary antibodies (Goat anti-Rabbit IgG (H+L) Secondary Antibody, Alexa Fluor Plus 488, Invitrogen, cat. number A32731 and Goat anti-Guinea Pig IgG (H+L) Alexa Fluor 568, Invitrogen, cat. number A-11075) were diluted 1:500 in PBS and incubation was done 30 minutes at room temperature.

Slides were mounted in Fluoromount-G, slide mounting medium (Southern Biotech, cat. number 0100-01) and imaged on an Leica DM750 Fluorescence Microscope at 20X magnification.

### Results

Injection of wild type mice with AAV containing human CFH under a podocyte-specific promoter leads to infection of the kidney by the AAV and expression of the virus and human CFH in the kidney compared to wild type mice injected with saline control (Figure 21A**-C**). Immunofluorescence staining demonstrates the co-localization of nephrin and CFH in the mouse glomerulus (**Figure 21D**).

Although the invention has been disclosed in connection with specific preferred embodiments, it should be understood that the invention as claimed should not be unduly limited to such specific embodiments. Indeed, various modifications of the disclosed modes for carrying out the invention, which are obvious to the skilled person are intended to be within the scope of the following claims.

## Claims

1. An adeno-associated virus (AAV) vector comprising a nucleotide sequence encoding an inhibitor of the complement system, wherein the nucleotide sequence encoding an inhibitor of the complement system is operably linked to a podocyte-specific promoter, wherein the inhibitor of the complement system is selected from the list consisting of CFI, CFH, FHL-1, C1INH, C4BP, CD55, CD35, CD46, CD59, vitronectin and clusterin, and wherein the podocyte-specific promoter is a NPHS1 promoter or a NPHS2 promoter.

2. The AAV vector according to claim 1, wherein the AAV vector is an AAV vector particle.

3. The AAV vector according to claim 2, wherein the AAV vector comprises:
(a) AAV3B capsid proteins, LK03 capsid proteins, or AAV9 capsid proteins, preferably wherein the AAV vector particle comprises LK03 capsid proteins; and/or
(b) capsid proteins with at least 90% sequence identity, at least 95% sequence identity, at least 96% sequence identity, at least 97% sequence identity, at least 98% sequence identity, at least 99% sequence identity, or 100% sequence identity to one or more of SEQ ID NOs: 1-3, preferably wherein the AAV vector particle comprises capsid proteins with at least 90% sequence identity, at least 95% sequence identity, at least 96% sequence identity, at least 97% sequence identity, at least 98% sequence identity, at least 99% sequence identity, or 100% sequence identity to SEQ ID NO: 2, more preferably wherein the AAV vector particle comprises capsid proteins comprising or consisting of SEQ ID NO: 2.

4. The AAV vector according to any preceding claim, wherein:
(a) the podocyte-specific promoter is a minimal NPHS1 promoter or a minimal NPHS2 promoter; and/or
(b) the podocyte-specific promoter has at least 70% sequence identity, at least 80% sequence identity, at least 85% sequence identity, at least 90% sequence identity, at least 95% sequence identity, at least 98% sequence identity, at least 99% sequence identity, or 100% sequence identity to any of SEQ ID NOs: 4, 5, 27-29, preferably wherein the podocyte-specific promoter has at least 70% sequence identity, at least 80% sequence identity, at least 85% sequence identity, at least 90% sequence identity, at least 95% sequence identity, at least 98% sequence identity, at least 99% sequence identity, or 100% sequence identity to SEQ ID NO: 27, more preferably wherein the podocyte-specific promoter consists of the nucleotide sequence of SEQ ID NO: 27.

5. The AAV vector according to any preceding claim, wherein the inhibitor of the complement system is CFI, CFH, or FHL-1, preferably wherein the inhibitor of the complement system is CFI.

6. The AAV vector according to any preceding claim, wherein:
(a) the inhibitor of the complement system has at least 70% sequence identity, at least 80% sequence identity, at least 85% sequence identity, at least 90% sequence identity, at least 95% sequence identity, at least 98% sequence identity, at least 99% sequence identity, or 100% sequence identity to any of SEQ ID NOs: 11, 13, 15, 17-25, preferably wherein the inhibitor of the complement system has at least 70% sequence identity, at least 80% sequence identity, at least 85% sequence identity, at least 90% sequence identity, at least 95% sequence identity, at least 98% sequence identity, at least 99% sequence identity, or 100% sequence identity to SEQ ID NO: 11, SEQ ID NO: 13, or SEQ ID NO: 15, more preferably wherein the inhibitor of the complement system comprises or consists of the polypeptide of SEQ ID NO: 11, SEQ ID NO: 13, or SEQ ID NO: 15; and/or
(b) the nucleotide sequence encoding an inhibitor of the complement system has at least 70% sequence identity, at least 80% sequence identity, at least 85% sequence identity, at least 90% sequence identity, at least 95% sequence identity, at least 98% sequence identity, at least 99% sequence identity, or 100% sequence identity to SEQ ID NO: 12, SEQ ID NO: 14, or SEQ ID NO: 16, preferably wherein the nucleotide sequence encoding an inhibitor of the complement system comprises or consists of the nucleotide sequence of SEQ ID NO: 12, SEQ ID NO: 14, or SEQ ID NO: 16.

7. The AAV vector according to any preceding claim, wherein the nucleotide sequence encoding an inhibitor of the complement system is operably linked to: (a) a Woodchuck hepatitis post-transcriptional regulatory element (WPRE); and/or (b) a polyadenylation signal; and/or (c) a Kozak sequence.

8. An isolated cell comprising an AAV vector according to any one of claims 1-7.

9. A pharmaceutical composition comprising an AAV vector according to any one of claims 1-7 or an isolated cell according to claim 8, in combination with a pharmaceutically acceptable carrier, diluent or excipient.

10. An AAV vector according to any one of claims 1-7, an isolated cell according to claim 8, or a pharmaceutical composition according to claim 9, for use as a medicament.

11. An AAV vector according to any one of claims 1-7, an isolated cell according to claim 8, or a pharmaceutical composition according to claim 9, for use in preventing or treating a complement-mediated kidney disease, wherein the complement-mediated kidney disease is IgA nephropathy, C3 glomerulopathy, atypical hemolytic uremic syndrome (aHUS), typical hemolytic-uremic syndrome, lupus nephritis, cryoglobulinemia, anti-GBM disease, Antineutrophil cytoplasmic antibody (ANCA)-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III.

12. The AAV vector, isolated cell, or pharmaceutical composition for use according to claim 11, wherein the complement-mediated kidney disease is IgA Nephropathy or C3 glomerulopathy, preferably wherein the C3 glomerulopathy is dense deposit disease or C3 glomerulonephritis.

13. The AAV vector, isolated cell, or pharmaceutical composition for use according to claim 11 or 12, wherein said AAV vector, said isolated cell, or said pharmaceutical composition is administered: (a) to a human subject; and/or (b) (i) systemically and/or by intravenous injection, and/or (ii) by injection into the renal artery or by ureteral or subcapsular injection.

## Patentansprüche

1. Vektor eines adeno-assoziierten Virus (AAV), umfassend eine Nukleotidsequenz, die für einen Inhibitor des Komplementsystems kodiert, wobei die Nukleotidsequenz, die für einen Inhibitor des Komplementsystems kodiert, funktionsfähig mit einem podocytenspezifischen Promotor verbunden ist, wobei der Inhibitor des Komplementsystems ausgewählt ist aus der Liste bestehend aus CFI, CFH, FHL-1, C1INH, C4BP, CD55, CD35, CD46, CD59, Vitronectin und Clusterin und wobei der podocytenspezifische Promotor ein NPHS1-Promotor oder ein NPHS2-Promotor ist.

2. AAV-Vektor nach Anspruch 1, wobei der AAV-Vektor ein AAV-Vektorpartikel ist.

3. AAV-Vektor nach Anspruch 2, wobei der AAV-Vektor Folgendes umfasst:
(a) AAV3B-Kapsidproteine, LK03-Kapsidproteine oder AAV9-Kapsidproteine, wobei das AAV-Vektorpartikel bevorzugt LK03-Kapsidproteine umfasst; und/oder
(b) Kapsidproteine mit mindestens 90 % Sequenzidentität, mindestens 95 % Sequenzidentität, mindestens 96 % Sequenzidentität, mindestens 97 % Sequenzidentität, mindestens 98 % Sequenzidentität, mindestens 99 % Sequenzidentität oder 100 % Sequenzidentität mit einer oder mehreren von SEQ ID NOs: 1-3, wobei das AAV-Vektorpartikel bevorzugt Kapsidproteine mit mindestens 90 % Sequenzidentität, mindestens 95 % Sequenzidentität, mindestens 96 % Sequenzidentität, mindestens 97 % Sequenzidentität, mindestens 98 % Sequenzidentität, mindestens 99 % Sequenzidentität oder 100 % Sequenzidentität mit SEQ ID NO: 2 umfasst, wobei das AAV-Vektorpartikel noch bevorzugter Kapsidproteine umfasst, die SEQ ID NO: 2 umfassen oder daraus bestehen.

4. AAV-Vektor nach einem vorhergehenden Anspruch, wobei:
(a) der podocytenspezifische Promotor ein minimaler NPHS1-Promotor oder ein minimaler NPHS2-Promotor ist; und/oder
(b) der podocytenspezifische Promotor mindestens 70 % Sequenzidentität, mindestens 80 % Sequenzidentität, mindestens 85 % Sequenzidentität, mindestens 90 % Sequenzidentität, mindestens 95 % Sequenzidentität, mindestens 98 % Sequenzidentität, mindestens 99 % Sequenzidentität oder 100 % Sequenzidentität mit einer von SEQ ID NOs: 4, 5, 27-29 aufweist, wobei der podocytenspezifische Promotor bevorzugt mindestens 70 % Sequenzidentität, mindestens 80 % Sequenzidentität, mindestens 85 % Sequenzidentität, mindestens 90 % Sequenzidentität, mindestens 95 % Sequenzidentität, mindestens 98 % Sequenzidentität, mindestens 99 % Sequenzidentität oder 100 % Sequenzidentität mit SEQ ID NO: 27 aufweist, wobei der podocytenspezifische Promotor noch bevorzugter aus der Nukleotidsequenz von SEQ ID NO: 27 besteht.

5. AAV-Vektor nach einem der vorhergehenden Ansprüche, wobei der Inhibitor des Komplementsystems CFI, CFH oder FHL-1 ist, wobei der Inhibitor des Komplementsystems bevorzugt CFI ist.

6. AAV-Vektor nach einem vorhergehenden Anspruch, wobei:
(a) der Inhibitor des Komplementsystems mindestens 70 % Sequenzidentität, mindestens 80 % Sequenzidentität, mindestens 85 % Sequenzidentität, mindestens 90 % Sequenzidentität, mindestens 95 % Sequenzidentität, mindestens 98 % Sequenzidentität, mindestens 99 % Sequenzidentität oder 100 % Sequenzidentität mit einer von SEQ ID NOs: 11, 13, 15, 17-25 aufweist, wobei der Inhibitor des Komplementsystems bevorzugt mindestens 70 % Sequenzidentität, mindestens 80 % Sequenzidentität, mindestens 85 % Sequenzidentität, mindestens 90 % Sequenzidentität, mindestens 95 % Sequenzidentität, mindestens 98 % Sequenzidentität, mindestens 99 % Sequenzidentität oder 100 % Sequenzidentität mit SEQ ID NO: 11, SEQ ID NO: 13 oder SEQ ID NO: 15 aufweist, wobei der Inhibitor des Komplementsystems noch bevorzugter das Polypeptid von SEQ ID NO: 11, SEQ ID NO: 13 oder SEQ ID NO: 15 umfasst oder daraus besteht; und/oder
(b) die Nukleotidsequenz, die für einen Inhibitor des Komplementsystems kodiert, mindestens 70 % Sequenzidentität, mindestens 80 % Sequenzidentität, mindestens 85 % Sequenzidentität, mindestens 90 % Sequenzidentität, mindestens 95 % Sequenzidentität, mindestens 98 % Sequenzidentität, mindestens 99 % Sequenzidentität oder 100 % Sequenzidentität mit SEQ ID NO: 12, SEQ ID NO: 14 oder SEQ ID NO: 16 aufweist, wobei die Nukleotidsequenz, die für einen Inhibitor des Komplementsystems kodiert, bevorzugt die Nukleotidsequenz von SEQ ID NO: 12, SEQ ID NO: 14 oder SEQ ID NO: 16 umfasst oder daraus besteht.

7. AAV-Vektor nach einem der vorhergehenden Ansprüche, wobei die Nukleotidsequenz, die für einen Inhibitor des Komplementsystems kodiert, funktionsfähig mit Folgendem verbunden ist: (a) einem posttranskriptionellen regulatorischen Element (WPRE) von Woodchuck-Hepatitis; und/oder (b) einem Polyadenylierungssignal; und/oder (c) einer Kozak-Sequenz.

8. Isolierte Zelle, umfassend einen AAV-Vektor nach einem der Ansprüche 1-7.

9. Pharmazeutische Zusammensetzung, umfassend einen AAV-Vektor nach einem der Ansprüche 1-7 oder eine isolierte Zelle nach Anspruch 8, in Kombination mit einem pharmazeutisch unbedenklichen Träger, Verdünnungsmittel oder Hilfsstoff.

10. AAV-Vektor nach einem der Ansprüche 1-7, isolierte Zelle nach Anspruch 8 oder pharmazeutische Zusammensetzung nach Anspruch 9 zur Verwendung als Medikament.

11. AAV-Vektor nach einem der Ansprüche 1-7, isolierte Zelle nach Anspruch 8 oder pharmazeutische Zusammensetzung nach Anspruch 9 zur Verwendung beim Vorbeugen oder Behandeln einer komplementvermittelten Nierenerkrankung, wobei es sich bei der komplementvermittelten Nierenerkrankung um Folgendes handelt, IgA-Nephropathie, C3-Glomerulopathie, atypisches hämolytisch-urämisches Syndrom (aHUS), typisches hämolytisch-urämisches Syndrom, Lupus-Nephritis, Kryoglobulinämie, Anti-GBM-Erkrankung, mit dem Antineutrophilen cytoplasmatischen Antikörper (ANCA) assoziierte Vaskulitis, bakterielle Endokarditis, postinfektiöse Glomerulonephritis, Antikörper-vermittelte Abstoßung von Nierentransplantation, membranöse Nephropathie, membranoproliferative Glomerulonephritis I oder membranoproliferative Glomerulonephritis III.

12. AAV-Vektor, isolierte Zelle oder pharmazeutische Zusammensetzung zur Verwendung nach Anspruch 11, wobei es sich bei der komplementvermittelten Nierenerkrankung um eine IgA-Nephropathie oder eine C3-Glomerulopathie handelt, wobei die C3-Glomerulopathie bevorzugt eine Dense Deposit Disease oder eine C3-Glomerulonephritis ist.

13. AAV-Vektor, isolierte Zelle oder pharmazeutische Zusammensetzung zur Verwendung nach Anspruch 11 oder 12, wobei der AAV-Vektor, die isolierte Zelle oder die pharmazeutische Zusammensetzung wie folgt verabreicht wird: (a) an ein menschliches Subjekt; und/oder (b) (i) systemisch und/oder durch intravenöse Injektion und/oder (ii) durch Injektion in die Nierenarterie oder durch ureterale oder subkapsuläre Injektion.

## Revendications

1. Vecteur de virus adéno-associé (AAV) comprenant une séquence nucléotidique codant pour un inhibiteur du système du complément, dans lequel la séquence nucléotidique codant pour un inhibiteur du système du complément est liée de manière opérationnelle à un promoteur spécifique des podocytes, dans lequel l'inhibiteur du système du complément est choisi dans la liste constituée de CFI, CFH, FHL-1, C1INH, C4BP, CD55, CD35, CD46, CD59, la vitronectine et la clusterine, et dans lequel le promoteur spécifique des podocytes est un promoteur NPHS1 ou un promoteur NPHS2.

2. Vecteur AAV selon la revendication 1, dans lequel le vecteur AAV est une particule de vecteur AAV.

3. Vecteur AAV selon la revendication 2, dans lequel le vecteur AAV comprend :
(a) des protéines de capside AAV3B, des protéines de capside LK03 ou des protéines de capside AAV9, dans lequel de préférence la particule de vecteur AAV comprend des protéines de capside LK03 ; et/ou
(b) des protéines de capside présentant au moins 90 % d'identité de séquence, au moins 95 % d'identité de séquence, au moins 96 % d'identité de séquence, au moins 97 % d'identité de séquence, au moins 98 % d'identité de séquence, au moins 99 % d'identité de séquence ou 100 % d'identité de séquence avec une ou plusieurs des SEQ ID N° : 1 à 3, dans lequel de préférence la particule de vecteur AAV comprend des protéines de capside présentant au moins 90 % d'identité de séquence, au moins 95 % d'identité de séquence, au moins 96 % d'identité de séquence, au moins 97 % d'identité de séquence, au moins 98 % d'identité de séquence, au moins 99 % d'identité de séquence ou 100 % d'identité de séquence avec SEQ ID N° : 2, dans lequel idéalement la particule de vecteur AAV comprend des protéines de capside comprenant ou constituées de SEQ ID N° : 2.

4. Vecteur AAV selon une quelconque revendication précédente, dans lequel :
(a) le promoteur spécifique des podocytes est un promoteur NPHS1 minimal ou un promoteur NPHS2 minimal ; et/ou
(b) le promoteur spécifique des podocytes présente au moins 70 % d'identité de séquence, au moins 80 % d'identité de séquence, au moins 85 % d'identité de séquence, au moins 90 % d'identité de séquence, au moins 95 % d'identité de séquence, au moins 98 % d'identité de séquence, au moins 99 % d'identité de séquence ou 100 % d'identité de séquence avec l'une quelconque des SEQ ID N° : 4, 5, 27 à 29, dans lequel de préférence le promoteur spécifique des podocytes présente au moins 70 % d'identité de séquence, au moins 80 % d'identité de séquence, au moins 85 % d'identité de séquence, au moins 90 % d'identité de séquence, au moins 95 % d'identité de séquence, au moins 98 % d'identité de séquence, au moins 99 % d'identité de séquence ou 100 % d'identité de séquence avec SEQ ID N° : 27, dans lequel idéalement le promoteur spécifique des podocytes est constitué de la séquence nucléotidique de SEQ ID N° : 27.

5. Vecteur AAV selon une quelconque revendication précédente, dans lequel l'inhibiteur du système du complément est CFI, CFH ou FHL-1, dans lequel de préférence l'inhibiteur du système du complément est CFI.

6. Vecteur AAV selon une quelconque revendication précédente, dans lequel :
(a) l'inhibiteur du système du complément présente au moins 70 % d'identité de séquence, au moins 80 % d'identité de séquence, au moins 85 % d'identité de séquence, au moins 90 % d'identité de séquence, au moins 95 % d'identité de séquence, au moins 98 % d'identité de séquence, au moins 99 % d'identité de séquence ou 100 % d'identité de séquence avec l'une quelconque des SEQ ID N° : 11, 13, 15, 17 à 25, dans lequel de préférence l'inhibiteur du système du complément présente au moins 70 % d'identité de séquence, au moins 80 % d'identité de séquence, au moins 85 % d'identité de séquence, au moins 90 % d'identité de séquence, au moins 95 % d'identité de séquence, au moins 98 % d'identité de séquence, au moins 99 % d'identité de séquence ou 100 % d'identité de séquence avec SEQ ID N° : 11, SEQ ID N° : 13 ou SEQ ID N° : 15, dans lequel idéalement l'inhibiteur du système du complément comprend ou est constitué du polypeptide de SEQ ID N° : 11, SEQ ID N° : 13 ou SEQ ID N° : 15 ; et/ou
(b) la séquence nucléotidique codant pour un inhibiteur du système du complément présente au moins 70 % d'identité de séquence, au moins 80 % d'identité de séquence, au moins 85 % d'identité de séquence, au moins 90 % d'identité de séquence, au moins 95 % d'identité de séquence, au moins 98 % d'identité de séquence, au moins 99 % d'identité de séquence ou 100 % d'identité de séquence avec SEQ ID N° : 12, SEQ ID N° : 14 ou SEQ ID N° : 16, dans lequel de préférence la séquence nucléotidique codant pour un inhibiteur du système du complément comprend ou est constituée de la séquence nucléotidique de SEQ ID N° : 12, SEQ ID N° : 14 ou SEQ ID N° : 16.

7. Vecteur AAV selon une quelconque revendication précédente, dans lequel la séquence nucléotidique codant pour un inhibiteur du système du complément est liée de manière opérationnelle à : (a) un élément régulateur post-transcriptionnel de l'hépatite de la marmotte (WPRE) ; et/ou (b) un signal de polyadénylation ; et/ou (c) une séquence de Kozak.

8. Cellule isolée comprenant un vecteur AAV selon l'une quelconque des revendications 1 à 7.

9. Composition pharmaceutique comprenant un vecteur AAV selon l'une quelconque des revendications 1 à 7 ou une cellule isolée selon la revendication 8, en combinaison avec un support, un diluant ou un excipient acceptable pharmaceutiquement.

10. Vecteur AAV selon l'une quelconque des revendications 1 à 7, cellule isolée selon la revendication 8, ou composition pharmaceutique selon la revendication 9, destiné(e) à être utilisé(e) en tant que médicament.

11. Vecteur AAV selon l'une quelconque des revendications 1 à 7, cellule isolée selon la revendication 8 ou composition pharmaceutique selon la revendication 9, destiné(e) à être utilisé(e) dans la prévention ou le traitement d'une maladie rénale médiée par le complément, ladite maladie rénale médiée par le complément étant une néphropathie à IgA, une glomérulopathie à C3, un syndrome hémolytique et urémique atypique (SHUa), un syndrome hémolytique et urémique typique, une néphrite lupique, une cryoglobulinémie, une maladie anti-GBM, une vascularite associée aux anticorps anticytoplasme des neutrophiles (ANCA), une endocardite bactérienne, une glomérulonéphrite post-infectieuse, un rejet de greffe rénale médié par les anticorps, une néphropathie membraneuse, une glomérulonéphrite membranoproliférative de type I ou une glomérulonéphrite membranoproliférative de type III.

12. Vecteur AAV, cellule isolée ou composition pharmaceutique destiné(e) à être utilisé(e) selon la revendication 11, ladite maladie rénale médiée par le complément étant une néphropathie à IgA ou une glomérulopathie à C3, ladite glomérulopathie à C3 étant de préférence une maladie des dépôts denses ou une glomérulonéphrite à C3.

13. Vecteur AAV, cellule isolée ou composition pharmaceutique destiné(e) à être utilisé(e) selon la revendication 11 ou 12, ledit vecteur AAV, ladite cellule isolée ou ladite composition pharmaceutique étant administré(e) : (a) à un sujet humain ; et/ou (b) (i) par voie systémique et/ou par injection intraveineuse, et/ou (ii) par injection dans l'artère rénale ou par injection urétérale ou sous-capsulaire.
